(19)

**Europäisches Patentamt**

**European Patent Office**

**Office européen des brevets**

(11) **EP 3 215 532 B1**

(12) **EUROPEAN PATENT SPECIFICATION**

(45) Date of publication and mention
of the grant of the patent:
**23.10.2019 Bulletin 2019/43**

(51) Int Cl.:
*C07K 16/28* *(2006.01)*      *A61K 47/50* *(2017.01)*
*A61P 35/00* *(2006.01)*

(21) Application number: **15790559.7**

(22) Date of filing: **05.11.2015**

(86) International application number:
**PCT/EP2015/075820**

(87) International publication number:
**WO 2016/071448 (12.05.2016 Gazette 2016/19)**

(54) **ANTI-TIM3 ANTIBODIES AND METHODS OF USE**

ANTI-TIM3-ANTIKÖRPER UND VERFAHREN ZUR VERWENDUNG

ANTICORPS ANTI-TIM3 ET PROCÉDÉS D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.11.2014 EP 14192175**
**02.10.2015 EP 15188056**

(43) Date of publication of application:
**13.09.2017 Bulletin 2017/37**

(73) Proprietor: **F. Hoffmann-La Roche AG**
**4070 Basel (CH)**

(72) Inventors:
• **LIFKE, Valeria**
**82377 Penzberg (DE)**
• **GEORGES, Guy**
**82392 Habach (DE)**
• **LEVITSKY, Victor**
**CH-8903 Birmensdorf (CH)**
• **PLOETTNER, Oliver**
**82205 Gilching (DE)**
• **SEEBER, Stefan**
**82404 Sindelsdorf (DE)**
• **WEISER, Barbara**
**82377 Penzberg (DE)**
• **WUENSCHE, Ildiko**
**82377 Penzberg (DE)**
• **ZWICK, Adrian**
**82377 Penzberg (DE)**

(74) Representative: **Burger, Alexander**
**Roche Diagnostics GmbH**
**Patentabteilung**
**Nonnenwald 2**
**82377 Penzberg (DE)**

(56) References cited:
WO-A1-2010/084999      WO-A2-03/063792
WO-A2-2013/006490      US-A1- 2012 189 617

• YOSHIKANE KIKUSHIGE ET AL: "TIM-3 as a novel therapeutic target for eradicating acute myelogenous leukemia stem cells", INTERNATIONAL JOURNAL OF HEMATOLOGY, vol. 98, no. 6, 18 September 2013 (2013-09-18), pages 627-633, XP055181432, ISSN: 0925-5710, DOI: 10.1007/s12185-013-1433-6

SEQUENCE LISTING

<110> F. Hoffmann-La Roche AG

<120> ANTI-TIM3 ANTIBODIES AND METHODS OF USE

<130> 32411

<150> EP14192175.9
<151> 2014-11-06

<150> EP15188056.4
<151> 2015-10-02

<160> 86

<170> PatentIn version 3.5

<210> 1
<211> 9
<212> PRT
<213> Mus musculus

<400> 1

```
Gly Phe Ser Leu Ser Thr Ser Gly Met
1               5
```

<210> 2
<211> 3
<212> PRT
<213> Mus musculus

<400> 2

```
Leu Asn Asp
1
```

<210> 3
<211> 8
<212> PRT
<213> Mus musculus

<400> 3

```
Asn Gly Tyr Leu Tyr Ala Leu Asp
1               5
```

<210> 4
<211> 6
<212> PRT
<213> Mus musculus

<400> 4

```
Ser Ser Ser Val Asn Tyr
1               5
```

<210> 5
<211> 3

<212> PRT
<213> Mus musculus

<400> 5

Asp Ala Phe
1

<210> 6
<211> 7
<212> PRT
<213> Mus musculus

<400> 6

Trp Ser Ser Tyr Pro Trp Thr
1               5

<210> 7
<211> 120
<212> PRT
<213> Mus musculus

<400> 7

Gln Val Thr Leu Lys Glu Ser Gly Pro Gly Ile Leu Gln Pro Ser Gln
1               5                   10                  15

Thr Leu Arg Leu Thr Cys Ser Phe Ser Gly Phe Ser Leu Ser Thr Ser
            20                  25                  30

Gly Met Ser Val Gly Trp Ile Arg Gln Pro Ser Gly Lys Gly Leu Glu
            35                  40                  45

Trp Leu Ala His Ile Trp Leu Asn Asp Asp Val Phe Phe Asn Pro Ala
        50                  55                  60

Leu Lys Ser Arg Leu Thr Ile Ser Lys Asp Thr Ser Asn Asn Gln Val
65                  70                  75                  80

Phe Leu Gln Ile Ala Ser Val Val Thr Ala Asp Thr Ala Thr Tyr Tyr
                85                  90                  95

Cys Val Arg Ala Asn Gly Tyr Leu Tyr Ala Leu Asp Tyr Trp Gly Gln
            100                 105                 110

Gly Thr Ser Val Thr Val Ser Ser
            115                 120

<210> 8
<211> 106
<212> PRT

<213> Mus musculus

<400> 8

```
Gln Ile Val Leu Thr Gln Ser Pro Ala Ile Met Ser Ala Ser Pro Gly
1               5               10              15

Gln Lys Val Thr Ile Thr Cys Ser Ala Ser Ser Ser Val Asn Tyr Thr
            20              25              30

Gln Trp Tyr Gln Gln Lys Leu Gly Ser Ser Pro Lys Leu Trp Ile Tyr
        35              40              45

Asp Ala Phe Lys Leu Ala Pro Gly Val Pro Ala Arg Phe Ser Gly Ser
        50              55              60

Gly Thr Gly Thr Ser Tyr Ser Leu Thr Ile Ser Ser Met Glu Ala Glu
65              70              75              80

Asp Ala Ala Ser Tyr Phe Cys His Gln Trp Ser Ser Tyr Pro Trp Thr
            85              90              95

Phe Gly Gly Gly Thr Lys Leu Glu Ile Lys
        100             105
```

<210> 9
<211> 120
<212> PRT
<213> Mus musculus

<400> 9

```
Gln Val Thr Leu Lys Glu Ser Gly Pro Gly Ile Leu Gln Pro Ser Gln
1               5               10              15

Thr Leu Ser Leu Thr Cys Ser Phe Ser Gly Phe Ser Leu Ser Thr Ser
            20              25              30

Gly Met Ser Val Gly Trp Ile Arg Gln Pro Ser Gly Lys Gly Leu Glu
        35              40              45

Trp Leu Ala His Ile Trp Leu Asn Asp Asp Val Phe Phe Asn Pro Ala
        50              55              60

Leu Lys Arg Arg Leu Thr Ile Ser Lys Asp Thr Ser Asn Asn Gln Val
65              70              75              80

Phe Leu Gln Ile Ala Ser Val Val Thr Ala Asp Thr Ala Thr Tyr Tyr
            85              90              95
```

```
        Cys Val Arg Ala Asn Gly Tyr Leu Tyr Ala Leu Asp Tyr Trp Gly Gln
                    100                 105             110


        Gly Ile Ser Val Thr Val Ser Ser
                    115             120
```

<210> 10
<211> 106
<212> PRT
<213> Mus musculus

<400> 10

```
        Gln Ile Val Leu Thr Gln Ser Pro Ala Ile Met Ser Ala Ser Pro Gly
        1               5               10              15


        Gln Lys Val Thr Ile Thr Cys Ser Ala Ser Ser Ser Val Asn Tyr Thr
                    20              25              30


        Gln Trp Tyr Gln Gln Lys Leu Gly Ser Ser Pro Lys Leu Trp Ile Tyr
                35              40              45


        Asp Ala Phe Lys Leu Ala Pro Gly Val Pro Ala Arg Phe Ser Gly Ser
            50              55              60


        Gly Thr Gly Thr Ser Tyr Ser Leu Thr Ile Ser Ser Met Glu Ala Glu
        65              70              75              80


        Asp Ala Ala Ser Tyr Phe Cys His Gln Trp Ser Ser Tyr Pro Trp Thr
                    85              90              95


        Phe Gly Gly Gly Thr Lys Leu Glu Ile Lys
                    100             105
```

<210> 11
<211> 6
<212> PRT
<213> Mus musculus

<400> 11

```
                        Ser Ser Ser Val Gln Tyr
                        1               5
```

<210> 12
<211> 6
<212> PRT
<213> Mus musculus

<400> 12

Ser Ser Ser Val Ser Tyr
1               5

<210> 13
<211> 7
<212> PRT
<213> Mus musculus


<400> 13

Gly Tyr Ser Phe Thr Ser Tyr
1               5

<210> 14
<211> 3
<212> PRT
<213> Mus musculus


<400> 14

Ser Asp Ser
1

<210> 15
<211> 9
<212> PRT
<213> Mus musculus


<400> 15

Gly Tyr Tyr Ala Trp Tyr Tyr Phe Asp
1               5

<210> 16
<211> 7
<212> PRT
<213> Mus musculus


<400> 16

Ser Gln Ser Ile Gly Asn Asn
1               5

<210> 17
<211> 3
<212> PRT
<213> Mus musculus


<400> 17

Tyr Ala Ser
1

<210> 18
<211> 6
<212> PRT
<213> Mus musculus

<400> 18

```
Ser Asn Ser Trp Pro Leu
1               5
```

<210> 19
<211> 120
<212> PRT
<213> Mus musculus

<400> 19

```
Gln Val Gln Leu Gln Gln Ser Gly Pro Gln Leu Val Arg Pro Gly Ala
1               5               10              15

Ser Val Gln Ile Ser Cys Lys Ala Ser Gly Tyr Ser Phe Thr Ser Tyr
            20              25              30

Leu Leu His Trp Leu Lys Gln Arg Pro Gly Gln Gly Leu Glu Trp Ile
            35              40              45

Gly Met Ile Asp Pro Ser Asp Ser Glu Thr Arg Leu Asn Gln Lys Phe
        50              55              60

Lys Asp Lys Ala Thr Leu Thr Val Asp Lys Ser Ser Ser Thr Ala Tyr
65              70              75              80

Met Gln Leu Ser Ser Pro Thr Ser Glu Asp Ser Ala Val Tyr Tyr Cys
            85              90              95

Ala Arg Asp Gly Tyr Tyr Ala Trp Tyr Tyr Phe Asp Cys Trp Gly Gln
            100             105             110

Gly Thr Thr Leu Thr Val Ser Ser
        115             120
```

<210> 20
<211> 107
<212> PRT
<213> Mus musculus

<400> 20

```
Asp Ile Val Leu Thr Gln Ser Pro Ala Thr Leu Ser Val Thr Pro Gly
1               5                   10                  15

Asp Arg Val Ser Leu Ser Cys Arg Ala Ser Gln Ser Ile Gly Asn Asn
            20                  25                  30

Leu His Trp Tyr Gln Gln Lys Ser His Glu Ser Pro Arg Leu Leu Ile
        35                  40                  45

Lys Tyr Ala Ser His Ser Ile Ser Gly Ile Pro Ser Lys Phe Ser Gly
    50                  55                  60

Thr Gly Ser Gly Thr Asp Phe Thr Leu Ser Phe Asn Ser Val Glu Thr
65                  70                  75                  80

Glu Asp Phe Gly Met Tyr Phe Cys Gln Gln Ser Asn Ser Trp Pro Leu
                85                  90                  95

Thr Phe Gly Ala Gly Thr Lys Leu Glu Leu Lys
            100                 105
```

<210> 21
<211> 5
<212> PRT
<213> Mus musculus

<400> 21

```
Gly Asp Ser Ile Ala
1               5
```

<210> 22
<211> 3
<212> PRT
<213> Mus musculus

<400> 22

```
Tyr Ser Gly
1
```

<210> 23
<211> 4
<212> PRT
<213> Mus musculus

<400> 23

```
Asp Tyr Phe Asp
1
```

<210> 24
<211> 7

<212> PRT
<213> Mus musculus

<400> 24

```
                        Arg Gln Asp Val Arg Lys Asn
                        1                   5
```

<210> 25
<211> 3
<212> PRT
<213> Mus musculus

<400> 25

```
                            Tyr Thr Ser
                            1
```

<210> 26
<211> 6
<212> PRT
<213> Mus musculus

<400> 26

```
                        Tyr Asp Asn Leu Pro Phe
                        1                   5
```

<210> 27
<211> 114
<212> PRT
<213> Mus musculus

<400> 27

```
Glu Val Gln Leu Gln Glu Ser Gly Pro Ser Leu Val Lys Pro Ser Gln
1                   5                   10                  15

Thr Leu Ser Leu Thr Cys Ser Val Thr Gly Asp Ser Ile Ala Ser Ala
            20                  25                  30

Tyr Trp Asn Trp Ile Arg Lys Phe Pro Gly Asn Lys Leu Glu Tyr Met
        35                  40                  45

Gly Tyr Ile Asn Tyr Ser Gly Ser Thr Tyr Tyr Asn Pro Ser Leu Lys
    50                  55                  60

Ser Arg Ile Ser Ile Thr Arg Asp Thr Ser Gln Asn Gln Tyr Tyr Leu
65                  70                  75                  80

Gln Leu Asn Ser Val Thr Thr Glu Asp Thr Ala Thr Tyr Tyr Cys Val
            85                  90                  95

Thr Gly Asp Tyr Phe Asp Tyr Trp Gly Arg Gly Thr Thr Leu Thr Val
            100                 105                 110

Ser Ser
```

<210> 28
<211> 107
<212> PRT
<213> Mus musculus

<400> 28

```
Asp Ile Gln Met Thr Gln Ser Pro Ser Ser Leu Ser Ala Tyr Leu Gly
1               5                   10                  15

Gly Lys Val Thr Ile Thr Cys Lys Ala Arg Gln Asp Val Arg Lys Asn
            20                  25                  30

Ile Gly Trp Tyr Gln His Lys Pro Gly Lys Gly Pro Arg Leu Leu Ile
            35                  40                  45

Trp Tyr Thr Ser Thr Leu Gln Ser Gly Ile Pro Ser Arg Phe Ser Gly
        50                  55                  60

Ser Gly Ser Gly Arg Asp Tyr Ser Phe Asn Ile Asn Asn Leu Glu Pro
65                  70                  75                  80

Glu Asp Ile Ala Thr Tyr Tyr Cys Leu Gln Tyr Asp Asn Leu Pro Phe
                85                  90                  95

Thr Phe Gly Thr Gly Thr Lys Leu Glu Ile Arg
            100                 105
```

<210> 29
<211> 5
<212> PRT
<213> Mus musculus

<400> 29

```
                        Gly Tyr Thr Phe Thr
                        1               5
```

<210> 30
<211> 3
<212> PRT
<213> Mus musculus

<400> 30

```
                        Glu Thr Tyr
                        1
```

<210> 31
<211> 4
<212> PRT
<213> Mus musculus

<400> 31

```
                        Gly Tyr Pro Ala
                        1
```

<210> 32
<211> 12

<212> PRT
<213> Mus musculus

<400> 32

```
            Ser Arg Thr Ile Leu His Ser Ser Gly Asn Thr Tyr
            1               5                   10
```

<210> 33
<211> 3
<212> PRT
<213> Mus musculus

<400> 33

```
                        Lys Val Ser
                        1
```

<210> 34
<211> 6
<212> PRT
<213> Mus musculus

<400> 34

```
                    Asp Ser His Val Pro Phe
                    1               5
```

<210> 35
<211> 115
<212> PRT
<213> Mus musculus

<400> 35

```
        Gln Ile Gln Leu Val Gln Ser Gly Pro Glu Leu Lys Lys Pro Gly Glu
        1               5                   10                  15

        Thr Val Lys Ile Ser Cys Lys Ala Ser Gly Tyr Thr Phe Thr Asp Tyr
                        20                  25                  30

        Ser Met His Trp Val Lys Gln Ala Pro Gly Arg Gly Leu Lys Trp Met
                        35                  40                  45

        Gly Tyr Ile Asn Thr Glu Thr Tyr Glu Pro Thr Phe Gly Ala Asp Phe
                50                  55                  60

        Lys Gly Arg Phe Ala Phe Ser Leu Asp Thr Ser Ala Thr Thr Ala Tyr
        65                  70                  75                  80

        Leu Gln Ile Asn Ser Leu Lys Thr Glu Asp Thr Ala Thr Phe Phe Cys
                            85                  90                  95
```

```
        Gly Gly Gly Gly Tyr Pro Ala Tyr Trp Gly Gln Gly Thr Val Val Ile
                    100                 105                 110

        Val Ser Ala
                115


<210> 36
<211> 112
<212> PRT
<213> Mus musculus


<400> 36


        Asp Val Leu Met Thr Gln Thr Pro Leu Ser Leu Pro Val Ser Leu Gly
        1               5                   10                  15


        Asp Gln Ala Ser Ile Ser Cys Arg Ser Ser Arg Thr Ile Leu His Ser
                    20                  25                  30


        Ser Gly Asn Thr Tyr Leu Glu Trp Tyr Leu Gln Lys Pro Gly Gln Ser
                    35                  40                  45


        Pro Lys Leu Leu Ile Tyr Lys Val Ser Asn Arg Phe Ser Gly Val Pro
                50                  55                  60


        Asp Arg Phe Ser Gly Ser Gly Ser Gly Thr Asp Phe Thr Leu Asn Ile
        65                  70                  75                  80


        Ser Arg Val Glu Ala Glu Asp Leu Gly Val Tyr Tyr Cys Phe Gln Asp
                        85                  90                  95


        Ser His Val Pro Phe Thr Phe Gly Thr Gly Thr Lys Leu Glu Ile Lys
                    100                 105                 110

<210> 37
<211> 7
<212> PRT
<213> Mus musculus

<400> 37


                        Gly Phe Asn Ile Lys Thr Thr
                        1               5

<210> 38
<211> 3
<212> PRT
<213> Mus musculus


<400> 38
```

Ala Asp Asp

1

<210> 39
<211> 8
<212> PRT
<213> Mus musculus

<400> 39

Phe Gly Tyr Val Ala Trp Phe Ala
1               5

<210> 40
<211> 7
<212> PRT
<213> Mus musculus

<400> 40

Ser Gln Ser Val Asp Asn Tyr
1               5

<210> 41
<211> 3
<212> PRT
<213> Mus musculus

<400> 41

Tyr Ala Ser
1

<210> 42
<211> 6
<212> PRT
<213> Mus musculus

<400> 42

His Tyr Ser Ser Pro Tyr
1               5

<210> 43
<211> 119
<212> PRT
<213> Mus musculus

<400> 43

```
Glu Val Gln Leu Gln Gln Ser Val Ala Glu Leu Val Arg Pro Gly Ala
1               5               10              15

Ser Val Lys Leu Ser Cys Thr Ala Ser Gly Phe Asn Ile Lys Thr Thr
            20              25              30

Tyr Met His Trp Val Lys Gln Arg Pro Glu Gln Gly Leu Glu Trp Ile

        35              40              45

Gly Arg Ile Asp Pro Ala Asp Asp Asn Thr Lys Tyr Ala Pro Lys Phe
        50              55              60

Gln Gly Lys Ala Thr Ile Thr Ala Asp Thr Ser Ser Asn Thr Ala Tyr
65              70              75              80

Leu Gln Leu Ser Ser Leu Thr Ser Glu Asp Ala Ala Ile Tyr Tyr Cys
            85              90              95

Val Arg Asp Phe Gly Tyr Val Ala Trp Phe Ala Tyr Trp Gly Gln Gly
            100             105             110

Thr Leu Val Thr Phe Ser Ala
            115
```

<210> 44
<211> 107
<212> PRT
<213> Mus musculus

<400> 44

```
Asn Ile Val Met Thr Pro Thr Pro Lys Phe Leu Pro Val Ser Ser Gly
1               5               10              15

Asp Arg Val Thr Met Thr Cys Arg Ala Ser Gln Ser Val Asp Asn Tyr
            20              25              30

Val Ala Trp Tyr Gln Gln Lys Pro Gly Gln Ser Pro Lys Leu Leu Ile
            35              40              45

Tyr Tyr Ala Ser Asn Arg Tyr Ile Gly Val Pro Asp Arg Phe Thr Gly
    50              55              60

Ser Gly Ser Gly Thr Asp Phe Thr Phe Thr Ile Ser Ser Val Gln Val
65              70              75              80

Glu Asp Leu Ala Val Tyr Phe Cys Gln Gln His Tyr Ser Ser Pro Tyr
            85              90              95

Thr Phe Gly Ser Gly Thr Lys Leu Glu Ile Lys
            100             105
```

<210> 45
<211> 7
<212> PRT
<213> Mus musculus

<400> 45

```
Gly Tyr Pro Phe Ser Glu Tyr
1               5
```

<210> 46
<211> 3
<212> PRT
<213> Mus musculus

<400> 46

```
Glu Thr Gly
1
```

<210> 47
<211> 4
<212> PRT
<213> Mus musculus

<400> 47

```
Gly Tyr Pro Ala
1
```

<210> 48
<211> 12

<212> PRT
<213> Mus musculus

<400> 48

```
Ser Arg Ser Ile Val His Ser Ser Gly Asn Thr Tyr
1               5                   10
```

<210> 49
<211> 3
<212> PRT
<213> Mus musculus

<400> 49

```
Lys Val Ser
1
```

<210> 50
<211> 5
<212> PRT
<213> Mus musculus

<400> 50

```
Asp Ser His Val Pro
1               5
```

<210> 51
<211> 115
<212> PRT
<213> Mus musculus

<400> 51

```
Gln Ile Gln Leu Val Gln Ser Gly Pro Glu Leu Lys Lys Pro Gly Glu
1               5               10              15

Thr Val Lys Ile Ser Cys Lys Ala Ser Gly Tyr Pro Phe Ser Glu Tyr
            20              25              30

Ser Ile His Trp Val Lys Gln Ala Pro Gly Lys Gly Leu Lys Trp Met
        35              40              45

Val Tyr Val Asn Thr Glu Thr Gly Gln Pro Ile Val Gly Asp Asp Phe
    50              55              60

Arg Gly Arg Phe Val Leu Ser Leu Glu Thr Ser Ala Ser Thr Ala Tyr
65              70              75              80

Leu Gln Ile Asn Asn Leu Lys Asn Glu Asp Thr Ala Thr Tyr Phe Cys
            85              90              95

Gly Gly Gly Gly Tyr Pro Ala Tyr Trp Gly Gln Gly Thr Leu Val Thr
        100             105             110

Val Ser Ala
        115
```

<210> 52
<211> 112
<212> PRT
<213> Mus musculus

<400> 52

```
Asp Val Leu Met Thr Gln Thr Pro Leu Ser Leu Pro Val Ser Leu Gly
1               5               10              15

Asp Gln Ala Ser Ile Ser Cys Arg Ser Ser Arg Ser Ile Val His Ser
            20              25              30

Ser Gly Asn Thr Tyr Leu Glu Trp Tyr Leu Gln Lys Pro Gly Gln Ser
        35              40              45

Pro Lys Leu Leu Ile Tyr Lys Val Ser Asn Arg Phe Ser Gly Val Pro
    50              55              60

Asp Arg Phe Ser Gly Ser Gly Ser Gly Thr Asp Phe Thr Leu Asn Ile
65              70              75              80
```

```
Ser Arg Val Glu Ala Glu Asp Leu Gly Val Tyr Tyr Cys Phe Gln Asp
                85                  90                  95


Ser His Val Pro Phe Thr Phe Gly Thr Gly Thr Lys Leu Glu Ile Lys
                100                 105                 110
```

<210> 53
<211> 7
<212> PRT
<213> Mus musculus

<400> 53

```
Gly Phe Thr Phe Ser Ser Ser
1                   5
```

<210> 54
<211> 3
<212> PRT
<213> Mus musculus

<400> 54

```
Ala Thr Gly
1
```

<210> 55
<211> 8
<212> PRT
<213> Mus musculus

<400> 55

```
Tyr Pro His Tyr Tyr Ala Met Asp
1                   5
```

<210> 56
<211> 7
<212> PRT
<213> Mus musculus

<400> 56

```
Ser Glu Asn Ile Phe Ser Asn
1                   5
```

<210> 57
<211> 3
<212> PRT
<213> Mus musculus

<400> 57

```
Ser Ala Thr
1
```

<210> 58

<211> 6
<212> PRT
<213> Mus musculus

<400> 58

```
            Phe Tyr Lys Ile Pro Phe
            1               5
```

<210> 59
<211> 121
<212> PRT
<213> Mus musculus

<400> 59

```
Gln Gly Gln Met His Gln Ser Gly Ala Glu Leu Val Lys Pro Gly Ser
1               5                   10                  15

Ser Val Lys Leu Ser Cys Lys Thr Ser Gly Phe Thr Phe Ser Ser Ser
            20                  25                  30

Phe Ile Ser Trp Leu Lys Gln Lys Pro Gly Gln Ser Leu Glu Trp Ile
            35                  40                  45

Ala Trp Ile Tyr Ala Ala Thr Gly Ser Thr Ser Tyr Asn Gln Lys Phe
            50                  55                  60

Thr Asn Lys Ala Gln Leu Thr Val Asp Thr Ser Ser Ser Ala Ala Tyr
65                  70                  75                  80

Met Gln Phe Ser Ser Leu Thr Thr Glu Asp Ser Ala Ile Tyr Tyr Cys
                85                  90                  95

Ala Arg His Ala Gly Tyr Pro His Tyr Tyr Ala Met Asp Tyr Trp Gly
                100                 105                 110

Gln Gly Thr Ser Val Thr Val Ser Ser
                115                 120
```

<210> 60
<211> 107
<212> PRT
<213> Mus musculus

<400> 60

```
Asp Ile Gln Met Thr Gln Ser Pro Ala Ser Leu Ser Ala Ser Val Gly
1               5                   10                  15

Glu Thr Val Thr Ile Thr Cys Arg Ala Ser Glu Asn Ile Phe Ser Asn
```

73

```
                  20                      25                          30

        Leu Ala Trp Tyr Gln Gln Lys Gln Gly Lys Ser Pro Gln Leu Leu Val
                     35                  40                  45

        Tyr Ser Ala Thr Asn Leu Gly Asp Gly Val Pro Ser Arg Phe Ser Gly
                     50                  55                  60

        Ser Gly Ser Gly Thr Gln Phe Ser Leu Lys Ile Asn Ser Leu Gln Pro
        65                  70                  75                      80

        Glu Asp Phe Gly Asn Tyr Tyr Cys Gln His Phe Tyr Lys Ile Pro Phe
                         85                  90                  95

        Thr Phe Gly Thr Gly Thr Lys Leu Glu Ile Lys
                        100                 105
```

<210> 61
<211> 7
<212> PRT
<213> Mus musculus

<400> 61

```
                    Gly Phe Asn Ile Lys Asp Tyr
                    1               5
```

<210> 62
<211> 3
<212> PRT
<213> Mus musculus

<400> 62

```
                    Glu Asp Gly
                    1
```

<210> 63
<211> 8
<212> PRT
<213> Mus musculus

<400> 63

```
                    His Gly Tyr Val Gly Trp Phe Ala
                    1               5
```

<210> 64
<211> 8
<212> PRT
<213> Mus musculus

<400> 64

```
Ala Ser Glu Asn Val Asp Thr Tyr
1                   5
```

<210> 65
<211> 3
<212> PRT
<213> Mus musculus

<400> 65

```
                    Gly Ala Ser
                    1
```

<210> 66
<211> 6
<212> PRT
<213> Mus musculus

<400> 66

```
            Ser Tyr Ser Tyr Pro Trp
            1               5
```

<210> 67
<211> 119
<212> PRT
<213> Mus musculus

<400> 67

```
Ala Ser Glu Asn Val Asp Thr Tyr
```

```
Glu Val Gln Leu Gln Gln Ser Gly Ala Glu Pro Leu Lys Pro Gly Ala
1               5               10              15

Ser Val Lys Leu Thr Cys Thr Thr Ser Gly Phe Asn Ile Lys Asp Tyr
            20              25              30

Tyr Ile His Trp Val Lys Gln Arg Ser Asp Gln Gly Leu Glu Trp Ile
        35              40              45

Gly Arg Ile Asp Pro Glu Asp Gly Glu Leu Ile Tyr Ala Pro Lys Phe
    50              55              60

Gln Asp Lys Ala Thr Ile Thr Val Asp Thr Ser Ser Asn Ile Ala Tyr
65              70              75              80

Leu Gln Leu Asn Ser Leu Thr Ser Glu Asp Thr Ala Val Tyr Tyr Cys
                85              90              95

Ser Arg Asp His Gly Tyr Val Gly Trp Phe Ala Tyr Trp Gly Gln Gly
            100             105             110

Thr Leu Val Thr Val Ser Ala
            115
```

<210> 68
<211> 107
<212> PRT
<213> Mus musculus

<400> 68

```
Asn Val Val Met Thr Gln Ser Pro Lys Ser Met Ile Met Ser Val Gly
1               5               10                  15

Gln Arg Val Thr Leu Asn Cys Lys Ala Ser Glu Asn Val Asp Thr Tyr
            20              25              30

Val Ser Trp Tyr Gln Gln Lys Pro Glu Gln Ser Pro Lys Leu Leu Ile
        35              40              45

Tyr Gly Ala Ser Asn Arg Tyr Thr Gly Val Pro Asp Arg Phe Thr Gly
    50              55              60

Ser Arg Ser Ala Thr Asp Phe Thr Leu Thr Ile Ser Ser Val Gln Ala
65              70              75              80

Glu Asp Leu Ala Val Tyr Tyr Cys Gly Gln Ser Tyr Ser Tyr Pro Trp
            85              90              95

Thr Phe Gly Gly Gly Thr Lys Leu Glu Phe Arg
        100             105
```

<210> 69
<211> 219
<212> PRT
<213> Artificial

<220>
<223> an exemplary Pseudomonas exotoxin A variant 1(deimunized PE24 example)

<400> 69

```
Pro Thr Gly Ala Glu Phe Leu Gly Asp Gly Gly Asp Val Ser Phe Ser
1               5               10                  15

Thr Arg Gly Thr Gln Asn Trp Thr Val Glu Arg Leu Leu Gln Ala His
            20              25              30

Ala Gln Leu Glu Glu Arg Gly Tyr Val Phe Val Gly Tyr His Gly Thr
            35              40              45

Phe Leu Glu Ala Ala Gln Ser Ile Val Phe Gly Gly Val Ala Ala Arg
        50              55              60
```

```
Ser Gln Asp Leu Ala Ala Ile Trp Ala Gly Phe Tyr Ile Ala Gly Asp
65                  70              75                  80


Pro Ala Leu Ala Tyr Gly Tyr Ala Gln Asp Gln Glu Pro Asp Ala Ala
                85                  90                  95


Gly Arg Ile Arg Asn Gly Ala Leu Leu Arg Val Tyr Val Pro Ala Ser
            100                 105                 110


Ser Leu Pro Gly Phe Tyr Arg Thr Ser Leu Thr Leu Ala Ala Pro Glu
            115                 120                 125


Ala Ala Gly Glu Val Glu Arg Leu Ile Gly His Pro Leu Pro Leu Ala
    130                 135                 140


Leu Asp Ala Ile Thr Gly Pro Glu Glu Glu Gly Gly Arg Leu Glu Thr
145                 150                 155                 160


Ile Leu Gly Trp Pro Leu Ala Glu Arg Thr Val Val Ile Pro Ser Ala
                165                 170                 175


Ile Pro Thr Asp Pro Arg Asn Val Gly Gly Asp Leu Asp Pro Ser Ser
            180                 185                 190


Ile Pro Asp Lys Glu Gln Ala Ile Ser Ala Leu Pro Asp Tyr Ala Ser
            195                 200                 205


Gln Pro Gly Lys Pro Pro Arg Glu Asp Leu Lys
    210                 215
```

<210> 70
<211> 219
<212> PRT
<213> Artificial

<220>
<223> an exemplary Pseudomonas exotoxin A variant 2(deimunized PE24 example)

<400> 70

```
Pro Thr Gly Ala Glu Phe Leu Gly Asp Gly Gly Asp Val Ser Phe Ser
1               5                   10                  15


Thr Arg Gly Thr Gln Asn Trp Thr Val Glu Arg Leu Leu Gln Ala His
            20                  25                  30


Ala Gln Leu Glu Glu Arg Gly Tyr Val Phe Val Gly Tyr His Gly Thr
            35                  40                  45
```

```
Ala Leu Glu Ala Ala Gln Ser Ile Val Phe Gly Gly Val Arg Ala Arg
    50                  55                  60

Ser Gln Asp Leu Arg Ala Ile Trp Arg Gly Phe Tyr Ile Ala Gly Asp
65                  70                  75                  80

Pro Ala His Ala Tyr Gly Tyr Ala Gln Asp Gln Glu Pro Asp Ala Arg
                85                  90                  95

Gly Arg Ile Ala Asn Gly Ala Leu Leu Arg Val Tyr Val Pro Ala Ser
            100                 105                 110

Ser Leu Pro Gly Phe Tyr Arg Thr Ser Leu Thr Leu Ala Ala Pro Glu
            115                 120                 125

Ala Ala Gly Glu Val Glu Arg Leu Ile Gly His Pro Leu Pro Leu Arg
            130                 135                 140

Leu Asp Ala Ile Thr Gly Pro Glu Glu Glu Gly Gly Arg Glu Glu Thr
145                 150                 155                 160

Ile Leu Gly Trp Pro Leu Ala Glu Arg Thr Val Val Ile Pro Ser Ala
                165                 170                 175

Ile Pro Thr Asp Pro Arg Asn Val Gly Gly Asp Leu Asp Pro Ser Ser
            180                 185                 190

Ile Pro Asp Lys Glu Gln Ala Ile Ser Ala Leu Pro Asp Tyr Ala Ser
            195                 200                 205

Gln Pro Gly Lys Pro Pro Arg Glu Asp Leu Lys
    210                 215
```

<210> 71
<211> 107
<212> PRT
<213> Homo Sapiens

<400> 71

```
Arg Thr Val Ala Ala Pro Ser Val Phe Ile Phe Pro Pro Ser Asp Glu
1                   5                   10                  15

Gln Leu Lys Ser Gly Thr Ala Ser Val Val Cys Leu Leu Asn Asn Phe
            20                  25                  30

Tyr Pro Arg Glu Ala Lys Val Gln Trp Lys Val Asp Asn Ala Leu Gln
            35                  40                  45
```

```
Ser Gly Asn Ser Gln Glu Ser Val Thr Glu Gln Asp Ser Lys Asp Ser
    50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                    85                  90                  95

Pro Val Thr Lys Ser Phe Asn Arg Gly Glu Cys
              100                 105
```

<210> 72
<211> 105
<212> PRT
<213> Homo Sapiens

<400> 72

```
Gln Pro Lys Ala Ala Pro Ser Val Thr Leu Phe Pro Pro Ser Ser Glu
1               5                   10                  15

Glu Leu Gln Ala Asn Lys Ala Thr Leu Val Cys Leu Ile Ser Asp Phe
              20                  25                  30

Tyr Pro Gly Ala Val Thr Val Ala Trp Lys Ala Asp Ser Ser Pro Val
          35                  40                  45

Lys Ala Gly Val Glu Thr Thr Thr Pro Ser Lys Gln Ser Asn Asn Lys
    50                  55                  60

Tyr Ala Ala Ser Ser Tyr Leu Ser Leu Thr Pro Glu Gln Trp Lys Ser
65                  70                  75                  80

His Arg Ser Tyr Ser Cys Gln Val Thr His Glu Gly Ser Thr Val Glu
                85                  90                  95

Lys Thr Val Ala Pro Thr Glu Cys Ser
              100                 105
```

<210> 73
<211> 330
<212> PRT
<213> Homo Sapiens

<400> 73

```
Ala Ser Thr Lys Gly Pro Ser Val Phe Pro Leu Ala Pro Ser Ser Lys
1               5                   10                  15
```

```
Ser Thr Ser Gly Gly Thr Ala Ala Leu Gly Cys Leu Val Lys Asp Tyr
            20              25                  30

Phe Pro Glu Pro Val Thr Val Ser Trp Asn Ser Gly Ala Leu Thr Ser
            35              40                  45

Gly Val His Thr Phe Pro Ala Val Leu Gln Ser Ser Gly Leu Tyr Ser
            50              55                  60

Leu Ser Ser Val Val Thr Val Pro Ser Ser Ser Leu Gly Thr Gln Thr
65              70                  75                  80

Tyr Ile Cys Asn Val Asn His Lys Pro Ser Asn Thr Lys Val Asp Lys
            85                  90                  95

Lys Val Glu Pro Lys Ser Cys Asp Lys Thr His Thr Cys Pro Pro Cys
            100             105                 110

Pro Ala Pro Glu Leu Leu Gly Gly Pro Ser Val Phe Leu Phe Pro Pro
            115             120                 125

Lys Pro Lys Asp Thr Leu Met Ile Ser Arg Thr Pro Glu Val Thr Cys
            130             135                 140

Val Val Val Asp Val Ser His Glu Asp Pro Glu Val Lys Phe Asn Trp
145             150                 155                 160

Tyr Val Asp Gly Val Glu Val His Asn Ala Lys Thr Lys Pro Arg Glu
                165             170                 175

Glu Gln Tyr Asn Ser Thr Tyr Arg Val Val Ser Val Leu Thr Val Leu
            180             185                 190

His Gln Asp Trp Leu Asn Gly Lys Glu Tyr Lys Cys Lys Val Ser Asn
            195             200                 205

Lys Ala Leu Pro Ala Pro Ile Glu Lys Thr Ile Ser Lys Ala Lys Gly
            210             215                 220

Gln Pro Arg Glu Pro Gln Val Tyr Thr Leu Pro Pro Ser Arg Asp Glu
225             230                 235                 240

Leu Thr Lys Asn Gln Val Ser Leu Thr Cys Leu Val Lys Gly Phe Tyr
            245                 250                 255

Pro Ser Asp Ile Ala Val Glu Trp Glu Ser Asn Gly Gln Pro Glu Asn
            260             265                 270
```

```
Asn Tyr Lys Thr Thr Pro Pro Val Leu Asp Ser Asp Gly Ser Phe Phe
        275             280             285

Leu Tyr Ser Lys Leu Thr Val Asp Lys Ser Arg Trp Gln Gln Gly Asn
        290             295             300

Val Phe Ser Cys Ser Val Met His Glu Ala Leu His Asn His Tyr Thr
305             310             315             320

Gln Lys Ser Leu Ser Leu Ser Pro Gly Lys
        325             330
```

<210> 74
<211> 330
<212> PRT
<213> homo sapiens

<400> 74

```
Ala Ser Thr Lys Gly Pro Ser Val Phe Pro Leu Ala Pro Ser Ser Lys
1           5               10              15

Ser Thr Ser Gly Gly Thr Ala Ala Leu Gly Cys Leu Val Lys Asp Tyr
        20              25              30

Phe Pro Glu Pro Val Thr Val Ser Trp Asn Ser Gly Ala Leu Thr Ser
        35              40              45

Gly Val His Thr Phe Pro Ala Val Leu Gln Ser Ser Gly Leu Tyr Ser
        50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
            85              90              95

Lys Val Glu Pro Lys Ser Cys Asp Lys Thr His Thr Cys Pro Pro Cys
        100             105             110

Pro Ala Pro Glu Ala Ala Gly Gly Pro Ser Val Phe Leu Phe Pro Pro
        115             120             125

Lys Pro Lys Asp Thr Leu Met Ile Ser Arg Thr Pro Glu Val Thr Cys
        130             135             140

Val Val Val Asp Val Ser His Glu Asp Pro Glu Val Lys Phe Asn Trp
145             150             155             160
```

```
Tyr Val Asp Gly Val Glu Val His Asn Ala Lys Thr Lys Pro Arg Glu
                165             170             175

Glu Gln Tyr Asn Ser Thr Tyr Arg Val Val Ser Val Leu Thr Val Leu
            180             185             190

His Gln Asp Trp Leu Asn Gly Lys Glu Tyr Lys Cys Lys Val Ser Asn
            195             200             205

Lys Ala Leu Pro Ala Pro Ile Glu Lys Thr Ile Ser Lys Ala Lys Gly
    210             215             220

Gln Pro Arg Glu Pro Gln Val Tyr Thr Leu Pro Pro Ser Arg Asp Glu
225             230             235             240

Leu Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn
            260             265             270

Asn Tyr Lys Thr Thr Pro Pro Val Leu Asp Ser Asp Gly Ser Phe Phe
            275             280             285

Leu Tyr Ser Lys Leu Thr Val Asp Lys Ser Arg Trp Gln Gln Gly Asn
    290             295             300

Val Phe Ser Cys Ser Val Met His Glu Ala Leu His Asn His Tyr Thr
305             310             315             320

Gln Lys Ser Leu Ser Leu Ser Pro Gly Lys
            325             330
```

<210> 75
<211> 330
<212> PRT
<213> homo sapiens

<400> 75

```
Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
            20              25              30

Phe Pro Glu Pro Val Thr Val Ser Trp Asn Ser Gly Ala Leu Thr Ser
            35              40              45
```

```
Gly Val His Thr Phe Pro Ala Val Leu Gln Ser Ser Gly Leu Tyr Ser
    50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
            85              90              95

Lys Val Glu Pro Lys Ser Cys Asp Lys Thr His Thr Cys Pro Pro Cys
        100             105             110

Pro Ala Pro Glu Ala Ala Gly Gly Pro Ser Val Phe Leu Phe Pro Pro
        115             120             125

Lys Pro Lys Asp Thr Leu Met Ile Ser Arg Thr Pro Glu Val Thr Cys
    130             135             140

Val Val Val Asp Val Ser His Glu Asp Pro Glu Val Lys Phe Asn Trp
145             150             155             160

Tyr Val Asp Gly Val Glu Val His Asn Ala Lys Thr Lys Pro Arg Glu
            165             170             175

Glu Gln Tyr Asn Ser Thr Tyr Arg Val Val Ser Val Leu Thr Val Leu
            180             185             190

His Gln Asp Trp Leu Asn Gly Lys Glu Tyr Lys Cys Lys Val Ser Asn
        195             200             205

Lys Ala Leu Gly Ala Pro Ile Glu Lys Thr Ile Ser Lys Ala Lys Gly
    210             215             220

Gln Pro Arg Glu Pro Gln Val Tyr Thr Leu Pro Pro Ser Arg Asp Glu
225             230             235             240

Leu Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn
            260             265             270

Asn Tyr Lys Thr Thr Pro Pro Val Leu Asp Ser Asp Gly Ser Phe Phe
        275             280             285

Leu Tyr Ser Lys Leu Thr Val Asp Lys Ser Arg Trp Gln Gln Gly Asn
    290             295             300
```

84

```
Val Phe Ser Cys Ser Val Met His Glu Ala Leu His Asn His Tyr Thr
305             310             315             320

Gln Lys Ser Leu Ser Leu Ser Pro Gly Lys
            325             330
```

<210> 76
<211> 327
<212> PRT
<213> Homo Sapiens

<400> 76

```
Ala Ser Thr Lys Gly Pro Ser Val Phe Pro Leu Ala Pro Cys Ser Arg
1               5               10              15

Ser Thr Ser Glu Ser Thr Ala Ala Leu Gly Cys Leu Val Lys Asp Tyr
            20              25              30

Phe Pro Glu Pro Val Thr Val Ser Trp Asn Ser Gly Ala Leu Thr Ser
            35              40              45

Gly Val His Thr Phe Pro Ala Val Leu Gln Ser Ser Gly Leu Tyr Ser
        50              55              60

Leu Ser Ser Val Val Thr Val Pro Ser Ser Ser Leu Gly Thr Lys Thr
65              70              75              80

Tyr Thr Cys Asn Val Asp His Lys Pro Ser Asn Thr Lys Val Asp Lys
                85              90              95

Arg Val Glu Ser Lys Tyr Gly Pro Pro Cys Pro Ser Cys Pro Ala Pro
            100             105             110

Glu Phe Leu Gly Gly Pro Ser Val Phe Leu Phe Pro Pro Lys Pro Lys
            115             120             125

Asp Thr Leu Met Ile Ser Arg Thr Pro Glu Val Thr Cys Val Val Val
        130             135             140

Asp Val Ser Gln Glu Asp Pro Glu Val Gln Phe Asn Trp Tyr Val Asp
145             150             155             160

Gly Val Glu Val His Asn Ala Lys Thr Lys Pro Arg Glu Glu Gln Phe
            165             170             175

Asn Ser Thr Tyr Arg Val Val Ser Val Leu Thr Val Leu His Gln Asp
            180             185             190
```

```
Trp Leu Asn Gly Lys Glu Tyr Lys Cys Lys Val Ser Asn Lys Gly Leu
        195             200             205

Pro Ser Ser Ile Glu Lys Thr Ile Ser Lys Ala Lys Gly Gln Pro Arg
        210             215             220

Glu Pro Gln Val Tyr Thr Leu Pro Pro Ser Gln Glu Glu Met Thr Lys
225             230             235             240

Asn Gln Val Ser Leu Thr Cys Leu Val Lys Gly Phe Tyr Pro Ser Asp
                245             250             255

Ile Ala Val Glu Trp Glu Ser Asn Gly Gln Pro Glu Asn Asn Tyr Lys
            260             265             270

Thr Thr Pro Pro Val Leu Asp Ser Asp Gly Ser Phe Phe Leu Tyr Ser
        275             280             285

Arg Leu Thr Val Asp Lys Ser Arg Trp Gln Glu Gly Asn Val Phe Ser
    290             295             300

Cys Ser Val Met His Glu Ala Leu His Asn His Tyr Thr Gln Lys Ser
305             310             315             320

Leu Ser Leu Ser Leu Gly Lys
                325
```

<210> 77
<211> 280
<212> PRT
<213> homo sapiens

<400> 77

```
Ser Glu Val Glu Tyr Arg Ala Glu Val Gly Gln Asn Ala Tyr Leu Pro
1               5               10              15

Cys Phe Tyr Thr Pro Ala Ala Pro Gly Asn Leu Val Pro Val Cys Trp
            20              25              30

Gly Lys Gly Ala Cys Pro Val Phe Glu Cys Gly Asn Val Val Leu Arg
            35              40              45

Thr Asp Glu Arg Asp Val Asn Tyr Trp Thr Ser Arg Tyr Trp Leu Asn
    50              55              60

Gly Asp Phe Arg Lys Gly Asp Val Ser Leu Thr Ile Glu Asn Val Thr
65              70              75              80
```

```
Leu Ala Asp Ser Gly Ile Tyr Cys Cys Arg Ile Gln Ile Pro Gly Ile
            85              90              95

Met Asn Asp Glu Lys Phe Asn Leu Lys Leu Val Ile Lys Pro Ala Lys
            100             105             110

Val Thr Pro Ala Pro Thr Arg Gln Arg Asp Phe Thr Ala Ala Phe Pro
            115             120             125

Arg Met Leu Thr Thr Arg Gly His Gly Pro Ala Glu Thr Gln Thr Leu
    130             135             140

Gly Ser Leu Pro Asp Ile Asn Leu Thr Gln Ile Ser Thr Leu Ala Asn
145             150             155             160

Glu Leu Arg Asp Ser Arg Leu Ala Asn Asp Leu Arg Asp Ser Gly Ala
            165             170             175

Thr Ile Arg Ile Gly Ile Tyr Ile Gly Ala Gly Ile Cys Ala Gly Leu
            180             185             190

Ala Leu Ala Leu Ile Phe Gly Ala Leu Ile Phe Lys Trp Tyr Ser His
            195             200             205

Ser Lys Glu Lys Ile Gln Asn Leu Ser Leu Ile Ser Leu Ala Asn Leu
    210             215             220

Pro Pro Ser Gly Leu Ala Asn Ala Val Ala Glu Gly Ile Arg Ser Glu
225             230             235             240

Glu Asn Ile Tyr Thr Ile Glu Glu Asn Val Tyr Glu Val Glu Glu Pro
            245             250             255

Asn Glu Tyr Tyr Cys Tyr Val Ser Ser Arg Gln Gln Pro Ser Gln Pro
            260             265             270

Leu Gly Cys Arg Phe Ala Met Pro
            275             280
```

<210> 78
<211> 181
<212> PRT
<213> homo sapiens

<400> 78

```
Ser Glu Val Glu Tyr Arg Ala Glu Val Gly Gln Asn Ala Tyr Leu Pro
1               5               10              15
```

```
Cys Phe Tyr Thr Pro Ala Ala Pro Gly Asn Leu Val Pro Val Cys Trp
            20              25              30

Gly Lys Gly Ala Cys Pro Val Phe Glu Cys Gly Asn Val Val Leu Arg
        35              40              45

Thr Asp Glu Arg Asp Val Asn Tyr Trp Thr Ser Arg Tyr Trp Leu Asn
        50              55              60

Gly Asp Phe Arg Lys Gly Asp Val Ser Leu Thr Ile Glu Asn Val Thr
65              70              75              80

Leu Ala Asp Ser Gly Ile Tyr Cys Cys Arg Ile Gln Ile Pro Gly Ile
            85              90              95

Met Asn Asp Glu Lys Phe Asn Leu Lys Leu Val Ile Lys Pro Ala Lys
            100             105             110

Val Thr Pro Ala Pro Thr Arg Gln Arg Asp Phe Thr Ala Ala Phe Pro
            115             120             125

Arg Met Leu Thr Thr Arg Gly His Gly Pro Ala Glu Thr Gln Thr Leu
    130             135             140

Gly Ser Leu Pro Asp Ile Asn Leu Thr Gln Ile Ser Thr Leu Ala Asn
145             150             155             160

Glu Leu Arg Asp Ser Arg Leu Ala Asn Asp Leu Arg Asp Ser Gly Ala
            165             170             175

Thr Ile Arg Ile Gly
            180
```

<210> 79
<211> 120
<212> PRT
<213> Artificial

<220>
<223> VH humanized version of Tim3_0016 variant (0018) (= Tim3-0433)

<400> 79

```
Gln Ile Thr Leu Lys Glu Ser Gly Pro Thr Leu Val Lys Pro Thr Gln
1               5               10              15

Thr Leu Thr Leu Thr Cys Thr Phe Ser Gly Phe Ser Leu Ser Thr Ser
            20              25              30
```

88

```
Gly Met Ser Val Gly Trp Ile Arg Gln Pro Pro Gly Lys Gly Leu Glu
        35              40              45

Trp Leu Ala His Ile Trp Leu Asn Asp Asp Val Phe Phe Asn Pro Ala
        50              55              60

Leu Lys Ser Arg Leu Thr Ile Thr Lys Asp Thr Ser Lys Asn Gln Val
    65              70              75              80

Val Leu Thr Met Thr Asn Met Asp Pro Val Asp Thr Ala Thr Tyr Tyr
                85              90              95

Cys Val Arg Ala Asn Gly Tyr Leu Tyr Ala Leu Asp Tyr Trp Gly Gln
            100             105             110

Gly Thr Leu Val Thr Val Ser Ser
        115             120
```

<210> 80
<211> 106
<212> PRT
<213> Artificial

<220>
<223> VL humanized version of Tim3_0016 variant (0018) (= Tim3-0433)

<400> 80

```
Glu Thr Thr Leu Thr Gln Ser Pro Ala Phe Met Ser Ala Thr Pro Gly
1               5               10              15

Asp Lys Val Asn Ile Ala Cys Ser Ala Ser Ser Ser Val Ser Tyr Thr
        20              25              30

Gln Trp Tyr Gln Gln Lys Pro Gly Glu Ala Pro Lys Leu Trp Ile Tyr
        35              40              45

Asp Ala Phe Lys Leu Ala Pro Gly Ile Pro Pro Arg Phe Ser Gly Ser
        50              55              60

Gly Tyr Gly Thr Asp Phe Thr Leu Thr Ile Asn Asn Ile Glu Ser Glu
    65              70              75              80

Asp Ala Ala Tyr Tyr Phe Cys His Gln Trp Ser Ser Tyr Pro Trp Thr
                85              90              95

Phe Gly Gln Gly Thr Lys Leu Glu Ile Lys
            100             105
```

<210> 81
<211> 120
<212> PRT
<213> Artificial

<220>
<223> VH humanized version of Tim3_0016 variant (0018) (= Tim3-0434)

<400> 81

```
Gln Ile Thr Leu Lys Glu Ser Gly Pro Thr Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Met Ser Val Gly Trp Ile Arg Gln Pro Pro Gly Lys Gly Leu Glu
            35                  40                  45

Trp Leu Ala His Ile Trp Leu Asn Asp Asp Val Phe Phe Asn Pro Ala
        50                  55                  60

Leu Lys Ser Arg Leu Thr Ile Thr Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95

Cys Val Arg Ala Asn Gly Tyr Leu Tyr Ala Leu Asp Tyr Trp Gly Gln
            100                 105                 110

Gly Thr Leu Val Thr Val Ser Ser
            115                 120
```

<210> 82
<211> 106
<212> PRT
<213> Artificial

<220>
<223> VL humanized version of Tim3_0016 variant (0018) (= Tim3-0434)

<400> 82

```
Asp Ile Gln Leu Thr Gln Ser Pro Ser Phe Leu Ser Ala Ser Val Gly
1               5               10                  15

Asp Arg Val Thr Ile Thr Cys Ser Ala Ser Ser Val Ser Tyr Thr
            20              25              30

Gln Trp Tyr Gln Gln Lys Pro Gly Lys Ala Pro Lys Leu Trp Ile Tyr
        35              40                  45

Asp Ala Phe Lys Leu Ala Pro Gly Val Pro Ser Arg Phe Ser Gly Ser
    50              55              60

Gly Ser Gly Thr Glu Phe Thr Leu Thr Ile Ser Ser Leu Gln Pro Glu
65              70              75                  80

Asp Phe Ala Thr Tyr Phe Cys His Gln Trp Ser Ser Tyr Pro Trp Thr
            85              90              95

Phe Gly Gln Gly Thr Lys Leu Glu Ile Lys
            100             105
```

<210> 83
<211> 119
<212> PRT
<213> Artificial

<220>
<223> VH humanized version of Tim3-0028 (= Tim3-0438)

<400> 83

Glu Val Gln Leu Val Glu Ser Gly Gly Gly Leu Val Gln Pro Gly Gly
1               5               10              15

Ser Leu Arg Leu Ser Cys Ala Ala Ser Gly Phe Asn Ile Lys Thr Thr
        20              25              30

Tyr Met His Trp Val Arg Gln Ala Pro Gly Lys Gly Leu Glu Trp Val
        35              40              45

Gly Arg Ile Asp Pro Ala Asp Asp Asn Thr Lys Tyr Ala Pro Lys Phe
    50              55              60

Gln Gly Lys Ala Thr Ile Ser Ala Asp Thr Ser Lys Asn Thr Ala Tyr
65              70              75              80

Leu Gln Met Asn Ser Leu Arg Ala Glu Asp Thr Ala Val Tyr Tyr Cys
            85              90              95

Val Arg Asp Phe Gly Tyr Val Ala Trp Phe Ala Tyr Trp Gly Gln Gly
        100             105             110

Thr Leu Val Thr Val Ser Ser
        115

<210> 84
<211> 107
<212> PRT
<213> Artificial

<220>
<223> VL humanized version of Tim3-0028 (= Tim3-0438)

<400> 84

92

```
Asp Ile Val Met Thr Gln Ser Pro Leu Ser Leu Pro Val Thr Pro Gly
1               5               10              15
```

```
Glu Pro Ala Ser Ile Ser Cys Arg Ala Ser Gln Ser Val Asp Asn Tyr
            20              25              30
```

```
Val Ala Trp Tyr Leu Gln Lys Pro Gly Gln Ser Pro Gln Leu Leu Ile
            35              40              45
```

```
Tyr Tyr Ala Ser Asn Arg Tyr Ile Gly Val Pro Asp Arg Phe Ser Gly
    50              55              60
```

```
Ser Gly Ser Gly Thr Asp Phe Thr Leu Lys Ile Ser Arg Val Glu Ala
65              70              75              80
```

```
Glu Asp Val Gly Val Tyr Tyr Cys Gln Gln His Tyr Ser Ser Pro Tyr
            85              90              95
```

```
Thr Phe Gly Gln Gly Thr Lys Val Glu Ile Lys
        100             105
```

<210> 85
<211> 119
<212> PRT
<213> Artificial

<220>
<223> VH humanized version of Tim3-0028 (= Tim3-0443)

<400> 85

```
Glu Val Gln Leu Val Glu Ser Gly Gly Gly Leu Val Gln Pro Gly Gly
1               5               10              15
```

```
Ser Leu Arg Leu Ser Cys Ala Ala Ser Gly Phe Asn Ile Lys Thr Thr
            20              25              30
```

```
Tyr Met His Trp Val Arg Gln Ala Pro Gly Lys Gly Leu Glu Trp Val
            35              40              45
```

```
Gly Arg Ile Asp Pro Ala Asp Asp Asn Thr Lys Tyr Ala Pro Lys Phe
    50              55              60
```

```
Gln Gly Lys Ala Thr Ile Ser Ala Asp Thr Ser Lys Asn Thr Ala Tyr
65              70              75              80
```

93

```
        Leu Gln Met Asn Ser Leu Arg Ala Glu Asp Thr Ala Val Tyr Tyr Cys
                    85                  90                  95


        Val Arg Asp Phe Gly Tyr Val Ala Trp Phe Ala Tyr Trp Gly Gln Gly
                    100                 105                 110


        Thr Leu Val Thr Phe Ser Ser
                    115
```

<210> 86
<211> 107
<212> PRT
<213> Artificial

<220>
<223> VL humanized version of Tim3-0028 (= Tim3-0443)

<400> 86

```
        Asp Ile Val Met Thr Gln Ser Pro Leu Ser Leu Pro Val Thr Pro Gly
        1               5                   10                  15


        Glu Pro Ala Ser Ile Ser Cys Arg Ala Ser Gln Ser Val Asp Asn Tyr
                    20                  25                  30


        Val Ala Trp Tyr Leu Gln Lys Pro Gly Gln Ser Pro Gln Leu Leu Ile
                    35                  40                  45


        Tyr Tyr Ala Ser Asn Arg Tyr Ile Gly Val Pro Asp Arg Phe Ser Gly
            50                  55                  60


        Ser Gly Ser Gly Thr Asp Phe Thr Leu Lys Ile Ser Arg Val Glu Ala
        65                  70                  75                  80


        Glu Asp Val Gly Val Tyr Tyr Cys Gln Gln His Tyr Ser Ser Pro Tyr
                    85                  90                  95


        Thr Phe Gly Gln Gly Thr Lys Val Glu Ile Lys
                    100                 105
```

## Description

### FIELD OF THE INVENTION

The present invention relates to anti-TIM3 antibodies and methods of using the same.

### BACKGROUND

TIM3 is a human protein which belongs to the immunoglobulin superfamily, and TIM family of proteins. In humans, as similar to mice, TIM-3 is expressed on T-cells as well as phagocytic cells such as macrophages and dendritic cells. Binding of TIM-3 to a protein ligand (e.g., galectin-9) can inhibit the Th1 response via mechanism of apoptosis induction, and therefore lead to such as induction of peripheral tolerance. The reduction in expression of human TIM-3 with siRNA or the inhibition of human TIM-3 by blocking-antibody increased the secretion of interferon alpha from CD4 positive T-cells, supporting the inhibitory role of TIM-3 in human T cells. In phagocytes, TIM-3 also functions as a receptor for recognizing the apoptosis cells. Analysis of clinical samples from autoimmune disease patients showed no expression of TIM-3 in CD4 positive cells. In particular, in T cell clones derived from the cerebrospinal fluid of patients with multiple sclerosis, the expression level of TIM-3 was lower and the secretion level of IFN-gamma was higher than those of clones derived from normal healthy persons (Koguchi K et al., J Exp Med. 203 (2006) 1413-1418).

There are reports on relation of TIM-3 with allergic diseases or asthma (WO96/27603 and WO2003/063792).

According to the microarray analysis of hematopoietic stem cells from acute myeloid leukemia (hereinafter referred to as "AML") patients and normal hematopoietic stem cells, TIM-3 is expressed on AML stem cells and therefore the analysis suggested involvement of TIM-3 in hematological malignancy (Majeti R et al., PNAS, 106 (2009) 3396-3401 and WO2009/091547).

Examples of the anti-TIM-3 monoclonal antibodies include anti-human TIM- 3 rat monoclonal antibody (Clone 344823, manufactured by R&D Systems) and anti-human TIM-3 mouse monoclonal antibody (Clone F38-2E2, manufactured by R&D Systems). WO2013/06490 relates to anti-TIM-3 antibodies which show rapid internalization and immunoconjugates thereof for treating cancer and reducing inflammation. US2012/189617 relates to anti-TIM-3 antibodies which exhibit higher effector activity such as an antibody-dependent cellular cytotoxicity (ADCC activity) for diseases relating to a human TIM-3 expressing cell.

### SUMMARY

The invention is defined in the appended claims and any other aspects set forth herein not falling within the scope of the claims are for information only.

The invention provides anti-TIM3 antibodies and methods of using the same.

The invention provides an isolated antibody that binds to human TIM3, wherein the antibody comprises (a) a VH domain comprising (i) HVR-H1 comprising the amino acid sequence of SEQ ID NO:37, (ii) HVR-H2 comprising the amino acid sequence of SEQ ID NO:38, and (iii) HVR-H3 comprising an amino acid sequence selected from SEQ ID NO:39; and (b) a VL domain comprising (i) HVR-L1 comprising the amino acid sequence of SEQ ID NO:40; (ii) HVR-L2 comprising the amino acid sequence of SEQ ID NO:41 and (iii) HVR-L3 comprising the amino acid sequence of SEQ ID NO:42.

The invention further provides an isolated antibody that binds to human TIM3, wherein the antibody comprises

i) a VH sequence of SEQ ID NO:83 and a VL sequence of SEQ ID NO:84, or

ii) a VH sequence of SEQ ID NO:85 and a VL sequence of SEQ ID NO:86.

In one embodiment the anti-TIM3 antibody according to the invention is a full length IgG1 antibody.

In one embodiment the anti-TIM3 antibody according to the invention is a full length IgG1 antibody with mutations L234A, L235A and P329G (numbering according to the EU index of Kabat).

Another aspect of the invention is an isolated nucleic acid encoding the antibody according to the invention.

Another aspect of the invention is an immunoconjugate comprising the antibody according to the invention and a cytotoxic agent.

In one preferred embodiment of the invention is such an immunoconjugate wherein the cytotoxic agent is Pseudomonas Exotoxin A or an Amatoxin.

Another aspect of the invention is a pharmaceutical formulation comprising the antibody according to the invention or the immunoconjugate according to the invention and a pharmaceutically acceptable carrier.

Another aspect of the invention is an antibody according to the invention or an immunoconjugate according to the invention for use as a medicament.

Another aspect of the invention is an antibody according to the invention or the immunoconjugate according to the invention for use in treating cancer.

Another aspect of the invention is the use of an antibody according to the invention or the immunoconjugate according to the invention for use in the manufacture of a medicament.

Another aspect of the invention is the use of an antibody according to the invention or the immunoconjugate according to the invention for use in the manufacture of a medicament for treatment of cancer.

Another aspect of the invention is a method of treating an individual having cancer comprising administering to the individual an effective amount of an antibody according to the invention or the immunoconjugate according to the invention.

The anti-TIM3 antibodies of the present invention show highly valuable properties like a rapid and strong internalization on TIM3-expressing cancer cells, a strong cytotoxic activity as immunoconjugate (e.g. when conjugated with Pseudomonas exotoxins or amatoxins).They are therefore useful therapeutics for the treatment of different cancers, especially blood tumors, like leukemias and lymphomas. Furthermore they show a strong immunestimulatory cytokine release in a Mixed Lymphocyte Reaction (MLR) and are therefore useful as immunestimulatory cancer therapy. In addition humanized antibody versions have improved binding and binding specificity to CD4 Tcells when compared to the parental antibodies.

## BRIEF DESCRIPTION OF THE FIGURES

FIG 1A: Time dependent FACS based internalization of anti-TIM3 antibody Tim3_0022 (abbreviated as <TIM-3> Ab(022)) internalized into rec CHOK1 cells expressing huTIM-3 after incubation at 37°C.

FIG 1B: Results from the FACS based internalization assay show that Fab fragment of anti-TIM3 antibody Tim3_0022 (abbreviated as <TIM-3> Ab(022)) internalized into rec CHOK1 cells expressing huTIM-3 after incubation at 37°C with similar kinetic as full IgG format.

FIG. 2A: Binding of anti-TIM3 antibodies to RPMI-8226 cells (antibody designation clone 0016 refers to antibody Tim3_0016, clone 0016 refers to antibody Tim3_0016 variant (antibody Tim3_0018), clone 0022 refers to antibody Tim3_00122, etc.).

FIG. 2B: Binding of anti-TIM3 antibodies to Pfeiffer cells (antibody designation clone 0016 refers to antibody Tim3_0016, clone 0016 refers to antibody Tim3_0016 variant (antibody Tim3_0018), clone 0022 refers to antibody Tim3_00122, etc.).

FIG 3: expression level of TIM-3 on different patient AML cell samples by FACS using anti-TIM-3 mAbs.

FIG 4: Direct omparison of binding of TIM3 antibodies to different peripheral blood mononuclear cells (Monocytes, NK cells, T cells, CD4 T cells):

4A: % positive cells to which Tim3_0016 variant (antibody Tim3_0018) and humanized versions are binding to.
4B: Mean fluorescence inensity- binding of Tim3_0016 variant (antibody Tim3_0018) and humanized versions to different peripheral blood mononuclear cells.
4C: % positive cells to which Tim3_0028 and chimeric and humanized versions are binding to.
4D: Mean fluorescence inensity- binding of Tim3_0028 and chimeric and humanized versions to different peripheral blood mononuclear cells.

## DETAILED DESCRIPTION OF EMBODIMENTS OF THE INVENTION

## I. DEFINITIONS

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence. (define germlines if appropriate)

The terms "anti-TIM3 antibody" and "an antibody that binds to TIM3" refer to an antibody that is capable of binding TIM3 with sufficient affinity such that the antibody is useful as a diagnostic and/or therapeutic agent in targeting TIM3. In one embodiment, the extent of binding of an anti-TIM3 antibody to an unrelated, non-TIM3 protein is less than about 10% of the binding of the antibody to TIM3 as measured, e.g., by a Surface Plasmon Resonance assay (e.g. BIACORE). In certain embodiments, an antigen binding protein that binds to human TIM3 has a KD value of the binding affinity for binding to human TIM3 of $\leq 1\ \mu M$, $\leq 100$ nM, $\leq 10$ nM, $\leq 1$ nM, $\leq 0.1$ nM, $\leq 0.01$ nM, or $\leq 0.001$ nM (e.g. $10^{-8}$ M or less, e.g. from $10^{-8}$ M to $10^{-13}$ M, e.g., from $10^{-9}$ M to $10^{-13}$ M). In one preferred embodiment the respective KD value of the binding affinities is determined in a Surface Plasmon Resonance assay using the Extracellular domain (ECD) of human TIM3 (TIM3 -ECD) for the TIM3 binding affinity.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')$_2$; diabodies; linear antibodies; single-chain antibody molecules (e.g. scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG$_1$, IgG$_2$, IgG$_3$, IgG$_4$, IgA$_1$, and IgA$_2$. The heavy chain constant domains that correspond to the different classes of immunoglobulins are called $\alpha$, $\delta$, $\varepsilon$, $\gamma$, and $\mu$, respectively.

The term "cytotoxic agent" as used herein refers to a substance that inhibits or prevents a cellular function and/or causes cell death or destruction. Cytotoxic agents include, but are not limited to, radioactive isotopes (e.g., At211, 1131, 1125, Y90, Re186, Re188, Sm153, Bi212, P32, Pb212 and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

In one preferred embodiment the "cytotoxic agent" is a Pseudomonas exotoxin A (or variants thereof) WO2005052006, WO2007016150, WO2007014743, WO2007031741, WO200932954, WO201132022, WO2012/154530, and WO 2012/170617, Liu W, et al, PNAS 109 (2012) 11782-11787, Mazor R, et al PNAS 111 (2014) 8571-8576 and Alewine C, et al, Mol Cancer Ther. (2014) 2653-61. In one preferred embodiment the "Pseudomonas exotoxin A" comprises the amino acid sequences of SEQ ID NO:69 or comprises the amino acid sequences of SEQ ID NO:70 (their preparation is also described in Mazor R, et al PNAS 111 (2014) 8571-8576 and Alewine C, et al, Mol Cancer Ther. (2014) 2653-61).

In another preferred embodiment the "cytotoxic agent" is an amatoxin (or variants thereof) as described e.g. WO2010/115630, WO2010/115629, WO2012/119787, WO2012/041504, and WO2014135282 with preferred variants described in WO2012/041504 (e.g. conjugated via the 6' C-atom of amatoxin amino acid 4, particularly via an oxygen atom bound to the 6' C-atom of amatoxin amino acid, and wherein the TIM3 antibody is connected by a linker via a urea moiety) and WO2014135282.

An "effective amount" of an agent, e.g., a pharmaceutical formulation, refers to an amount effective, at dosages and for periods of time necessary, to achieve the desired therapeutic or prophylactic result.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat, E.A. et al., Sequences of Proteins of Immunological Interest, 5th ed., Public Health Service, National Institutes of Health, Bethesda, MD (1991), NIH Publication 91-3242.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat, E.A. et al., Sequences of Proteins of Immunological Interest, 5th ed., Bethesda MD (1991), NIH Publication 91-3242, Vols. 1-3. In one embodiment, for the VL, the subgroup is subgroup kappa I as in Kabat et al., *supra.* In one embodiment, for the VH, the subgroup is subgroup III as in Kabat et al., *supra.*

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs herein include:

(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));

(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));

(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and

(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

An "immunoconjugate" is an antibody conjugated to one or more heterologous molecule(s), including but not limited to a cytotoxic agent. In one preferred embodiment an immunoconjugate is an anti-TIM3 antibody as described herein conjugated to one or more cytotoxic agents (preferably a Pseudomonas Exotoxin A or a amatoxin).

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman, S. et al., J. Chromatogr. B 848 (2007) 79-87.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-TIM3 antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation. (Include if Prior art has immunoconjugates).

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa ($\kappa$) and lambda ($\lambda$), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

The term "TIM3," as used herein, refers to any native T cell immunoglobulin mucin 3 (TIM3) protein (also known as hepatitis A virus cellular receptor 2 (HAVcr-2), kidney injury molecule-3 (KIM-3), TIM-3, Tim3, or Tim-3) from any vertebrate source, including mammals such as primates (e.g. humans) and rodents (e.g., mice and rats), unless otherwise indicated. The term encompasses "full-length," unprocessed TIM3 as well as any form of TIM3 that results from processing in the cell. The term also encompasses naturally occurring variants of TIM3, e.g., splice variants or allelic variants. The amino acid sequence of an exemplary human TIM3 is shown in SEQ ID NO:77. The amino acid sequence of the Extracellular Domain (ECD) of TIM3 is shown in SEQ ID NO:78.

TIM3 is a human protein which belongs to the immunoglobulin superfamily, and TIM family of proteins. In humans, as similar to mice, TIM-3 is expressed on T-cells as well as phagocytic cells such as macrophages and dendritic cells. Binding of TIM-3 to a protein ligand (e.g., galectin-9) can inhibit the Th1 response via mechanism of apoptosis induction, and therefore lead to such as induction of peripheral tolerance. The reduction in expression of human TIM-3 with siRNA or the inhibition of human TIM-3 by blocking-antibody increased the secretion of interferon alpha from CD4 positive T-cells, supporting the inhibitory role of TIM-3 in human T cells. In phagocytes, TIM-3 also functions as a receptor for recognizing the apoptosis cells. Analysis of clinical samples from autoimmune disease patients showed no expression of TIM-3 in CD4 positive cells. In particular, in T cell clones derived from the cerebrospinal fluid of patients with multiple sclerosis, the expression level of TIM-3 was lower and the secretion level of IFN-□ □ was higher than those of clones derived from normal healthy persons (Koguchi K et al., J Exp Med. 203 (2006) 1413-1418).

There are reports on relation of TIM-3 with allergic diseases or asthma (WO96/27603 and WO2003/063792).

According to the microarray analysis of hematopoietic stem cells from acute myeloid leukemia (hereinafter referred to as "AML") patients and normal hematopoietic stem cells, TIM-3 is expressed on AML stem cells and therefore the analysis suggested involvement of TIM-3 in hematological malignancy (Majeti R et al., PNAS, 106 (2009) 3396-3401 and WO2009/091547).

Examples of the anti-TIM-3 monoclonal antibodies include anti-human TIM- 3 rat monoclonal antibody (Clone 344823, manufactured by R&D Systems) and anti-human TIM-3 mouse monoclonal antibody (Clone F38-2E2, manufactured by R&D Systems).

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt, T.J. et al. Kuby Immunology, 6th ed., W.H. Freeman and Co., N.Y. (2007), page 91) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano, S. et al., J. Immunol. 150 (1993) 880-887; Clackson, T. et al., Nature 352 (1991) 624-628).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

## II. COMPOSITIONS AND METHODS

In one aspect, the invention is based, in part, on the finding that the selected anti-TIM3 antibodies of the invention bind to certain epitopes of TIM3, showing a strong and rapid internalization and/or high cytotoxic activity against cancer cells as immunoconjugate and/or showing strong immunestimulatory cytokine (e.g. interferon gamma) release. In certain embodiments, antibodies that bind to TIM3 are provided. Antibodies of the invention are useful, e.g., for the diagnosis or treatment of cancer.

### A. Exemplary Anti-TIM3Antibodies

The invention is defined in the appended claims and any other aspects set forth herein not falling within the scope of the claims are for information only.

In one aspect, the invention provides isolated antibodies that bind to TIM3. In certain embodiments, an anti-TIM3 is provided wherein the antibody:

- induces internalization of TIM3 (in a FACS assay on TIM3 expressing RPMI8226 cells (ATCC ® CCL-155™)) of at least 45% after 120 Minutes at 37 °C (see Example 6)
- In on embodiment the antibody induces internalization of TIM3 (in a FACS assay on TIM3 expressing RPMI8226 cells (ATCC ® CCL-155™)) of at least 50% after 120 Minutes at 37 °C (see Example 6)
- In on embodiment the antibody induces internalization of TIM3 (in a FACS assay on TIM3 expressing RPMI8226 cells (ATCC ® CCL-155™)) of at least 55% after 120 Minutes at 37 °C (see Example 6)
- In on embodiment the antibody induces internalization of TIM3 (in a FACS assay on TIM3 expressing RPMI8226 cells (ATCC ® CCL-155™)) of at least 60% after 240 Minutes at 37 °C (see Example 6)
- In on embodiment the antibody induces internalization of TIM3 (in a FACS assay on TIM3 expressing RPMI8226 cells (ATCC ® CCL-155™)) of at least 65% after 240 Minutes at 37 °C (see Example 6)

In certain embodiments, an anti- TIM3 is provided, wherein the antibody:

- competes for binding to TIM3 with an anti-Tim3 antibody comprising the VH and VL of Tim3_0016
- binds to a human and cynomolgus TIM3
- shows as immunoconjugate a cytotoxic activity on TIM3 expressing cells (in one embodiment the immunoconjugates has a relative IC50 value of the cytotoxic activity as Pseudomonas exotoxin A conjugate on RPMI-8226 cells of 0.1 or lower (as measured in Example 11)
- induces interferon-gamma release (in MLR assay -see Example 5).

In one aspect, the invention provides an anti-TIM3 antibody comprising at least one, two, three, four, five, or six HVRs selected from (a) HVR-H1 comprising the amino acid sequence of SEQ ID NO:1; (b) HVR-H2 comprising the amino acid sequence of SEQ ID NO:2; (c) HVR-H3 comprising the amino acid sequence of SEQ ID NO:3; (d) HVR-L1 comprising the amino acid sequence of SEQ ID NO:4; or HVR-L1 comprising the amino acid sequence of SEQ ID NO:11; HVR-L1 comprising the amino acid sequence of SEQ ID NO:12; (e) HVR-L2 comprising the amino acid sequence of SEQ ID NO:5; and (f) HVR-L3 comprising the amino acid sequence of SEQ ID NO:6.

In one aspect, the invention provides an anti-TIM3 antibody comprising (a) HVR-H1 comprising the amino acid sequence of SEQ ID NO:1; (b) HVR-H2 comprising the amino acid sequence of SEQ ID NO:2; (c) HVR-H3 comprising the amino acid sequence of SEQ ID NO:3; (d) HVR-L1 comprising the amino acid sequence of SEQ ID NO:4; or HVR-L1 comprising the amino acid sequence of SEQ ID NO:11; or HVR-L1 comprising the amino acid sequence of SEQ ID NO:12; (e) HVR-L2 comprising the amino acid sequence of SEQ ID NO:5; and (f) HVR-L3 comprising the amino acid sequence of SEQ ID NO:6.

In one aspect, the invention provides an anti-TIM3 antibody comprising (a) HVR-H1 comprising the amino acid sequence of SEQ ID NO:1; (b) HVR-H2 comprising the amino acid sequence of SEQ ID NO:2; (c) HVR-H3 comprising the amino acid sequence of SEQ ID NO:3; (d) HVR-L1 comprising the amino acid sequence of SEQ ID NO:4; (e) HVR-L2 comprising the amino acid sequence of SEQ ID NO:5; and (f) HVR-L3 comprising the amino acid sequence of SEQ ID NO:6.

In one aspect, the invention provides an anti-TIM3 antibody comprising (a) HVR-H1 comprising the amino acid sequence of SEQ ID NO:1; (b) HVR-H2 comprising the amino acid sequence of SEQ ID NO:2; (c) HVR-H3 comprising the amino acid sequence of SEQ ID NO:3; (d) HVR-L1 comprising the amino acid sequence of SEQ ID NO:11; (e) HVR-L2 comprising the amino acid sequence of SEQ ID NO:5; and (f) HVR-L3 comprising the amino acid sequence of SEQ ID NO:6.

In one aspect, the invention provides an anti-TIM3 antibody comprising (a) HVR-H1 comprising the amino acid sequence of SEQ ID NO:1; (b) HVR-H2 comprising the amino acid sequence of SEQ ID NO:2; (c) HVR-H3 comprising the amino acid sequence of SEQ ID NO:3; (d) HVR-L1 comprising the amino acid sequence of SEQ ID NO:12; (e) HVR-L2 comprising the amino acid sequence of SEQ ID NO:5; and (f) HVR-L3 comprising the amino acid sequence of SEQ ID NO:6.

In another aspect, an antibody of the invention comprises (a) a VH domain comprising at least one, at least two, or all three VH HVR sequences selected from (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising at least one, at least two, or all three VL HVR sequences selected from (i) HVR-L1 comprising the amino acid sequence of SEQ ID NO:4; or HVR-L1 comprising the amino acid sequence of SEQ ID NO:11; or HVR-L1 comprising the amino acid sequence of SEQ ID NO:12; (ii) HVR-L2 comprising the amino acid sequence of SEQ ID NO:5 and (c) HVR-L3 comprising the amino acid sequence of SEQ ID NO:6.

In another aspect, an antibody of the invention comprises (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:4; or HVR-L1 comprising the amino acid sequence of SEQ ID NO:11; or HVR-L1 comprising the amino acid sequence of SEQ ID NO:12; (ii) HVR-L2 comprising the amino acid sequence of SEQ ID NO:5 and (iii) HVR-L3 comprising the amino acid sequence of SEQ ID NO:6.

In another aspect, an antibody of the invention comprises (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:4; (ii) HVR-L2 comprising the amino acid sequence of SEQ ID NO:5 and (iii) HVR-L3 comprising the amino acid sequence of SEQ ID NO:6.

In another aspect, an antibody of the invention comprises (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:11; (ii) HVR-L2 comprising the amino acid sequence of SEQ ID NO:5 and (iii) HVR-L3 comprising the amino acid sequence of SEQ ID NO:6.

In another aspect, an antibody of the invention comprises (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:12; (ii) HVR-L2 comprising the amino acid sequence of SEQ ID NO:5 and (iii) HVR-L3 comprising the amino acid sequence of SEQ ID NO:6.

In one embodiment such anti-TIM3 antibody comprises

i) a VH sequence of SEQ ID NO:7 and a VL sequence of SEQ ID NO:8;

ii) a VH sequence of SEQ ID NO:9 and a VL sequence of SEQ ID NO:10;

iii) or humanized variant of the VH and VL of the antibody under i) or ii).

In one preferred embodiment such anti-TIM3 antibody comprises

i) a VH sequence of SEQ ID NO:79 and a VL sequence of SEQ ID NO:80,or
ii) a VH sequence of SEQ ID NO:81 and a VL sequence of SEQ ID NO:82.

One preferred embodiment is an antibody that binds to human TIM3 antibody wherein the antibody comprises a VH sequence of SEQ ID NO:79 and a VL sequence of SEQ ID NO:80.

One preferred embodiment is an antibody that binds to human TIM3 antibody wherein the antibody comprises a VH sequence of SEQ ID NO:81 and a VL sequence of SEQ ID NO:82.

In one aspect, the invention provides an anti-TIM3 antibody comprising at least one, two, three, four, five, or six HVRs selected from (a) HVR-H1 comprising the amino acid sequence of SEQ ID NO:13; (b) HVR-H2 comprising the amino acid sequence of SEQ ID NO:14; (c) HVR-H3 comprising the amino acid sequence of SEQ ID NO:15; (d) HVR-L1 comprising the amino acid sequence of SEQ ID NO:16; (e) HVR-L2 comprising the amino acid sequence of SEQ ID NO:17; and (f) HVR-L3 comprising the amino acid sequence of SEQ ID NO:18.

In one aspect, the invention provides an anti-TIM3 antibody comprising (a) HVR-H1 comprising the amino acid sequence of SEQ ID NO:13; (b) HVR-H2 comprising the amino acid sequence of SEQ ID NO:14; (c) HVR-H3 comprising the amino acid sequence of SEQ ID NO:15; (d) HVR-L1 comprising the amino acid sequence of SEQ ID NO:16; (e) HVR-L2 comprising the amino acid sequence of SEQ ID NO:17; and (f) HVR-L3 comprising the amino acid sequence of SEQ ID NO:18.

In another aspect, an antibody of the invention comprises (a) a VH domain comprising at least one, at least two, or all three VH HVR sequences selected from (i) HVR-H1 comprising the amino acid sequence of SEQ ID NO:13, (ii) HVR-H2 comprising the amino acid sequence of SEQ ID NO:14, and (iii) HVR-H3 comprising an amino acid sequence selected from SEQ ID NO:15; and (b) a VL domain comprising at least one, at least two, or all three VL HVR sequences selected from (i) HVR-L1 comprising the amino acid sequence of SEQ ID NO:16; (ii) HVR-L2 comprising the amino acid sequence of SEQ ID NO:17 and (c) HVR-L3 comprising the amino acid sequence of SEQ ID NO:18.

In another aspect, an antibody of the invention comprises (a) a VH domain comprising (i) HVR-H1 comprising the amino acid sequence of SEQ ID NO:13, (ii) HVR-H2 comprising the amino acid sequence of SEQ ID NO:14, and (iii) HVR-H3 comprising an amino acid sequence selected from SEQ ID NO:15; and (b) a VL domain comprising (i) HVR-L1 comprising the amino acid sequence of SEQ ID NO:16; (ii) HVR-L2 comprising the amino acid sequence of SEQ ID NO:17 and (iii) HVR-L3 comprising the amino acid sequence of SEQ ID NO:18.

In one embodiment such anti-TIM3 antibody comprises

i) a VH sequence of SEQ ID NO:19 and a VL sequence of SEQ ID NO:20;

ii) or humanized variant of the VH and VL of the antibody under i).

In one aspect, the invention provides an anti-TIM3 antibody comprising at least one, two, three, four, five, or six HVRs selected from (a) HVR-H1 comprising the amino acid sequence of SEQ ID NO:21; (b) HVR-H2 comprising the amino acid sequence of SEQ ID NO:22; (c) HVR-H3 comprising the amino acid sequence of SEQ ID NO:23; (d) HVR-L1 comprising the amino acid sequence of SEQ ID NO:24; (e) HVR-L2 comprising the amino acid sequence of SEQ ID NO:25; and (f) HVR-L3 comprising the amino acid sequence of SEQ ID NO:26.

In one aspect, the invention provides an anti-TIM3 antibody comprising (a) HVR-H1 comprising the amino acid sequence of SEQ ID NO:21; (b) HVR-H2 comprising the amino acid sequence of SEQ ID NO:22; (c) HVR-H3 comprising the amino acid sequence of SEQ ID NO:23; (d) HVR-L1 comprising the amino acid sequence of SEQ ID NO:24; (e) HVR-L2 comprising the amino acid sequence of SEQ ID NO:25; and (f) HVR-L3 comprising the amino acid sequence of SEQ ID NO:26.

In another aspect, an antibody of the invention comprises (a) a VH domain comprising at least one, at least two, or all three VH HVR sequences selected from (i) HVR-H1 comprising the amino acid sequence of SEQ ID NO:21, (ii) HVR-H2 comprising the amino acid sequence of SEQ ID NO:22, and (iii) HVR-H3 comprising an amino acid sequence selected from SEQ ID NO:23; and (b) a VL domain comprising at least one, at least two, or all three VL HVR sequences selected from (i) HVR-L1 comprising the amino acid sequence of SEQ ID NO:24; (ii) HVR-L2 comprising the amino acid sequence of SEQ ID NO:25 and (c) HVR-L3 comprising the amino acid sequence of SEQ ID NO:26.

In another aspect, an antibody of the invention comprises (a) a VH domain comprising (i) HVR-H1 comprising the amino acid sequence of SEQ ID NO:21, (ii) HVR-H2 comprising the amino acid sequence of SEQ ID NO:22, and (iii) HVR-H3 comprising an amino acid sequence selected from SEQ ID NO:23; and (b) a VL domain comprising (i) HVR-L1 comprising the amino acid sequence of SEQ ID NO:24; (ii) HVR-L2 comprising the amino acid sequence of SEQ ID NO:25 and (iii) HVR-L3 comprising the amino acid sequence of SEQ ID NO:26.

In one embodiment such anti-TIM3 antibody comprises

i) a VH sequence of SEQ ID NO:27 and a VL sequence of SEQ ID NO:28;

ii) or humanized variant of the VH and VL of the antibody under i).

In one aspect, the invention provides an anti-TIM3 antibody comprising at least one, two, three, four, five, or six HVRs selected from (a) HVR-H1 comprising the amino acid sequence of SEQ ID NO:29; (b) HVR-H2 comprising the amino acid sequence of SEQ ID NO:30; (c) HVR-H3 comprising the amino acid sequence of SEQ ID NO:31; (d) HVR-L1 comprising the amino acid sequence of SEQ ID NO:32; (e) HVR-L2 comprising the amino acid sequence of SEQ ID NO:33; and (f) HVR-L3 comprising the amino acid sequence of SEQ ID NO:34.

In one aspect, the invention provides an anti-TIM3 antibody comprising (a) HVR-H1 comprising the amino acid sequence of SEQ ID NO:29; (b) HVR-H2 comprising the amino acid sequence of SEQ ID NO:30; (c) HVR-H3 comprising the amino acid sequence of SEQ ID NO:31; (d) HVR-L1 comprising the amino acid sequence of SEQ ID NO:32; (e) HVR-L2 comprising the amino acid sequence of SEQ ID NO:33; and (f) HVR-L3 comprising the amino acid sequence of SEQ ID NO:34.

In another aspect, an antibody of the invention comprises (a) a VH domain comprising at least one, at least two, or all three VH HVR sequences selected from (i) HVR-H1 comprising the amino acid sequence of SEQ ID NO:29, (ii) HVR-H2 comprising the amino acid sequence of SEQ ID NO:30, and (iii) HVR-H3 comprising an amino acid sequence selected from SEQ ID NO:31; and (b) a VL domain comprising at least one, at least two, or all three VL HVR sequences selected from (i) HVR-L1 comprising the amino acid sequence of SEQ ID NO:32; (ii) HVR-L2 comprising the amino acid sequence of SEQ ID NO:33 and (c) HVR-L3 comprising the amino acid sequence of SEQ ID NO:34.

In another aspect, an antibody of the invention comprises (a) a VH domain comprising (i) HVR-H1 comprising the amino acid sequence of SEQ ID NO:29, (ii) HVR-H2 comprising the amino acid sequence of SEQ ID NO:30, and (iii) HVR-H3 comprising an amino acid sequence selected from SEQ ID NO:31; and (b) a VL domain comprising (i) HVR-L1 comprising the amino acid sequence of SEQ ID NO:32; (ii) HVR-L2 comprising the amino acid sequence of SEQ ID NO:33 and (iii) HVR-L3 comprising the amino acid sequence of SEQ ID NO:34.

In one embodiment such anti-TIM3 antibody comprises

i) a VH sequence of SEQ ID NO:35 and a VL sequence of SEQ ID NO:36;

ii) or humanized variant of the VH and VL of the antibody under i).

In one aspect, the invention provides an anti-TIM3 antibody comprising at least one, two, three, four, five, or six HVRs selected from (a) HVR-H1 comprising the amino acid sequence of SEQ ID NO:37; (b) HVR-H2 comprising the amino acid sequence of SEQ ID NO:38; (c) HVR-H3 comprising the amino acid sequence of SEQ ID NO:39; (d) HVR-L1 comprising the amino acid sequence of SEQ ID NO:40; (e) HVR-L2 comprising the amino acid sequence of SEQ ID NO:41; and (f) HVR-L3 comprising the amino acid sequence of SEQ ID NO:42.

In one aspect, the invention provides an anti-TIM3 antibody comprising (a) HVR-H1 comprising the amino acid sequence of SEQ ID NO:37; (b) HVR-H2 comprising the amino acid sequence of SEQ ID NO:38; (c) HVR-H3 comprising the amino acid sequence of SEQ ID NO:39; (d) HVR-L1 comprising the amino acid sequence of SEQ ID NO:40; (e) HVR-L2 comprising the amino acid sequence of SEQ ID NO:41; and (f) HVR-L3 comprising the amino acid sequence of SEQ ID NO:42.

In another aspect, an antibody of the invention comprises (a) a VH domain comprising at least one, at least two, or all three VH HVR sequences selected from (i) HVR-H1 comprising the amino acid sequence of SEQ ID NO:37, (ii) HVR-H2 comprising the amino acid sequence of SEQ ID NO:38, and (iii) HVR-H3 comprising an amino acid sequence selected from SEQ ID NO:39; and (b) a VL domain comprising at least one, at least two, or all three VL HVR sequences selected from (i) HVR-L1 comprising the amino acid sequence of SEQ ID NO:40; (ii) HVR-L2 comprising the amino acid sequence of SEQ ID NO:41 and (c) HVR-L3 comprising the amino acid sequence of SEQ ID NO:42.

In another aspect, an antibody of the invention comprises (a) a VH domain comprising (i) HVR-H1 comprising the amino acid sequence of SEQ ID NO:37, (ii) HVR-H2 comprising the amino acid sequence of SEQ ID NO:38, and (iii) HVR-H3 comprising an amino acid sequence selected from SEQ ID NO:39; and (b) a VL domain comprising (i) HVR-L1 comprising the amino acid sequence of SEQ ID NO:40; (ii) HVR-L2 comprising the amino acid sequence of SEQ ID NO:41 and (iii) HVR-L3 comprising the amino acid sequence of SEQ ID NO:42.

In one embodiment such anti-TIM3 antibody comprises

i) a VH sequence of SEQ ID NO:43 and a VL sequence of SEQ ID NO:44;

ii) or humanized variant of the VH and VL of the antibody under i).

In one preferred embodiment such anti-TIM3 antibody comprises

i) a VH sequence of SEQ ID NO:83 and a VL sequence of SEQ ID NO:84,or

ii) a VH sequence of SEQ ID NO:85 and a VL sequence of SEQ ID NO:86.

One preferred embodiment is an antibody that binds to human TIM3 antibody wherein the antibody comprises a VH sequence of SEQ ID NO:83 and a VL sequence of SEQ ID NO:84.

One preferred embodiment is an antibody that binds to human TIM3 antibody wherein the antibody comprises a VH sequence of SEQ ID NO:85 and a VL sequence of SEQ ID NO:86.

In one aspect, the invention provides an anti-TIM3 antibody comprising at least one, two, three, four, five, or six HVRs selected from (a) HVR-H1 comprising the amino acid sequence of SEQ ID NO:45; (b) HVR-H2 comprising the amino acid sequence of SEQ ID NO:46; (c) HVR-H3 comprising the amino acid sequence of SEQ ID NO:47; (d) HVR-L1 comprising the amino acid sequence of SEQ ID NO:48; (e) HVR-L2 comprising the amino acid sequence of SEQ ID NO:49; and (f) HVR-L3 comprising the amino acid sequence of SEQ ID NO:50.

In one aspect, the invention provides an anti-TIM3 antibody comprising (a) HVR-H1 comprising the amino acid sequence of SEQ ID NO:45; (b) HVR-H2 comprising the amino acid sequence of SEQ ID NO:46; (c) HVR-H3 comprising the amino acid sequence of SEQ ID NO:47; (d) HVR-L1 comprising the amino acid sequence of SEQ ID NO:48; (e) HVR-L2 comprising the amino acid sequence of SEQ ID NO:49; and (f) HVR-L3 comprising the amino acid sequence of SEQ ID NO:50.

In another aspect, an antibody of the invention comprises (a) a VH domain comprising at least one, at least two, or all three VH HVR sequences selected from (i) HVR-H1 comprising the amino acid sequence of SEQ ID NO:45, (ii) HVR-H2 comprising the amino acid sequence of SEQ ID NO:46, and (iii) HVR-H3 comprising an amino acid sequence selected from SEQ ID NO:47; and (b) a VL domain comprising at least one, at least two, or all three VL HVR sequences selected from (i) HVR-L1 comprising the amino acid sequence of SEQ ID NO:48; (ii) HVR-L2 comprising the amino acid sequence of SEQ ID NO:49 and (c) HVR-L3 comprising the amino acid sequence of SEQ ID NO:50.

In another aspect, an antibody of the invention comprises (a) a VH domain comprising (i) HVR-H1 comprising the amino acid sequence of SEQ ID NO:45, (ii) HVR-H2 comprising the amino acid sequence of SEQ ID NO:46, and (iii) HVR-H3 comprising an amino acid sequence selected from SEQ ID NO:47; and (b) a VL domain comprising (i) HVR-L1 comprising the amino acid sequence of SEQ ID NO:48; (ii) HVR-L2 comprising the amino acid sequence of SEQ ID NO:49 and (iii) HVR-L3 comprising the amino acid sequence of SEQ ID NO:50.

In one embodiment such anti-TIM3 antibody comprises

i) a VH sequence of SEQ ID NO:51 and a VL sequence of SEQ ID NO:52;

ii) or humanized variant of the VH and VL of the antibody under i).

In one aspect, the invention provides an anti-TIM3 antibody comprising at least one, two, three, four, five, or six HVRs selected from (a) HVR-H1 comprising the amino acid sequence of SEQ ID NO:53; (b) HVR-H2 comprising the amino acid sequence of SEQ ID NO:54; (c) HVR-H3 comprising the amino acid sequence of SEQ ID NO:55; (d) HVR-L1 comprising the amino acid sequence of SEQ ID NO:56; (e) HVR-L2 comprising the amino acid sequence of SEQ ID NO:57; and (f) HVR-L3 comprising the amino acid sequence of SEQ ID NO:58.

In one aspect, the invention provides an anti-TIM3 antibody comprising (a) HVR-H1 comprising the amino acid sequence of SEQ ID NO:53; (b) HVR-H2 comprising the amino acid sequence of SEQ ID NO:54; (c) HVR-H3 comprising the amino acid sequence of SEQ ID NO:55; (d) HVR-L1 comprising the amino acid sequence of SEQ ID NO:56; (e) HVR-L2 comprising the amino acid sequence of SEQ ID NO:57; and (f) HVR-L3 comprising the amino acid sequence of SEQ ID NO:58.

In another aspect, an antibody of the invention comprises (a) a VH domain comprising at least one, at least two, or all three VH HVR sequences selected from (i) HVR-H1 comprising the amino acid sequence of SEQ ID NO:53, (ii) HVR-H2 comprising the amino acid sequence of SEQ ID NO:54, and (iii) HVR-H3 comprising an amino acid sequence selected from SEQ ID NO:55; and (b) a VL domain comprising at least one, at least two, or all three VL HVR sequences selected from (i) HVR-L1 comprising the amino acid sequence of SEQ ID NO:56; (ii) HVR-L2 comprising the amino acid sequence of SEQ ID NO:57 and (c) HVR-L3 comprising the amino acid sequence of SEQ ID NO:58.

In another aspect, an antibody of the invention comprises (a) a VH domain comprising (i) HVR-H1 comprising the amino acid sequence of SEQ ID NO:53, (ii) HVR-H2 comprising the amino acid sequence of SEQ ID NO:54, and (iii) HVR-H3 comprising an amino acid sequence selected from SEQ ID NO:55; and (b) a VL domain comprising (i) HVR-L1 comprising the amino acid sequence of SEQ ID NO:56; (ii) HVR-L2 comprising the amino acid sequence of SEQ ID NO:57 and (iii) HVR-L3 comprising the amino acid sequence of SEQ ID NO:58.

In one embodiment such anti-TIM3 antibody comprises

i) a VH sequence of SEQ ID NO:59 and a VL sequence of SEQ ID NO:60;

ii) or humanized variant of the VH and VL of the antibody under i).

In one aspect, the invention provides an anti-TIM3 antibody comprising at least one, two, three, four, five, or six HVRs selected from (a) HVR-H1 comprising the amino acid sequence of SEQ ID NO:61; (b) HVR-H2 comprising the amino acid sequence of SEQ ID NO:62; (c) HVR-H3 comprising the amino acid sequence of SEQ ID NO:63; (d) HVR-L1 comprising the amino acid sequence of SEQ ID NO:64; (e) HVR-L2 comprising the amino acid sequence of SEQ ID NO:65; and (f) HVR-L3 comprising the amino acid sequence of SEQ ID NO:66.

In one aspect, the invention provides an anti-TIM3 antibody comprising (a) HVR-H1 comprising the amino acid sequence of SEQ ID NO:61; (b) HVR-H2 comprising the amino acid sequence of SEQ ID NO:62; (c) HVR-H3 comprising the amino acid sequence of SEQ ID NO:63; (d) HVR-L1 comprising the amino acid sequence of SEQ ID NO:64; (e) HVR-L2 comprising the amino acid sequence of SEQ ID NO:65; and (f) HVR-L3 comprising the amino acid sequence of SEQ ID NO:66.

In another aspect, an antibody of the invention comprises (a) a VH domain comprising at least one, at least two, or all three VH HVR sequences selected from (i) HVR-H1 comprising the amino acid sequence of SEQ ID NO:61, (ii) HVR-H2 comprising the amino acid sequence of SEQ ID NO:62, and (iii) HVR-H3 comprising an amino acid sequence selected from SEQ ID NO:63; and (b) a VL domain comprising at least one, at least two, or all three VL HVR sequences selected from (i) HVR-L1 comprising the amino acid sequence of SEQ ID NO:64; (ii) HVR-L2 comprising the amino acid sequence of SEQ ID NO:65 and (c) HVR-L3 comprising the amino acid sequence of SEQ ID NO:66.

In another aspect, an antibody of the invention comprises (a) a VH domain comprising (i) HVR-H1 comprising the amino acid sequence of SEQ ID NO:61, (ii) HVR-H2 comprising the amino acid sequence of SEQ ID NO:62, and (iii) HVR-H3 comprising an amino acid sequence selected from SEQ ID NO:63; and (b) a VL domain comprising (i) HVR-L1 comprising the amino acid sequence of SEQ ID NO:64; (ii) HVR-L2 comprising the amino acid sequence of SEQ ID NO:65 and (iii) HVR-L3 comprising the amino acid sequence of SEQ ID NO:66.

In one embodiment such anti-TIM3 antibody comprises

i) a VH sequence of SEQ ID NO:67 and a VL sequence of SEQ ID NO:68;

ii) or humanized variant of the VH and VL of the antibody under i).

In any of the above embodiments, an anti-TIM3 antibody is humanized. In one embodiment, an anti-TIM3 antibody comprises HVRs as in any of the above embodiments, and further comprises an acceptor human framework, e.g. a human immunoglobulin framework or a human consensus framework. In another embodiment, an anti-TIM3 antibody comprises HVRs as in any of the above embodiments, and further comprises a VH and VL comprising such HVRs.

In a further aspect, the invention provides an antibody that binds to the same epitope as an anti-TIM3 antibody provided herein. For example, in certain embodiments, an antibody is provided that binds to the same epitope as anti-TIM3 antibody comprising a VH sequence of SEQ ID NO:7 and a VL sequence of SEQ ID NO:8, or an antibody is provided that binds to the same epitope as anti-TIM3 antibody comprising a VH sequence of SEQ ID NO:9 and a VL sequence of SEQ ID NO:10, or an antibody is provided that binds to the same epitope as anti-TIM3 antibody comprising a VH sequence of SEQ ID NO:19 and a VL sequence of SEQ ID NO:20, or an antibody is provided that binds to the same epitope as anti-TIM3 antibody comprising a VH sequence of SEQ ID NO:27 and a VL sequence of SEQ ID NO:28, or an antibody is provided that binds to the same epitope as anti-TIM3 antibody comprising a VH sequence of SEQ ID NO:35 and a VL sequence of SEQ ID NO36, or an antibody is provided that binds to the same epitope as anti-TIM3 antibody comprising a VH sequence of SEQ ID NO:43 and a VL sequence of SEQ ID NO:44, or an antibody is provided that binds to the same epitope as anti-TIM3 antibody comprising a VH sequence of SEQ ID NO:51 and a VL sequence of SEQ ID NO:52, or an antibody is provided that binds to the same epitope as anti-TIM3 antibody comprising a VH sequence of SEQ ID NO:59 and a VL sequence of SEQ ID NO:60, or an antibody is provided that binds to the same epitope as anti-TIM3 antibody comprising a VH sequence of SEQ ID NO:67 and a VL sequence of SEQ ID NO:68.

In one preferred embodiment an antibody is provided that binds to the same epitope as an anti-TIM3 antibody comprising a VH sequence of SEQ ID NO:7 and a VL sequence of SEQ ID NO:8.

In one preferred embodiment an antibody is provided that competes for binding to human TIM3 with anti- TIM3 antibody comprising a VH sequence of SEQ ID NO:7 and a VL sequence of SEQ ID NO:8 (as determined in a competition assay described in Example 4 on RPMI-8226 cells (ATCC ® CCL-155™)).

In one aspect, the invention provides an anti-TIM3 antibody (e.g. an antibody that binds to human TIM3) comprising

A) (a) HVR-H1 comprising the amino acid sequence of SEQ ID NO:1; (b) HVR-H2 comprising the amino acid sequence of SEQ ID NO:2; (c) HVR-H3 comprising the amino acid sequence of SEQ ID NO:3; (d) HVR-L1 comprising the amino acid sequence of SEQ ID NO:4; (e) HVR-L2 comprising the amino acid sequence of SEQ ID NO:5; and (f) HVR-L3 comprising the amino acid sequence of SEQ ID NO:6; or

B) (a) HVR-H1 comprising the amino acid sequence of SEQ ID NO:1; (b) HVR-H2 comprising the amino acid sequence of SEQ ID NO:2; (c) HVR-H3 comprising the amino acid sequence of SEQ ID NO:3; (d) HVR-L1 comprising the amino acid sequence of SEQ ID NO:11; (e) HVR-L2 comprising the amino acid sequence of SEQ ID NO:5; and (f) HVR-L3 comprising the amino acid sequence of SEQ ID NO:6; or

C) (a) HVR-H1 comprising the amino acid sequence of SEQ ID NO:1; (b) HVR-H2 comprising the amino acid sequence of SEQ ID NO:2; (c) HVR-H3 comprising the amino acid sequence of SEQ ID NO:3; (d) HVR-L1 comprising the amino acid sequence of SEQ ID NO:12; (e) HVR-L2 comprising the amino acid sequence of SEQ ID NO:5; and (f) HVR-L3 comprising the amino acid sequence of SEQ ID NO:6; or

D) (a) HVR-H1 comprising the amino acid sequence of SEQ ID NO:13; (b) HVR-H2 comprising the amino acid sequence of SEQ ID NO:14; (c) HVR-H3 comprising the amino acid sequence of SEQ ID NO:15; (d) HVR-L1 comprising the amino acid sequence of SEQ ID NO:16; (e) HVR-L2 comprising the amino acid sequence of SEQ ID NO:17; and (f) HVR-L3 comprising the amino acid sequence of SEQ ID NO:18; or

E) (a) HVR-H1 comprising the amino acid sequence of SEQ ID NO:21; (b) HVR-H2 comprising the amino acid sequence of SEQ ID NO:22; (c) HVR-H3 comprising the amino acid sequence of SEQ ID NO:23; (d) HVR-L1 comprising the amino acid sequence of SEQ ID NO:24; (e) HVR-L2 comprising the amino acid sequence of SEQ ID NO:25; and (f) HVR-L3 comprising the amino acid sequence of SEQ ID NO:26; or

F) (a) HVR-H1 comprising the amino acid sequence of SEQ ID NO:29; (b) HVR-H2 comprising the amino acid sequence of SEQ ID NO:30; (c) HVR-H3 comprising the amino acid sequence of SEQ ID NO:31; (d) HVR-L1 comprising the amino acid sequence of SEQ ID NO:32; (e) HVR-L2 comprising the amino acid sequence of SEQ ID NO:33; and (f) HVR-L3 comprising the amino acid sequence of SEQ ID NO:34; or

G) (a) HVR-H1 comprising the amino acid sequence of SEQ ID NO:37; (b) HVR-H2 comprising the amino acid sequence of SEQ ID NO:38; (c) HVR-H3 comprising the amino acid sequence of SEQ ID NO:39; (d) HVR-L1 comprising the amino acid sequence of SEQ ID NO:40; (e) HVR-L2 comprising the amino acid sequence of SEQ ID NO:41; and (f) HVR-L3 comprising the amino acid sequence of SEQ ID NO:42; or

H) (a) HVR-H1 comprising the amino acid sequence of SEQ ID NO:45; (b) HVR-H2 comprising the amino acid sequence of SEQ ID NO:46; (c) HVR-H3 comprising the amino acid sequence of SEQ ID NO:47; (d) HVR-L1 comprising the amino acid sequence of SEQ ID NO:48; (e) HVR-L2 comprising the amino acid sequence of SEQ ID NO:49; and (f) HVR-L3 comprising the amino acid sequence of SEQ ID NO:50; or.

I) (a) HVR-H1 comprising the amino acid sequence of SEQ ID NO:53; (b) HVR-H2 comprising the amino acid sequence of SEQ ID NO:54; (c) HVR-H3 comprising the amino acid sequence of SEQ ID NO:55; (d) HVR-L1 comprising the amino acid sequence of SEQ ID NO:56; (e) HVR-L2 comprising the amino acid sequence of SEQ ID NO:57; and (f) HVR-L3 comprising the amino acid sequence of SEQ ID NO:58; or

J) (a) HVR-H1 comprising the amino acid sequence of SEQ ID NO:61; (b) HVR-H2 comprising the amino acid sequence of SEQ ID NO:62; (c) HVR-H3 comprising the amino acid sequence of SEQ ID NO:63; (d) HVR-L1 comprising the amino acid sequence of SEQ ID NO:64; (e) HVR-L2 comprising the amino acid sequence of SEQ ID NO:65; and (f) HVR-L3 comprising the amino acid sequence of SEQ ID NO:66.

In another aspect the invention provides an anti-TIM3 antibody (e.g. an antibody that binds to human TIM3) comprising

A) (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:4; (ii) HVR-L2 comprising the amino acid sequence of SEQ ID NO:5 and (iii) HVR-L3 comprising the amino acid sequence of SEQ ID NO:6; or

B) (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:11; (ii) HVR-L2 comprising the amino acid sequence of SEQ ID NO:5 and (iii) HVR-L3 comprising the amino acid sequence of SEQ ID NO:6; or

C) (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:12; (ii) HVR-L2 comprising the amino acid sequence of SEQ ID NO:5 and (iii) HVR-L3 comprising the amino acid sequence of SEQ ID NO:6; or

D) (a) a VH domain comprising (i) HVR-H1 comprising the amino acid sequence of SEQ ID NO:13, (ii) HVR-H2 comprising the amino acid sequence of SEQ ID NO:14, and (iii) HVR-H3 comprising an amino acid sequence selected from SEQ ID NO:15; and (b) a VL domain comprising (i) HVR-L1 comprising the amino acid sequence of SEQ ID NO:16; (ii) HVR-L2 comprising the amino acid sequence of SEQ ID NO:17 and (iii) HVR-L3 comprising the amino acid sequence of SEQ ID NO:18; or

E) (a) a VH domain comprising (i) HVR-H1 comprising the amino acid sequence of SEQ ID NO:21, (ii) HVR-H2 comprising the amino acid sequence of SEQ ID NO:22, and (iii) HVR-H3 comprising an amino acid sequence selected from SEQ ID NO:23; and (b) a VL domain comprising (i) HVR-L1 comprising the amino acid sequence of SEQ ID NO:24; (ii) HVR-L2 comprising the amino acid sequence of SEQ ID NO:25 and (iii) HVR-L3 comprising the amino acid sequence of SEQ ID NO:26; or.

F) (a) a VH domain comprising (i) HVR-H1 comprising the amino acid sequence of SEQ ID NO:29, (ii) HVR-H2 comprising the amino acid sequence of SEQ ID NO:30, and (iii) HVR-H3 comprising an amino acid sequence selected from SEQ ID NO:31; and (b) a VL domain comprising (i) HVR-L1 comprising the amino acid sequence of SEQ ID NO:32; (ii) HVR-L2 comprising the amino acid sequence of SEQ ID NO:33 and (iii) HVR-L3 comprising the amino acid sequence of SEQ ID NO:34; or

G) (a) a VH domain comprising (i) HVR-H1 comprising the amino acid sequence of SEQ ID NO:37, (ii) HVR-H2 comprising the amino acid sequence of SEQ ID NO:38, and (iii) HVR-H3 comprising an amino acid sequence selected from SEQ ID NO:39; and (b) a VL domain comprising (i) HVR-L1 comprising the amino acid sequence of SEQ ID NO:40; (ii) HVR-L2 comprising the amino acid sequence of SEQ ID NO:41 and (iii) HVR-L3 comprising the amino acid sequence of SEQ ID NO:42; or

H) (a) a VH domain comprising (i) HVR-H1 comprising the amino acid sequence of SEQ ID NO:45, (ii) HVR-H2 comprising the amino acid sequence of SEQ ID NO:46, and (iii) HVR-H3 comprising an amino acid sequence selected from SEQ ID NO:47; and (b) a VL domain comprising (i) HVR-L1 comprising the amino acid sequence of SEQ ID NO:48; (ii) HVR-L2 comprising the amino acid sequence of SEQ ID NO:49 and (iii) HVR-L3 comprising the amino acid sequence of SEQ ID NO:50; or

I) (a) a VH domain comprising (i) HVR-H1 comprising the amino acid sequence of SEQ ID NO:53, (ii) HVR-H2 comprising the amino acid sequence of SEQ ID NO:54, and (iii) HVR-H3 comprising an amino acid sequence selected from SEQ ID NO:55; and (b) a VL domain comprising (i) HVR-L1 comprising the amino acid sequence of SEQ ID NO:56; (ii) HVR-L2 comprising the amino acid sequence of SEQ ID NO:57 and (iii) HVR-L3 comprising the amino acid sequence of SEQ ID NO:58; or

J) (a) a VH domain comprising (i) HVR-H1 comprising the amino acid sequence of SEQ ID NO:61, (ii) HVR-H2 comprising the amino acid sequence of SEQ ID NO:62, and (iii) HVR-H3 comprising an amino acid sequence selected from SEQ ID NO:63; and (b) a VL domain comprising (i) HVR-L1 comprising the amino acid sequence of SEQ ID NO:64; (ii) HVR-L2 comprising the amino acid sequence of SEQ ID NO:65 and (iii) HVR-L3 comprising the amino acid sequence of SEQ ID NO:66.

In one aspect, the invention provides an anti-TIM3 antibody (e.g. an antibody that binds to human TIM3) that

A1)

i) comprises a VH sequence of SEQ ID NO:7 and a VL sequence of SEQ ID NO:8;

ii) comprises a VH sequence of SEQ ID NO:9 and a VL sequence of SEQ ID NO:10;

iii) or humanized variant of the VH and VL of the antibody under i) or ii);

or A2)

i) comprises a VH sequence of SEQ ID NO:79 and a VL sequence of SEQ ID NO:80,or
ii) comprises a VH sequence of SEQ ID NO:81 and a VL sequence of SEQ ID NO:82.

or B)

i) comprises a VH sequence of SEQ ID NO:19 and a VL sequence of SEQ ID NO:20;

ii) or humanized variant of the VH and VL of the antibody under i);

or C)

i) comprises a VH sequence of SEQ ID NO:27 and a VL sequence of SEQ ID NO:28;

ii) or humanized variant of the VH and VL of the antibody under i);

or D)

i) comprises a VH sequence of SEQ ID NO:35 and a VL sequence of SEQ ID NO:36;

ii) or humanized variant of the VH and VL of the antibody under i);.

or E)

i) comprises a VH sequence of SEQ ID NO:43 and a VL sequence of SEQ ID NO:44;

ii) or humanized variant of the VH and VL of the antibody under i);

or F)

i) comprises a VH sequence of SEQ ID NO:51 and a VL sequence of SEQ ID NO:52;

ii) or humanized variant of the VH and VL of the antibody under i);

or G1)

i) comprises a VH sequence of SEQ ID NO:59 and a VL sequence of SEQ ID NO:60;
ii) or humanized variant of the VH and VL of the antibody under i);

or G2)

i) comprises a VH sequence of SEQ ID NO:83 and a VL sequence of SEQ ID NO:84,or

ii) comprises a VH sequence of SEQ ID NO:85 and a VL sequence of SEQ ID NO:86.

or H)

i) comprises a VH sequence of SEQ ID NO:67 and a VL sequence of SEQ ID NO:68;

ii) or humanized variant of the VH and VL of the antibody under i).

In another aspect the invention provides an anti-TIM3 antibody (e.g. an antibody that binds to human TIM3) comprising

A) (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:4; (ii) HVR-L2 comprising the amino acid sequence of SEQ ID NO:5 and (iii) HVR-L3 comprising the amino acid sequence of SEQ ID NO:6; or

B) (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:11; (ii) HVR-L2 comprising the amino acid sequence of SEQ ID NO:5 and (iii) HVR-L3 comprising the amino acid sequence of SEQ ID NO:6; or

C) (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:12; (ii) HVR-L2 comprising the amino acid sequence of SEQ ID NO:5 and (iii) HVR-L3 comprising the amino acid sequence of SEQ ID NO:6; or

D) (a) a VH domain comprising (i) HVR-H1 comprising the amino acid sequence of SEQ ID NO:13, (ii) HVR-H2 comprising the amino acid sequence of SEQ ID NO:14, and (iii) HVR-H3 comprising an amino acid sequence selected from SEQ ID NO:15; and (b) a VL domain comprising (i) HVR-L1 comprising the amino acid sequence of SEQ ID NO:16; (ii) HVR-L2 comprising the amino acid sequence of SEQ ID NO:17 and (iii) HVR-L3 comprising the amino acid sequence of SEQ ID NO:18; or

E) (a) a VH domain comprising (i) HVR-H1 comprising the amino acid sequence of SEQ ID NO:21, (ii) HVR-H2 comprising the amino acid sequence of SEQ ID NO:22, and (iii) HVR-H3 comprising an amino acid sequence selected from SEQ ID NO:23; and (b) a VL domain comprising (i) HVR-L1 comprising the amino acid sequence of SEQ ID NO:24; (ii) HVR-L2 comprising the amino acid sequence of SEQ ID NO:25 and (iii) HVR-L3 comprising the amino acid sequence of SEQ ID NO:26; or.

F) (a) a VH domain comprising (i) HVR-H1 comprising the amino acid sequence of SEQ ID NO:29, (ii) HVR-H2 comprising the amino acid sequence of SEQ ID NO:30, and (iii) HVR-H3 comprising an amino acid sequence selected from SEQ ID NO:31; and (b) a VL domain comprising (i) HVR-L1 comprising the amino acid sequence of SEQ ID NO:32; (ii) HVR-L2 comprising the amino acid sequence of SEQ ID NO:33 and (iii) HVR-L3 comprising the amino acid sequence of SEQ ID NO:34; or

G) (a) a VH domain comprising (i) HVR-H1 comprising the amino acid sequence of SEQ ID NO:37, (ii) HVR-H2 comprising the amino acid sequence of SEQ ID NO:38, and (iii) HVR-H3 comprising an amino acid sequence selected from SEQ ID NO:39; and (b) a VL domain comprising (i) HVR-L1 comprising the amino acid sequence of SEQ ID NO:40; (ii) HVR-L2 comprising the amino acid sequence of SEQ ID NO:41 and (iii) HVR-L3 comprising the amino acid sequence of SEQ ID NO:42; or

H) (a) a VH domain comprising (i) HVR-H1 comprising the amino acid sequence of SEQ ID NO:45, (ii) HVR-H2 comprising the amino acid sequence of SEQ ID NO:46, and (iii) HVR-H3 comprising an amino acid sequence selected from SEQ ID NO:47; and (b) a VL domain comprising (i) HVR-L1 comprising the amino acid sequence of SEQ ID NO:48; (ii) HVR-L2 comprising the amino acid sequence of SEQ ID NO:49 and (iii) HVR-L3 comprising the amino acid sequence of SEQ ID NO:50; or

I) (a) a VH domain comprising (i) HVR-H1 comprising the amino acid sequence of SEQ ID NO:53, (ii) HVR-H2 comprising the amino acid sequence of SEQ ID NO:54, and (iii) HVR-H3 comprising an amino acid sequence selected from SEQ ID NO:55; and (b) a VL domain comprising (i) HVR-L1 comprising the amino acid sequence of SEQ ID NO:56; (ii) HVR-L2 comprising the amino acid sequence of SEQ ID NO:57 and (iii) HVR-L3 comprising the amino acid sequence of SEQ ID NO:58; or

J) (a) a VH domain comprising (i) HVR-H1 comprising the amino acid sequence of SEQ ID NO:61, (ii) HVR-H2 comprising the amino acid sequence of SEQ ID NO:62, and (iii) HVR-H3 comprising an amino acid sequence selected from SEQ ID NO:63; and (b) a VL domain comprising (i) HVR-L1 comprising the amino acid sequence of SEQ ID NO:64; (ii) HVR-L2 comprising the amino acid sequence of SEQ ID NO:65 and (iii) HVR-L3 comprising the amino acid sequence of SEQ ID NO:66;
wherein the antibody is characterized independently by one or more of the following properties: anti-TIM3 antibody

- induces internalization of TIM3 (in a FACS assay on TIM3 expressing RPMI8226 cells) of at least 45% after 120 Minutes at 37 °C (see Example 6)
- In on embodiment the antibody induces internalization of TIM3 (in a FACS assay on TIM3 expressing RPMI8226 cells) of at least 50% after 120 Minutes at 37 °C (see Example 6)
- In on embodiment the antibody induces internalization of TIM3 (in a FACS assay on TIM3 expressing RPMI8226 cells) of at least 55% after 120 Minutes at 37 °C (see Example 6)
- In on embodiment the antibody induces internalization of TIM3 (in a FACS assay on TIM3 expressing RPMI8226 cells) of at least 60% after 240 Minutes at 37 °C (see Example 6)
- In on embodiment the antibody induces internalization of TIM3 (in a FACS assay on TIM3 expressing RPMI8226 cells) of at least 65% after 240 Minutes at 37 °C (see Example 6)

In another aspect the invention provides an anti-TIM3 antibody (e.g. an antibody that binds to human TIM3) comprising

A) (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:4; (ii) HVR-L2 comprising the amino acid sequence of SEQ ID NO:5 and (iii) HVR-L3 comprising the amino acid sequence of SEQ ID NO:6; or

B) (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:11; (ii) HVR-L2 comprising the amino acid sequence of SEQ ID NO:5 and (iii) HVR-L3 comprising the amino acid sequence of SEQ ID NO:6; or

C) (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:12; (ii) HVR-L2 comprising the amino acid sequence of SEQ ID NO:5 and (iii) HVR-L3 comprising the amino acid sequence of SEQ ID NO:6; or

D) (a) a VH domain comprising (i) HVR-H1 comprising the amino acid sequence of SEQ ID NO:13, (ii) HVR-H2 comprising the amino acid sequence of SEQ ID NO:14, and (iii) HVR-H3 comprising an amino acid sequence selected from SEQ ID NO:15; and (b) a VL domain comprising (i) HVR-L1 comprising the amino acid sequence of SEQ ID NO:16; (ii) HVR-L2 comprising the amino acid sequence of SEQ ID NO:17 and (iii) HVR-L3 comprising the amino acid sequence of SEQ ID NO:18; or

E) (a) a VH domain comprising (i) HVR-H1 comprising the amino acid sequence of SEQ ID NO:21, (ii) HVR-H2 comprising the amino acid sequence of SEQ ID NO:22, and (iii) HVR-H3 comprising an amino acid sequence selected from SEQ ID NO:23; and (b) a VL domain comprising (i) HVR-L1 comprising the amino acid sequence of SEQ ID NO:24; (ii) HVR-L2 comprising the amino acid sequence of SEQ ID NO:25 and (iii) HVR-L3 comprising the amino acid sequence of SEQ ID NO:26; or.

F) (a) a VH domain comprising (i) HVR-H1 comprising the amino acid sequence of SEQ ID NO:29, (ii) HVR-H2 comprising the amino acid sequence of SEQ ID NO:30, and (iii) HVR-H3 comprising an amino acid sequence selected from SEQ ID NO:31; and (b) a VL domain comprising (i) HVR-L1 comprising the amino acid sequence of SEQ ID NO:32; (ii) HVR-L2 comprising the amino acid sequence of SEQ ID NO:33 and (iii) HVR-L3 comprising the amino acid sequence of SEQ ID NO:34; or

G) (a) a VH domain comprising (i) HVR-H1 comprising the amino acid sequence of SEQ ID NO:37, (ii) HVR-H2 comprising the amino acid sequence of SEQ ID NO:38, and (iii) HVR-H3 comprising an amino acid sequence selected from SEQ ID NO:39; and (b) a VL domain comprising (i) HVR-L1 comprising the amino acid sequence of SEQ ID NO:40; (ii) HVR-L2 comprising the amino acid sequence of SEQ ID NO:41 and (iii) HVR-L3 comprising the amino acid sequence of SEQ ID NO:42; or

H) (a) a VH domain comprising (i) HVR-H1 comprising the amino acid sequence of SEQ ID NO:45, (ii) HVR-H2 comprising the amino acid sequence of SEQ ID NO:46, and (iii) HVR-H3 comprising an amino acid sequence selected from SEQ ID NO:47; and (b) a VL domain comprising (i) HVR-L1 comprising the amino acid sequence of SEQ ID NO:48; (ii) HVR-L2 comprising the amino acid sequence of SEQ ID NO:49 and (iii) HVR-L3 comprising the amino acid sequence of SEQ ID NO:50; or

I) (a) a VH domain comprising (i) HVR-H1 comprising the amino acid sequence of SEQ ID NO:53, (ii) HVR-H2 comprising the amino acid sequence of SEQ ID NO:54, and (iii) HVR-H3 comprising an amino acid sequence selected from SEQ ID NO:55; and (b) a VL domain comprising (i) HVR-L1 comprising the amino acid sequence of SEQ ID NO:56; (ii) HVR-L2 comprising the amino acid sequence of SEQ ID NO:57 and (iii) HVR-L3 comprising the amino acid sequence of SEQ ID NO:58; or

J) (a) a VH domain comprising (i) HVR-H1 comprising the amino acid sequence of SEQ ID NO:61, (ii) HVR-H2 comprising the amino acid sequence of SEQ ID NO:62, and (iii) HVR-H3 comprising an amino acid sequence selected from SEQ ID NO:63; and (b) a VL domain comprising (i) HVR-L1 comprising the amino acid sequence of SEQ ID NO:64; (ii) HVR-L2 comprising the amino acid sequence of SEQ ID NO:65 and (iii) HVR-L3 comprising the amino acid sequence of SEQ ID NO:66;

wherein the antibody is characterized independently by one or more of the following properties: the anti-TIM3 antibody

- induces internalization of TIM3 (in a FACS assay on TIM3 expressing RPMI8226 cells) of at least 45% after 120 Minutes at 37 °C (see Example 6)
- In on embodiment the antibody induces internalization of TIM3 (in a FACS assay on TIM3 expressing RPMI8226 cells) of at least 50% after 120 Minutes at 37 °C (see Example 6)
- In on embodiment the antibody induces internalization of TIM3 (in a FACS assay on TIM3 expressing RPMI8226 cells) of at least 55% after 120 Minutes at 37 °C (see Example 6)
- In on embodiment the antibody induces internalization of TIM3 (in a FACS assay on TIM3 expressing RPMI8226 cells) of at least 60% after 240 Minutes at 37 °C (see Example 6)
- In on embodiment the antibody induces internalization of TIM3 (in a FACS assay on TIM3 expressing RPMI8226 cells) of at least 65% after 240 Minutes at 37 °C (see Example 6)
- competes for binding to TIM3 with an anti-Tim3 antibody comprising the VH of SEQ ID NO:7 and VL of SEQ ID NO:8.
- binds to a human and cynomolgus TIM3
- shows as immunoconjugate a cytotoxic activity on TIM3 expressing cells (in one embodiment the immunoconjugates has a relative IC50 value of the cytotoxic activity as Pseudomonas exotoxin A conjugate on RPMI-8226 cells of 0.1 or lower (as measured in Example 11)
- induces interferon-gamma release (in a Mixed Lymphocyte Reaction (MLR) assay as described in Example 5).

In a further aspect of the invention, an anti-TIM3 antibody according to any of the above embodiments is a monoclonal antibody, including a chimeric, humanized or human antibody. In one embodiment, an anti-TIM3 antibody is an antibody fragment, e.g., a Fv, Fab, Fab', scFv, diabody, or F(ab')$_2$ fragment. In another embodiment, the antibody is a full length antibody, e.g., an intact IgG1 or IgG4 antibody or other antibody class or isotype as defined herein.

In a further aspect, an anti-TIM3 antibody according to any of the above embodiments may incorporate any of the features, singly or in combination, as described in Sections 1-7 below:

**1. Antibody Affinity**

In certain embodiments, an antibody provided herein has a dissociation constant KD of $\leq 1\ \mu M$, $\leq 100\ nM$, $\leq 10\ nM$, $\leq 1\ nM$, $\leq 0.1\ nM$, $\leq 0.01\ nM$, or $\leq 0.001\ nM$ (e.g. $10^{-8}$ M or less, e.g. from $10^{-8}$ M to $10^{-13}$ M, e.g., from $10^{-9}$ M to $10^{-13}$ M).

In one preferred embodiment, KD is measured using surface plasmon resonance assays using a BIACORE®) at 25°C with immobilized antigen CM5 chips at ~10 response units (RU). Briefly, carboxymethylated dextran biosensor chips (CM5, BIACORE, Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 $\mu$g/ml (~0.2 $\mu$M) before injection at a flow rate of 5 $\mu$l/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 $\mu$l/min. Association rates ($k_{on}$ or ka) and dissociation rates ($k_{off}$ or kd) are calculated using a simple one-to-one Langmuir binding model (BIACORE ® Evaluation Software version 3.2) by simultaneously fitting the association and dissociation sensorgrams. The equilibrium dissociation constant KD is calculated as the ratio kd/ka ($k_{off}/k_{on.}$) See, e.g., Chen, Y. et al., J. Mol. Biol. 293 (1999) 865-881. If the on-rate exceeds $10^6$ M$^{-1}$ s$^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophotometer (Aviv Instruments) or a 8000-series SLM-AMINCO ™ spectrophotometer (ThermoSpectronic) with a stirred cuvette.

**2. Antibody Fragments**

In certain embodiments, an antibody provided herein is an antibody fragment. Antibody fragments include, but are not limited to, Fab, Fab', Fab'-SH, F(ab')$_2$, Fv, and scFv fragments, and other fragments described below. For a review of certain antibody fragments, see Hudson, P.J. et al., Nat. Med. 9 (2003) 129-134. For a review of scFv fragments, see, e.g., Plueckthun, A., In; The Pharmacology of Monoclonal Antibodies, Vol. 113, Rosenburg and Moore (eds.), Springer-Verlag, New York (1994), pp. 269-315; see also WO 93/16185; and U.S. Patent Nos. 5,571,894 and 5,587,458. For discussion of Fab and F(ab')$_2$ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 0 404 097; WO 1993/01161; Hudson, P.J. et al., Nat. Med. 9 (2003) 129-134; and Holliger, P. et al., Proc. Natl. Acad. Sci. USA 90 (1993) 6444-6448. Triabodies and tetrabodies are also described in Hudson, P.J. et al., Nat. Med. 9 (20039 129-134).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see, e.g., U.S. Patent No. 6,248,516 B1).

Antibody fragments can be made by various techniques, including but not limited to proteolytic digestion of an intact antibody as well as production by recombinant host cells (e.g. E. coli or phage), as described herein.

### 3. Chimeric and Humanized Antibodies

In certain embodiments, an antibody provided herein is a chimeric antibody. Certain chimeric antibodies are described, e.g., in U.S. Patent No. 4,816,567; and Morrison, S.L. et al., Proc. Natl. Acad. Sci. USA 81 (1984) 6851-6855). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro, J.C. and Fransson, J., Front. Biosci. 13 (2008) 1619-1633, and are further described, e.g., in Riechmann, I. et al., Nature 332 (1988) 323-329; Queen, C. et al., Proc. Natl. Acad. Sci. USA 86 (1989) 10029-10033; US Patent Nos. 5, 821,337, 7,527,791, 6,982,321, and 7,087,409; Kashmiri, S.V. et al., Methods 36 (2005) 25-34 (describing SDR (a-CDR) grafting); Padlan, E.A., Mol. Immunol. 28 (1991) 489-498 (describing "resurfacing"); Dall'Acqua, W.F. et al., Methods 36 (2005) 43-60 (describing "FR shuffling"); and Osbourn, J. et al., Methods 36 (2005) 61-68 and Klimka, A. et al., Br. J. Cancer 83 (2000) 252-260 (describing the "guided selection" approach to FR shuffling).

Human framework regions that may be used for humanization include but are not limited to: framework regions selected using the "best-fit" method (see, e.g., Sims, M.J. et al., J. Immunol. 151 (1993) 2296-2308; framework regions derived from the consensus sequence of human antibodies of a particular subgroup of light or heavy chain variable regions (see, e.g., Carter, P. et al., Proc. Natl. Acad. Sci. USA 89 (1992) 4285-4289; and Presta, L.G. et al., J. Immunol. 151 (1993) 2623-2632); human mature (somatically mutated) framework regions or human germline framework regions (see, e.g., Almagro, J.C. and Fransson, J., Front. Biosci. 13 (2008) 1619-1633); and framework regions derived from screening FR libraries (see, e.g., Baca, M. et al., J. Biol. Chem. 272 (1997) 10678-10684 and Rosok, M.J. et al., J. Biol. Chem. 271 (19969 22611-22618).

### 4. Human Antibodies

In certain embodiments, an antibody provided herein is a human antibody. Human antibodies can be produced using various techniques known in the art. Human antibodies are described generally in van Dijk, M.A. and van de Winkel, J.G., Curr. Opin. Pharmacol. 5 (2001) 368-374 and Lonberg, N., Curr. Opin. Immunol. 20 (2008) 450-459.

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, N., Nat. Biotech. 23 (2005) 1117-1125. See also, e.g., U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor, D., J. Immunol. 133 (1984) 3001-3005; Brodeur, B.R. et al., Monoclonal Antibody Production Techniques and Applications, Marcel Dekker, Inc., New York (1987), pp. 51-63; and Boerner, P. et al., J. Immunol. 147 (1991) 86-95) Human antibodies generated via human B-cell hybridoma technology are also described in Li, J. et al., Proc. Natl. Acad. Sci. USA 103 (2006) 3557-3562. Additional methods include those described, for example, in U.S. Patent No. 7,189,826 (describing production of monoclonal human IgM antibodies from hybridoma cell lines) and Ni, J., Xiandai Mianyixue 26 (2006) 265-268 (describing human-human hybridomas). Human hybridoma technology (Trioma technology) is also described in Vollmers, H.P. and Brandlein, S., Histology and Histopathology 20 (2005) 927-937 and Vollmers, H.P. and Brandlein, S., Methods and Findings in Experimental and Clinical Pharmacology 27 (2005) 185-191.

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom, H.R. et al., Methods in Molecular Biology 178 (2001) 1-37 and further described, e.g., in the McCafferty, J. et al., Nature 348 (1990) 552-554; Clackson, T. et al., Nature 352 (1991) 624-628; Marks, J.D. et al., J. Mol. Biol. 222 (1992) 581-597; Marks, J.D. and Bradbury, A., Methods in Molecular Biology 248 (2003) 161-175; Sidhu, S.S. et al., J. Mol. Biol. 338 (2004) 299-310; Lee, C.V. et al., J. Mol. Biol. 340 (2004) 1073-1093; Fellouse, F.A., Proc. Natl. Acad. Sci. USA 101 (2004) 12467-12472; and Lee, C.V. et al., J. Immunol. Methods 284 (2004) 119-132.

In certain phage display methods, repertoires of VH and VL genes are separately cloned by polymerase chain reaction (PCR) and recombined randomly in phage libraries, which can then be screened for antigen-binding phage as described in Winter, G. et al., Ann. Rev. Immunol. 12 (1994) 433-455. Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self-antigens without any immunization as described by Griffiths, A.D. et al., EMBO J. 12 (1993) 725-734. Finally, naive libraries can also be made synthetically by cloning non-rearranged V-gene segments from stem cells, and using PCR primers containing random sequence to encode the highly variable CDR3 regions and to accomplish rearrangement *in vitro*, as described by Hoogenboom, H.R. and Winter, G., J. Mol. Biol. 227 (1992) 381-388. Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for TIM3and the other is for any other antigen. In certain embodiments, bispecific antibodies may bind to two different epitopes of TIM3. Bispecific antibodies may also be used to localize cytotoxic agents to cells which express TIM3. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein, C. and Cuello, A.C., Nature 305 (1983) 537-540, WO 93/08829, and Traunecker, A. et al., EMBO J. 10 (1991) 3655-3659), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004); cross-linking two or more antibodies or fragments (see, e.g., US Patent No. 4,676,980, and Brennan, M. et al., Science 229 (1985) 81-83); using leucine zippers to produce bi-specific antibodies (see, e.g., Kostelny, S.A. et al., J. Immunol. 148 (1992) 1547-1553; using "diabody" technology for making bispecific antibody fragments (see, e.g., Holliger, P. et al., Proc. Natl. Acad. Sci. USA 90 (1993) 6444-6448); and using single-chain Fv (scFv) dimers (see, e.g. Gruber, M et al., J. Immunol. 152 (1994) 5368-5374); and preparing trispecific antibodies as described, e.g., in Tutt, A. et al., J. Immunol. 147 (1991) 60-69).

Engineered antibodies with three or more functional antigen binding sites, including "Octopus antibodies," are also included herein (see, e.g. US 2006/0025576).

The antibody or fragment herein also includes a "Dual Acting Fab" or "DAF" comprising an antigen binding site that binds to TIM3as well as another, different antigen (see, US 2008/0069820, for example).

The antibody or fragment herein also includes multispecific antibodies described in WO 2009/080251, WO 2009/080252, WO 2009/080253, WO 2009/080254, WO 2010/112193, WO 2010/115589, WO 2010/136172, WO 2010/145792, and WO 2010/145793, WO2011/117330, WO2012/025525, WO2012/025530, WO2013/026835, WO2013/026831, WO2013/164325, or WO 2013/174873.

## 7. Antibody Variants

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

## a) Substitution, Insertion, and Deletion Variants

In certain embodiments, antibody variants having one or more amino acid substitutions are provided. Sites of interest for substitutional mutagenesis include the HVRs and FRs. Exemplary changes are provided in Table 1 under the heading of "exemplary substitutions", and as further described below in reference to amino acid side chain classes. Conservative substitutions are shown in Table 1 under the heading of "preferred substitutions". Amino acid substitutions may be introduced into an antibody of interest and the products screened for a desired activity, e.g., retained/improved antigen binding, decreased immunogenicity, or improved ADCC or CDC.

**TABLE 1**

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |

(continued)

| Original Residue | Exemplary Substitutions | Preferred Substitutions |
|---|---|---|
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;

(2) neutral hydrophilic: Cys, Ser, Thr, Asn, Gln;

(3) acidic: Asp, Glu;

(4) basic: His, Lys, Arg;

(5) residues that influence chain orientation: Gly, Pro;

(6) aromatic: Trp, Tyr, Phe.

Non-conservative substitutions will entail exchanging a member of one of these classes for another class.

One type of substitutional variant involves substituting one or more hypervariable region residues of a parent antibody (*e.g.* a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, P.S., Methods Mol. Biol. 207 (2008) 179-196), and/or SDRs (a-CDRs), with the resulting variant VH or VL being tested for binding affinity. Affinity maturation by constructing and reselecting from secondary libraries has been described, e.g., in Hoogenboom, H.R. et al. in Methods in Molecular Biology 178 (2002) 1-37. In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham, B.C. and Wells, J.A., Science 244 (1989) 1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

**b) Fc region variants**

In certain embodiments, one or more amino acid modifications may be introduced into the Fc region of an antibody provided herein, thereby generating an Fc region variant. The Fc region variant may comprise a human Fc region sequence (*e.g.*, a human IgG1, IgG2, IgG3 or IgG4 Fc region) comprising an amino acid modification (*e.g.* a substitution) at one or more amino acid positions.

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields, R.L. et al., J. Biol. Chem. 276 (2001) 6591-6604)

In one embodiment the invention such antibody is a IgG1 with mutations L234A and L235A or with mutations L234A, L235A and P329G. In another embodiment or IgG4 with mutations S228P and L235E or S228P, L235E or and P329G (numbering according to EU index of Kabat et al , Kabat et al., Sequences of Proteins of Immunological Interest, 5th Ed. Public Health Service, National Institutes of Health, Bethesda, MD, 1991).

Antibodies with increased half lives and improved binding to the neonatal Fc receptor (FcRn), which is responsible for the transfer of maternal IgGs to the fetus (Guyer, R.L. et al., J. Immunol. 117 (1976) 587-593, and Kim, J.K. et al., J. Immunol. 24 (1994) 2429-2434), are described in US 2005/0014934. Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan, A.R. and Winter, G., Nature 322 (1988) 738-740; US 5,648,260; US 5,624,821; and WO 94/29351 concerning other examples of Fc region variants.

**c) Cysteine engineered antibody variants**

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

**d) Antibody Derivatives**

In certain embodiments, an antibody provided herein may be further modified to contain additional non-proteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide copolymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and non-proteinaceous moiety that may be selectively heated by exposure to radiation are provided. In one embodiment, the non-proteinaceous moiety is a carbon nanotube (Kam, N.W. et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed.

## B. Recombinant Methods and Compositions

Antibodies may be produced using recombinant methods and compositions, e.g., as described in U.S. Patent No. 4,816,567. In one embodiment, isolated nucleic acid encoding an anti-TIM3antibody described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp20 cell). In one embodiment, a method of making an anti-TIM3antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-TIM3 antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., US 5,648,237, US 5,789,199, and US 5,840,523. (See also Charlton, K.A., In: Methods in Molecular Biology, Vol. 248, Lo, B.K.C. (ed.), Humana Press, Totowa, NJ (2003), pp. 245-254, describing expression of antibody fragments in *E. coli*.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, T.U., Nat. Biotech. 22 (2004) 1409-1414; and Li, H. et al., Nat. Biotech. 24 (2006) 210-215.

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham, F.L. et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather, J.P. et al., Annals N.Y. Acad. Sci. 383 (1982) 44-68; MRC 5 cells; and FS4 cells. Other useful mammalian host cell lines include Chinese hamster ovary (CHO) cells, including DHFR⁻ CHO cells (Urlaub, G. et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

## C. Assays

Anti-TIM3 antibodies provided herein may be identified, screened for, or characterized for their physical/chemical properties and/or biological activities by various assays known in the art.

## 1. Binding assays and other assays

In one aspect, an antibody of the invention is tested for its antigen binding activity, e.g., by known methods such as ELISA, Western blot, etc.

In another aspect, competition assays may be used to identify an antibody that competes with Tim3_0016 (comprising a VH sequence of SEQ ID NO:7 and a VL sequence of SEQ ID NO:8) for binding to TIM3 (or alternatively with the Tim3_0016 variant antibody Tim3_0018 with the identical 6 HVRs) . Thus one embodiment of the invention is antibody which competes for binding to human TIM3 with an anti-TIM3 antibody comprising all 3 HVRs of VH sequence of SEQ ID NO:7 and all 3 HVRs of VL sequence of SEQ ID NO:8). In certain embodiments, such a competing antibody binds to the same epitope (e.g., a linear or a conformational epitope) that is bound by anti-TIM3 antibody Tim3_0016. Detailed exemplary methods for mapping an epitope to which an antibody binds are provided in Morris, G.E. (ed.), Epitope Mapping Protocols, In: Methods in Molecular Biology, Vol. 66, Humana Press, Totowa, NJ (1996).

In an exemplary competition assay, immobilized TIM3(-ECD) is incubated in a solution comprising a first labeled antibody that binds to TIM3 (e.g., anti-TIM3 antibody aTim3_0016) and a second unlabeled antibody that is being tested for its ability to compete with the first antibody for binding to TIM3. The second antibody may be present in a hybridoma supernatant. As a control, immobilized TIM3 is incubated in a solution comprising the first labeled antibody but not the second unlabeled antibody. After incubation under conditions permissive for binding of the first antibody to TIM3, excess unbound antibody is removed, and the amount of label associated with immobilized TIM3 is measured. If the amount of label associated with immobilized TIM3 is substantially reduced in the test sample relative to the control sample, then that indicates that the second antibody is competing with the first antibody for binding to TIM3. See Harlow, E. and Lane, D., Antibodies: A Laboratory Manual, Chapter 14, Cold Spring Harbor Laboratory, Cold Spring Harbor, NY (1988). For another exemplary competition assay see Example 4.

## 2. Activity assays

In one aspect, assays are provided for identifying anti-TIM3 antibodies thereof having biological activity. Biological activity may include, e.g., TIM3 receptor internalization, or cytokine release, or cytotoxic activity (as immunoconjugates conjugated to a toxin), or cynomolgus binding crossreactivity, as well as binding to different cell types. Antibodies having such biological activity in vivo and/or in vitro are also provided.

In certain embodiments, an antibody of the invention is tested for such biological activity as described e.g. in Examples 5 to 15.

## D. Immunoconjugates (Cancer only or modify for target)

The invention also provides immunoconjugates comprising an anti-TIM3 antibody herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (see US 5,208,020, US 5,416,064 and EP 0 425 235 B1); an auristatin such as monomethyl auristatin drug moieties DE and DF (MMAE and MMAF) (see US 5,635,483, US 5,780,588, and US 7,498,298); a dolastatin; a calicheamicin or derivative thereof (see US 5,712,374, US 5,714,586, US 5,739,116, US 5,767,285, US 5,770,701, US 5,770,710, US 5,773,001, and US 5,877,296; Hinman, L.M. et al., Cancer Res. 53 (1993) 3336-3342; and Lode, H.N. et al., Cancer Res. 58 (1998) 2925-2928); an anthracycline such as daunomycin or doxorubicin (see Kratz, F. et al., Curr. Med. Chem. 13 (2006) 477-523; Jeffrey, S.C. et al., Bioorg. Med. Chem. Lett. 16 (2006) 358-362; Torgov, M.Y. et al., Bioconjug. Chem. 16 (2005) 717-721; Nagy, A. et al., Proc. Natl. Acad. Sci. USA 97 (2000) 829-834; Dubowchik, G.M. et al., Bioorg. & Med. Chem. Letters 12 (2002) 1529-1532; King, H.D. et al., J. Med. Chem. 45 (20029 4336-4343; and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another embodiment, an immunoconjugate comprises an antibody as described herein conjugated to a Pseudomonas exotoxin A (or variants) thereof. Pseudomonas exotoxin A (PE) is a bacterial toxin with cytotoxic activity that may be effective for destroying or inhibiting the growth of undesirable cells, e.g., cancer cells. Accordingly, PE may be useful for treating or preventing diseases such as, e.g., cancer. Several deimmunized Pseudomonas exotoxins (PE) are known in art. The domain II deleted versions (for example, PE24) may be less immunogenic and may cause fewer side effects (such as, for example, capillary leak syndrome and hepatotoxicity) as compared to PE38, which contains domain II. Different furin cleavable linkers may be employed in PE24 variants. Such deimmunized Pseudomonas exotoxins (PE) are described in, for example, International Patent Application Publications WO2005052006, WO2007016150, WO2007014743, WO2007031741, WO200932954, WO201132022, WO2012/154530, and WO 2012/170617. The term "a Pseudomonas exotoxin A" as used herein encompasses wildtype and deimmunized Pseudomonas exotoxins (PE). In one preferred embodiment "a Pseudomonas exotoxin A" refers to a deimmunized PE24 variant as e.g. described in but not limited to WO2009/32954, WO2011/32022, WO2012/154530, WO 2012/170617, Liu W, et al, PNAS 109 (2012) 11782-11787, Mazor R, et al PNAS 111 (2014) 8571-8576 and Alewine C, et al, Mol Cancer Ther. (2014) 2653-61. In one preferred embodiment the "a Pseudomonas exotoxin A" comprises the amino acid sequences of SEQ ID NO:69 or comprises the amino acid sequences of SEQ ID NO:70 (their preparation is also described in Mazor R, et al PNAS 111 (2014) 8571-8576 and Alewine C, et al, Mol Cancer Ther. (2014) 2653-61).

In another embodiment, an immunoconjugate comprises an antibody as described herein conjugated to a Amatoxin or variants thereof. Amatoxins (a-amanitin, β-amanitin, amanin) are cyclic peptides composed of 8 amino acids. They can be isolated from Amanita phalloides mushrooms or prepared from the building blocks by synthesis. Amatoxins inhibit specifically the DNA-dependent RNA polymerase II of mammalian cells, and by this transcription and protein biosynthesis of the cells affected. Inhibition of transcription in a cell causes stop of growth and proliferation. Though not covalently bound, the complex between amanitin and RNA-polymerase II is very tight (KD = 3nM). Dissociation of amanitin from the enzyme is a very slow process what makes recovery of an affected cell unlikely. When in a cell the inhibition of transcription will last too long, the cell undergoes programmed cell death (apoptosis), as shown in cultures of Jurkat cells incubated with $\alpha$-amanitin, or, with much higher efficiency, in Jurkat cells incubated with the membrane-permeable O-methyl-$\alpha$-amanitin oleate. In one preferred embodiment term "Amatoxin" as used herein refers to an alpha-amanitin or variant thereof as described e.g. in WO2010/115630, WO2010/115629, WO2012/119787, WO2012/041504, and WO2014135282 with preferred variants described in WO2012/041504( e.g. conjugated via the 6' C-atom of amatoxin amino acid 4, particularly via an oxygen atom bound to the 6' C-atom of amatoxin amino acid, and wherein the TIM3 antibody is connected by a linker via a urea moiety) and WO2014135282.

In another embodiment, an immunoconjugate comprises an antibody as described herein conjugated to a radioactive atom to form a radioconjugate. A variety of radioactive isotopes are available for the production of radiocon-jugates. Examples include $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu. When the radioconjugate is used for detection, it may comprise a radioactive atom for scintigraphic studies, for example $TC^{99m}$ or $I^{123}$, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta, E.S. et al., Science 238 (1987) 1098-1104. Carbon-14-labeled 1-isothiocyanatobenzyl-3-methyldiethylene triamine pentaacetic acid (MX-DTPA) is an exemplary chelating agent for conjugation of radionucleotide to the antibody. See WO 94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari, R.V. et al., Cancer Res. 52 (1992) 127-131; U.S. Patent No. 5,208,020) may be used.

The immunoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

## E. Methods and Compositions for Diagnostics and Detection

In certain embodiments, any of the anti-TIM3antibodies provided herein is useful for detecting the presence of TIM3 in a biological sample. The term "detecting" as used herein encompasses quantitative or qualitative detection. In certain embodiments, a biological sample comprises a cell or tissue, such as AML stem cancer cells.

In one embodiment, an anti-TIM3 antibody for use in a method of diagnosis or detection is provided. In a further aspect, a method of detecting the presence of TIM3 in a biological sample is provided. In certain embodiments, the method comprises contacting the biological sample with an anti-TIM3 antibody as described herein under conditions permissive for binding of the anti-TIM3 antibody to TIM3, and detecting whether a complex is formed between the anti-TIM3 antibody and TIM3. Such method may be an *in vitro* or *in vivo* method. In one embodiment, an anti-TIM3 antibody is used to select subjects eligible for therapy with an anti-TIM3 antibody, e.g. where TIM3 is a biomarker for selection of patients.

Exemplary disorders that may be diagnosed using an antibody of the invention include cancer including different form of hematological cancers like AML or multiple myelomas (MM).

In certain embodiments, labeled anti-TIM3 antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes $^{32}$P, $^{14}$C, $^{125}$I, $^{3}$H, and $^{131}$I, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

## F. Pharmaceutical Formulations

Pharmaceutical formulations of an anti-TIM3 antibody as described herein are prepared by mixing such antibody having the desired degree of purity with one or more optional pharmaceutically acceptable carriers (Remington's Pharmaceutical Sciences, 16th edition, Osol, A. (ed.) (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyl dimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as poly(vinylpyrrolidone); amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counterions such as sodium; metal complexes (*e.g.* Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rhuPH20 (HYLENEX®, Baxter International, Inc.). Certain exemplary sHASEGPs and methods of use, including rhuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO 2006/044908, the latter formulations including a histidine-acetate buffer.

The formulation herein may also contain more than one active ingredients as necessary for the particular indication being treated, preferably those with complementary activities that do not adversely affect each other. For example, it may be desirable to further provide [[list drugs that might be combined with the anti-TIM3 antibody]]. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methyl methacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semi-permeable matrices of solid hydrophobic polymers containing the antibody, which matrices are in the form of shaped articles, e.g. films, or microcapsules.

The formulations to be used for *in vivo* administration are generally sterile. Sterility may be readily accomplished, e.g., by filtration through sterile filtration membranes.

## G. Therapeutic Methods and Compositions

Any of the anti-TIM3 antibodies (or antigen binding proteins) or immunoconjugates of the anti-TIM3 antibodies (or antigen binding protein) conjugated to a cytotoxic agent, provided herein may be used in therapeutic methods.

In one aspect, an anti-TIM3 antibody or immunoconjugate of the anti-TIM3 antibody conjugated to a cytotoxic agent for use as a medicament is provided. In further aspects, an anti-TIM3 antibody or immunoconjugate of the anti-TIM3 antibody conjugated to a cytotoxic agent for use in treating cancer is provided. In certain embodiments, an anti-TIM3 antibody or immunoconjugates of the anti-TIM3 antibody conjugated to a cytotoxic agent for use in a method of treatment is provided. In certain embodiments, the invention provides an anti-TIM3 antibody or immunoconjugate of the anti-TIM3 antibody conjugated to a cytotoxic agent for use in a method of treating an individual having cancer comprising administering to the individual an effective amount of the anti-TIM3 antibody or the immunoconjugate of the anti-TIM3 antibody conjugated to a cytotoxic agent.

In further embodiments, the invention provides an anti-TIM3 antibody or immunoconjugate of the anti-TIM3 antibody conjugated to a cytotoxic agent for use in inducing apoptosis in a cancer cell/ or inhibiting cancer cell proliferation. In certain embodiments, the invention provides an anti-TIM3 antibody or immunoconjugate of the anti-TIM3 antibody conjugated to a cytotoxic agent for use in a method of inducing apoptosis in a cancer cell/ or inhibiting cancer cell proliferation in an individual comprising administering to the individual an effective of the anti-TIM3 antibody or immunoconjugate of the anti-TIM3 antibodies conjugated to a cytotoxic agent to induce apoptosis in a cancer cell/ or to inhibit cancer cell proliferation.

In further embodiments, the invention provides an anti-TIM3 antibody for use as immunestimulatory agent/ or stimulating IFN gamma secretion. In certain embodiments, the invention provides an anti-TIM3 antibody for use in a method of immunestimulation/ or stimulating IFN gamma secretion in an individual comprising administering to the individual an effective of the anti-TIM3 antibody for immunestimulation/ or stimulating IFN gamma secretion.

An "individual" according to any of the above embodiments is preferably a human. In a further aspect, the invention provides for the use of an anti-TIM3 antibody or an immunoconjugate of the anti-TIM3 antibody conjugated to a cytotoxic agent in the manufacture or preparation of a medicament. In one embodiment, the medicament is for treatment of cancer. In a further embodiment, the medicament is for use in a method of treating cancer comprising administering to an individual having cancer an effective amount of the medicament. In a further embodiment, the medicament is for inducing apoptosis in a cancer cell/ or inhibiting cancer cell proliferation. In a further embodiment, the medicament is for use in a method of inducing apoptosis in a cancer cell/ or inhibiting cancer cell proliferation in an individual suffering from cancer comprising administering to the individual an amount effective of the medicament to induce apoptosis in a cancer cell/ or to inhibit cancer cell proliferation. An "individual" according to any of the above embodiments may be a human.

In a further aspect, the invention provides a method for treating cancer. In one embodiment, the method comprises administering to an individual having cancer an effective amount of an anti-TIM3 antibody. An "individual" according to any of the above embodiments may be a human.

In a further aspect, the invention provides a method for inducing apoptosis in a cancer cell/ or inhibiting cancer cell proliferation in an individual suffering from cancer. In one embodiment, the method comprises administering to the individual an effective amount of an anti-TIM3 antibody or an immunoconjugate of the anti-TIM3 antibody conjugated to a cytotoxic compound to induce apoptosis in a cancer cell/ or to inhibit cancer cell proliferation in the individual suffering from cancer. In one embodiment, an "individual" is a human.

In a further aspect, the invention provides pharmaceutical formulations comprising any of the anti-TIM3 antibodies provided herein, e.g., for use in any of the above therapeutic methods. In one embodiment, a pharmaceutical formulation comprises any of the anti-TIM3 antibodies provided herein and a pharmaceutically acceptable carrier.

An antibody of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody of the invention (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 $\mu$g/kg to 15 mg/kg (e.g. 0.5mg/kg - 10 mg/kg) of antibody can be an initial candidate dosage for administration to the patient, whether, for example, by one or more separate administrations, or by continuous infusion. One typical daily dosage might range from about 1 $\mu$g/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (*e.g.* such that the patient receives from about two to about twenty, or *e.g.* about six doses of the antibody). An initial higher loading dose, followed by one or more lower doses may be administered. An exemplary dosing regimen comprises administering an initial loading dose of about 4 mg/kg, followed by a weekly maintenance dose of about 2 mg/kg of the antibody. However, other dosage regimens may be useful. The progress of this therapy is easily monitored by conventional techniques and assays.

It is understood that any of the above formulations or therapeutic methods may be carried out using an immunoconjugate of the invention in place of or in addition to an anti-TIM3 antibody.

It is understood that any of the above formulations or therapeutic methods may be carried out using an immunoconjugate of the invention in place of or in addition to an anti-TIM3 antibody.

### III. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is used for treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the invention in place of or in addition to an anti-TIM3 antibody.

**Description of the amino acid sequences**

| | |
|---|---|
| SEQ ID NO: 1 | heavy chain HVR-H1, Tim3_0016 |
| SEQ ID NO: 2 | heavy chain HVR-H2, Tim3_0016 |
| SEQ ID NO: 3 | heavy chain HVR-H3, Tim3_0016 |
| SEQ ID NO: 4 | light chain HVR-L1, Tim3_0016 |

(continued)

SEQ ID NO: 5          light chain HVR-L2, Tim3_0016
SEQ ID NO: 6          light chain HVR-L3, Tim3_0016
SEQ ID NO: 7          heavy chain variable domain VH, Tim3_0016
SEQ ID NO: 8          light chain variable domain VL, Tim3_0016
SEQ ID NO: 9          heavy chain variable domain VH, Tim3_0016 variant (0018)
SEQ ID NO: 10        light chain variable domain VL, Tim3_0016 variant (0018)
SEQ ID NO: 11        light chain HVR-L1, Tim3_0016_HVR-L1 variant 1 NQ (removal of glycosylation site by N to Q mutation)
SEQ ID NO: 12        light chain HVR-L1, Tim3_0016_HVR-L1 variant 2_NS (removal of glycosylation site by N to S mutation)

SEQ ID NO: 13        heavy chain HVR-H1, Tim3_0021
SEQ ID NO: 14        heavy chain HVR-H2, Tim3_0021
SEQ ID NO: 15        heavy chain HVR-H3, Tim3_0021
SEQ ID NO: 16        light chain HVR-L1, Tim3_0021
SEQ ID NO: 17        light chain HVR-L2, Tim3_0021
SEQ ID NO: 18        light chain HVR-L3, Tim3_0021
SEQ ID NO: 19        heavy chain variable domain VH, Tim3_0021
SEQ ID NO: 20        light chain variable domain VL, Tim3_0021

SEQ ID NO: 21        heavy chain HVR-H1, Tim3_0022
SEQ ID NO: 22        heavy chain HVR-H2, Tim3_0022
SEQ ID NO: 23        heavy chain HVR-H3, Tim3_0022
SEQ ID NO: 24        light chain HVR-L1, Tim3_0022
SEQ ID NO: 25        light chain HVR-L2, Tim3_0022
SEQ ID NO: 26        light chain HVR-L3, Tim3_0022
SEQ ID NO: 27        heavy chain variable domain VH, Tim3_0022
SEQ ID NO: 28        light chain variable domain VL, Tim3_0022

SEQ ID NO: 29        heavy chain HVR-H1, Tim3_0026
SEQ ID NO: 30        heavy chain HVR-H2, Tim3_0026
SEQ ID NO: 31        heavy chain HVR-H3, Tim3_0026
SEQ ID NO: 32        light chain HVR-L1, Tim3_0026
SEQ ID NO: 33        light chain HVR-L2, Tim3_0026
SEQ ID NO: 34        light chain HVR-L3, Tim3_0026
SEQ ID NO: 35        heavy chain variable domain VH, Tim3_0026
SEQ ID NO: 36        light chain variable domain VL, Tim3_0026
SEQ ID NO: 37        heavy chain HVR-H1, Tim3_0028
SEQ ID NO: 38        heavy chain HVR-H2, Tim3_0028
SEQ ID NO: 39        heavy chain HVR-H3, Tim3_0028
SEQ ID NO: 40        light chain HVR-L1, Tim3_0028
SEQ ID NO: 41        light chain HVR-L2, Tim3_0028
SEQ ID NO: 42        light chain HVR-L3, Tim3_0028
SEQ ID NO: 43        heavy chain variable domain VH, Tim3_0028
SEQ ID NO: 44        light chain variable domain VL, Tim3_0028

SEQ ID NO: 45        heavy chain HVR-H1, Tim3_0030
SEQ ID NO: 46        heavy chain HVR-H2, Tim3_0030
SEQ ID NO: 47        heavy chain HVR-H3, Tim3_0030
SEQ ID NO: 48        light chain HVR-L1, Tim3_0030
SEQ ID NO: 49        light chain HVR-L2, Tim3_0030

(continued)

| SEQ ID NO: 50 | light chain HVR-L3, Tim3_0030 |
| SEQ ID NO: 51 | heavy chain variable domain VH, Tim3_0030 |
| SEQ ID NO: 52 | light chain variable domain VL, Tim3_0030 |
| | |
| SEQ ID NO: 53 | heavy chain HVR-H1, Tim3_0033 |
| SEQ ID NO: 54 | heavy chain HVR-H2, Tim3_0033 |
| SEQ ID NO: 55 | heavy chain HVR-H3, Tim3_0033 |
| SEQ ID NO: 56 | light chain HVR-L1, Tim3_0033 |
| SEQ ID NO: 57 | light chain HVR-L2, Tim3_0033 |
| SEQ ID NO: 58 | light chain HVR-L3, Tim3_0033 |
| SEQ ID NO: 59 | heavy chain variable domain VH, Tim3_0033 |
| SEQ ID NO: 60 | light chain variable domain VL, Tim3_0033 |
| | |
| SEQ ID NO: 61 | heavy chain HVR-H1, Tim3_0038 |
| SEQ ID NO: 62 | heavy chain HVR-H2, Tim3_0038 |
| SEQ ID NO: 63 | heavy chain HVR-H3, Tim3_0038 |
| SEQ ID NO: 64 | light chain HVR-L1, Tim3_0038 |
| SEQ ID NO: 65 | light chain HVR-L2, Tim3_0038 |
| SEQ ID NO: 66 | light chain HVR-L3, Tim3_0038 |
| SEQ ID NO: 67 | heavy chain variable domain VH, Tim3_0038 |
| SEQ ID NO: 68 | light chain variable domain VL, Tim3_0038 |
| SEQ ID NO: 69 | an exemplary Pseudomonas exotoxin A variant 1 (deimmunized PE24 example) |
| SEQ ID NO: 70 | an exemplary Pseudomonas exotoxin A variant 2 (deimmunized PE24 example) |
| | |
| SEQ ID NO: 71 | human kappa light chain constant region |
| SEQ ID NO: 72 | human lambda light chain constant region |
| SEQ ID NO: 73 | human heavy chain constant region derived from IgG1 |
| SEQ ID NO: 74 | human heavy chain constant region derived from IgG1 with mutations L234A and L235A |
| SEQ ID NO: 75 | human heavy chain constant region derived from IgG1 with mutations L234A, L235A and P329G |
| SEQ ID NO: 76 | human heavy chain constant region derived from IgG4 |
| SEQ ID NO: 77 | exemplary human TIM3 sequences |
| SEQ ID NO: 78 | human TIM3 Extracellular Domain (ECD) |
| SEQ ID NO: 79 | VH humanized version of Tim3_0016 variant (0018) (= Tim3-0433) |
| SEQ ID NO: 80 | VL humanized version of Tim3_0016 variant (0018) (= Tim3-0433) |
| SEQ ID NO: 81 | VH humanized version of Tim3_0016 variant (0018) (= Tim3-0434) |
| SEQ ID NO: 82 | VL humanized version of Tim3_0016 variant (0018) (= Tim3-0434) |
| SEQ ID NO: 83 | VH humanized version of Tim3-0028 (= Tim3-0438) |
| SEQ ID NO: 84 | VL humanized version of Tim3-0028 (= Tim3-0438) |
| SEQ ID NO: 85 | VH humanized version of Tim3-0028 (= Tim3-0443) |
| SEQ ID NO: 86 | VL humanized version of Tim3-0028 (= Tim3-0443) |

In the following the amino acid sequences of the VH und VL domains including marked HVRs ( HVRs in bold, underlined letters) of anti-TIM3 antibodies Tim3-0016, Tim3-0016 variant (0018) and its humanized versions Tim3-0433 and Tim3-0434, Tim3-0021, Tim3-0022, Tim3-0026, Tim3-0028 and its humanized versions Tim3-0438 and Tim3-0443, Tim3-0030, and Tim3-0033, Tim3-0038 are listed:

**VH Tim3_0016:**

1  qvtlkesgpg  ilqpsqtl**rl**  tcsfs**gfsls**  **tsgm**svgwir qpsgkglewl

51  ahiw**lnd**dvf  fnpalksrlt  iskdtsnnqv  flqiasvvta dtatyycvra

101  **ngylyald**yw  gqg**t**svtvss

**VL Tim3_0016:**

1  qivltqspai  msaspgqkvt  itcsa**sssvn**  **y**tqwyqqklg sspklwiy**da**

51  **f**klapgvpar  fsgsgtgtsy  sltissmeae  daasyfchq**w** **ssypw**tfggg

101  tkleik

**VH Tim3_0018(= VL Tim3_0016 variant):**

1  qvtlkesgpg  ilqpsqtlsl  tcsfs**gfsls**  **tsgm**svgwir qpsgkglewl

51  ahiw**lnd**dvf  fnpalkrrlt  iskdtsnnqv  flqiasvvta dtatyycvra

101  **ngylyald**yw  gqgisvtvss

**VL Tim3_0018 (= VL Tim3_0016 variant):**

1  qivltqspai  msaspgqkvt  itcsa**sssvn**  **y**tqwyqqklg sspklwiy**da**

51  **f**klapgvpar  fsgsgtgtsy  sltissmeae  daasyfchq**w** **ssypw**tfggg

101  tkleik

**VH humanized version of Tim3_0016 variant (0018) (= Tim3-0433)**

1  qitlkesgpt  lvkptqtltl  tctfs**gfsls**  **tsgm**svgwir qppgkglewl

51  ahiw**lnd**dvf  fnpalksrlt  itkdtsknqv  vltmtnmdpv dtatyycvra

101  **ngylyald**yw  gqgtlvtvss

**VL humanized version of Tim3_0016 variant (0018) (= Tim3-0433)**

```
  1    ettltqspaf  msatpgdkvn  iacsasssvs  ytqwyqqkpg
eapklwiyda

 51    fklapgippr  fsgsgygtdf  tltinniese  daayyfchqw
ssypwtfgqg

101    tkleik
```

**VH humanized version of Tim3_0016 variant (0018) (= Tim3-0434)**

```
  1    qitlkesgpt  lvkptqtltl  tctfsgfsls  tsgmsvgwir
qppgkglewl

 51    ahiwlnddvf  fnpalksrlt  itkdtsknqv  vltmtnmdpv
dtatyycvra

101    ngylyaldyw  gqgtlvtvss
```

**VL humanized version of Tim3_0016 variant (0018) (= Tim3-0434**

```
  1    diqltqspsf  lsasvgdrvt  itcsasssvs  ytqwyqqkpg
kapklwiyda

 51    fklapgvpsr  fsgsgsgtef  tltisslqpe  dfatyfchqw
ssypwtfgqg

101    tkleik
```

**VH Tim3_0021:**

```
  1    QVQLQQSGPQ  LVRPGASVQI  SCKASGYSFT  SYLLHWLKQR
PGQGLEWIGM

 51    IDPSDSETRL  NQKFKDKATL  TVDKSSSTAY  MQLSSPTSED
SAVYYCARDG

101    YYAWYYFDCW  GQGTTLTVSS
```

**VL Tim3_0021:**

1    DIVLTQSPAT   LSVTPGDRVS   LSCRA**SQSIG**   **NN**LHWYQQKS
HESPRLLIK**Y**

51   **AS**HSISGIPS   KFSGTGSGTD   FTLSFNSVET   EDFGMYFCQQ
**SNSWPL**TFGA

101 GTKLELK

**VH Tim3_0022:**

1    EVQLQESGPS   LVKPSQTLSL   TCSVT**GDSIA**   SAYWNWIRKF
PGNKLEYMGY

51   IN**YSG**STYYN   PSLKSRISIT   RDTSQNQYYL   QLNSVTTEDT
ATYYCVTG**DY**

101 **FD**YWGRGTTL TVSS

**VL aTim3_0022:**

1    DIQMTQSPSS   LSAYLGGKVT   ITCKA**RQDVR**   **KN**IGWYQHKP
GKGPRLLIW**Y**

51   **TS**TLQSGIPS   RFSGSGSGRD   YSFNINNLEP   EDIATYYCLQ
**YDNLPF**TFGT

101 GTKLEIR

**VH Tim3_0026:**

1    QIQLVQSGPE   LKKPGETVKI   SCKAS**GYTFT**   DYSMHWVKQA
PGRGLKWMGY

51   INT**ETY**EPTF   GADFKGRFAF   SLDTSATTAY   LQINSLKTED
TATFFCGGG**G**

101 **YPA**YWGQGTV VIVSA

**VL Tim3_0026**

1    DVLMTQTPLS   LPVSLGDQAS   ISCRS**SRTIL**   **HSSGNTY**LEW
YLQKPGQSPK

51   LLIY**KVS**NRF   SGVPDRFSGS   GSGTDFTLNI   SRVEAEDLGV
YYCFQ**DSHVP**

101 **F**TFGTGTKLE IK

35

**VH Tim3_0028:**

```
        1    EVQLQQSVAE   LVRPGASVKL   SCTASGFNIK   TTYMHWVKQR
PEQGLEWIGR

       51    IDPADDNTKY   APKFQGKATI   TADTSSNTAY   LQLSSLTSED
AAIYYCVRDF

      101    GYVAWFAYWG   QGTLVTFSA
```

**VL Tim3_0028:**

```
        1    NIVMTPTPKF   LPVSSGDRVT   MTCRASQSVD   NYVAWYQQKP
GQSPKLLIYY

       51    ASNRYIGVPD   RFTGSGSGTD   FTFTISSVQV   EDLAVYFCQQ
HYSSPYTFGS

      101    GTKLEIK
```

**VH humanized version of Tim3-0028 (= Tim3-0438)**

```
        1    evqlvesggg   lvqpggslrl   scaasgfnik   ttymhwvrqa
pgkglewvgr

       51    idpaddntky   apkfqgkati   sadtskntay   lqmnslraed
tavyycvrdf

      101    gyvawfaywg   qgtlvtvss
```

**VL humanized version of Tim3-0028 (= Tim3-0438)**

```
        1    divmtqspls   lpvtpgepas   iscrasqsvd   nyvawylqkp
gqspqlliyy

       51    asnryigvpd   rfsgsgsgtd   ftlkisrvea   edvgvyycqq
hysspytfgq

      101    gtkveik
```

**VH humanized version of Tim3-0028 (= Tim3-0443)**

evqlvesggg lvqpggslrl scaas**gfnik** **tt**ymhwvrqa pgkglewvgr

idp**add**ntky apkfqgkati sadtskntay lqmnslraed tavyycvrd**f**

**gyvawfa**ywg qgtlvtfss

**VL humanized version of Tim3-0028 (= Tim3-0443)**

divmtqspls lpvtpgepas iscra**sqsvd** **ny**vawylqkp gqspqlliy**y**

**as**nryigvpd rfsgsgsgtd ftlkisrvea edvgvyycqq **hysspy**tfgq

gtkveik

**VH aTim3_0030:**

QIQLVQSGPE LKKPGETVKI SCKAS**GYPFS** **EY**SIHWVKQA PGKGLKWMVY

VNT**ETG**QPIV GDDFRGRFVL SLETSASTAY LQINNLKNED TATYFCGGG**G**

**YPA**YWGQGTL VTVSA

**VL aTim3 0030:**

DVLMTQTPLS LPVSLGDQAS ISCRS**SRSIV** **HSSGNTY**LEW YLQKPGQSPK

LLIY**KVS**NRF SGVPDRFSGS GSGTDFTLNI SRVEAEDLGV YYCFQ**DSHVP**

**F**TFGTGTKLE IK

**VH aTim3_0033:**

QGQMHQSGAE LVKPGSSVKL SCKTS**GFTFS** **SS**FISWLKQK PGQSLEWIAW

IYA**ATG**STSY NQKFTNKAQL TVDTSSSAAY MQFSSLTTED SAIYYCARHA
G**YPHYYAMD**Y WGQGTSVTVS S

**VL aTim3 0033:**

```
  1   DIQMTQSPAS   LSASVGETVT   ITCRASENIF   SNLAWYQQKQ
GKSPQLLVYS
 51   ATNLGDGVPS   RFSGSGSGTQ   FSLKINSLQP   EDFGNYYCQH
FYKIPFTFGT
101   GTKLEIK
```

**VH aTim3_0038:**

```
  1   EVQLQQSGAE   PLKPGASVKL   TCTTSGFNIK   DYYIHWVKQR
SDQGLEWIGR
 51   IDPEDGELIY   APKFQDKATI   TVDTSSNIAY   LQLNSLTSED
TAVYYCSRDH
101   GYVGWFAYWG   QGTLVTVSA
```

**VL aTim3_0038:**

```
  1   NVVMTQSPKS   MIMSVGQRVT   LNCKASENVD   TYVSWYQQKP
EQSPKLLIYG
 51   ASNRYTGVPD   RFTGSRSATD   FTLTISSVQA   EDLAVYYCGQ
SYSYPWTFGG
101   GTKLEFR
```

## V. EXAMPLES

The following are examples of methods and compositions of the invention.

Although the foregoing invention has been described in some detail by way of illustration and example for purposes of clarity of understanding, the descriptions and examples should not be construed as limiting the scope of the invention.

### Example 1a: Generation of anti-TIM3 antibodies

### Immunization of mice

NMRI mice were immunized genetically, using a plasmid expression vector coding for full-length human Tim-3 by intradermal application of 100 ug vector DNA (plasmid 15304_hTIM3-fl), followed by Electroporation (2 square pulses of 1000 V/cm, duration 0.1 ms, interval 0.125 s; followed by 4 square pulses of 287.5 V/cm, duration 10 ms, interval 0.125 s. Mice received either 6 consecutive immunizations at days 0, 14, 28, 42, 56, 70, and 84. Blood was taken at days 36, 78 and 92 and serum prepared, which was used for titer determination by ELISA (see below). Animals with highest titers were selected for boosting at day 96, by intravenous injection of 50 ug of recombinant human Tim-3 human Fc chimera, and monoclonal antibodies were isolated by hybridoma technology, by fusion of splenocytes to myeloma cell line 3 days after boost.

### Determination of serum titers (ELISA)

Human recombinant Tim-3 human Fc chimera was immobilized on a 96-wellNUNC Maxisorp plate at 0.3 ug/ml, 100 μl/well, in PBS, followed by: blocking of the plate with 2% Crotein C in PBS, 200 μl/well; application of serial dilutions of antisera, in duplicates, in 0.5% Crotein C in PBS, 100 μl/well; detection with HRP-conjugated goat anti-mouse antibody (Jackson Immunoresearch/Dianova 115-036-071; 1/16 000). For all steps, plates were incubated for 1 h at 37° C. Between all steps, plates were washed 3 times with 0.05% Tween 20 in PBS. Signal was developed by addition of BM Blue POD Substrate soluble (Roche), 100 ul/well; and stopped by addition of 1 M HCl, 100 ul/well. Absorbance was read out at 450 nm, against 690 nm as reference. Titer was defined as dilution of antisera resulting in half-maximal signal.

**Example 1b: Characterization anti-Tim3 antibodies**

**ELISA for Tim3**

Nunc-Maxi Sorp Streptavidine plates (MicroCoat #11974998/MC1099) were coated by 25 μl/well with Tim3-ECD-His-Biotin (biotinylated with BirA Ligase) and incubated at RT for 1 h while shaking at 400 rpm rotation. After washing (3x90 μl/well with PBST-buffer) 25 μl aTim3 samples or diluted (1:2 steps) reference antibody aTim3 F38-2E2 (Biolegend) was added and incubated 1h at RT. After washing (3x90 μl/well with PBST-buffer) 25μl/well sheep-anti-mouse-POD (GE NA9310V) was added in 1:9000 dilution and incubated at RT for 1 h while shaking at 400 rpm rotation. After washing (4x90 μl/well with PBST-buffer) 25 μl/well TMB substrate (Calbiochem, #CL07) was added and incubated until OD 1.5 - 2.5. Then the reaction was stopped by addition of 25 μl/well 1N HCL-solution. Measurement took place at 370/492 nm.

ELISA results are listed as EC50-values [ng/ml] in summary Table 2 below.

**Example: Cell ELISA for Tim3**

Adherent CHO-K1 cell line stably transfected with plasmid 15312_hTIM3-fl_pUC_Neo coding for full-length human Tim3 and selection with G418 (Neomycin resistance marker on plasmid) were seeded at a concentration of 1.2x10E6 cells/ml into 384-well flat bottom plates and grown over night.

At the next day 25 μl/well Tim3 sample or aTim3 reference antibody F38-2E2 Azide free (Biolegend, 354004) was added and incubated for 2h at 4°C (to avoid internalization). After washing (3x90μl/well PBST (BIOTEK Washer: Prog. 29, 1 x 90) cells were fixed by flicking out residual buffer and addition of 50μl/well 0,05% Glutaraldehyde: Dilution 1:500 of 25% Glutaraldehyde (Sigma Cat.No: G5882) in 1xPBS-buffer and incubated for 1h at RT. After washing (3x90μl/well PBST (BIOTEK Washer: Prog. 21, 3x90 GreinLysin) 25 μl/well secondary antibody was added for detection (Sheep-anti-mouse-POD; Horseradish POD linked F(ab')$_2$ Fragment; GE NA9310) followed by 2h incubation at RT while shaking at 400 rpm. After washing (3x90μl/well PBST (BIOTEK Washer: Prog. 21, 3x90 GreinLysin) 25 μl/well TMB substrate solution (Roche 11835033001) was added and incubated until OD 1.5 - 2.5. Then the reaction was stopped by addition of 25 μl/well 1N HCL-solution. Measurement took place at 370/492 nm.

Cell ELISA results are listed as "EC50 CHO-Tim3"-values [ng/ml] in summary Table 2 below.

**Table 2: Binding affinities of exemplary antibodies (ELISA and BIACORE)**

| Assay | Tim3_0018 | Tim3_0021 | Tim3_0028 | Tim3_0026 | Tim3_0033 | Tim3_0038 |
|---|---|---|---|---|---|---|
| Affinity KD [nM] monomer / dimer Tim3 | 3.4/1.1 | 204/4.1 | 173/2.8 | 6.2/1.5 | n.f./3.1 | 7.6/0.6 |
| EC50 ELISA [nM] | 0.56 | | 0.22 | | | 0.501 |
| EC50 ELISA [ng/ml] | 94 | 47 | 37 | 47 | 1321 | 83 |
| EC50 CHO-Tim3 [nM] | 0.52 | | 0.32 | | | 0.17 |
| EC50 CHO-Tim3 [ng/ml] | 87 | 73 | 53 | 69 | 3710 | 29 |

**BIAcore characterization of the Tim3 ABs**

A surface plasmon resonance (SPR) based assay has been used to determine the kinetic parameters of the binding between several murine Tim3 binders as well as commercial human Tim3 binding references. Therefore, an anti-mouse IgG was immobilized by amine coupling to the surface of a (BIAcore) CM5 sensor chip. The samples were then captured and monomeric hu/cy Tim3-ECD as well as a Fc-tagged human Tim3-ECD dimer was bound to them. The sensor chip surface was regenerated after each analysis cycle. The equilibrium constant $K_D$ was finally gained by fitting the data to a 1:1 Langmuir interaction model.

About 12000 response units (RU) of 30 μg/ml anti-mouse IgG (GE Healthcare #BR-1008-38) were coupled onto the spots 1,2,4 and 5 of the flow cells 1-4 (spots 1,5 are active and spots 2,4 are reference spots) of a CM5 sensor chip in a BIAcore B4000 at pH 5.0 by using an amine coupling kit supplied by GE Healthcare.

The sample and running buffer was HBS-EP+ (0.01 M HEPES, 0.15 M NaCl, 3 mM EDTA, 0.05% v/v Surfactant P20, pH 7.4). Flow cell temperature was set to 25 °C and sample compartment temperature to 12 °C. The system was primed with running buffer.

The samples were injected for 30 seconds with a concentration of 200 μg/ml and bound to the spots 1 and 5 of each flow cell, allowing the measurement of eight samples in parallel. Then a complete set of different (monomeric cyno, monomeric human and huFc fused dimeric human Tim3-ECD) concentrations (s. Table X) was injected over each sample for 240 s followed by a dissociation time of 30/1800 s (s. Table 1). Each analysis cycle (sample capture, spot 1 and 5 - Tim3 ECD injection) was then regenerated with a 30 seconds long injection of Glycine-HCl pH 1.7. The flow rate was set to 30μl/min for the whole run.

Finally the double referenced data was fitted to a 1:1 Langmuir interaction model with the BIAcore B4000 Evaluation Software. Resulting affinities to monomeric human, cyno Tim3 and huFc fused dimeric human Tim3 are shown in Table 2 and 3. The affinity to the hu Tim3 dimer is most likely affected by avidity and therefore apparently stronger than the affinity to the monomeric huTim3.

**Table 3a: Binding affinities determined by BIAcore-KD values gained by a kinetic SPR measurement.-n.f. means no fit possible, most likely due to no or weak binding.**

| Sample | huTim3 $K_D$ (25 °C) [M] | huTim3Fc $K_D$ (25 °C) [M] | cyTim3 $K_D$ (25 °C) [M] |
|---|---|---|---|
| TIM3-0016 | 3.29E-09 | 1.09E-09 | 2.16E-08 |
| TIM3-0016 variant (0018) | 3.40E-09 | 1.11E-09 | 4.19E-08 |
| TIM3-0021 | 2.04E-07 | 4.07E-09 | n.f. |
| TIM3-0022 | 1.26E-07 | 1.52E-09 | 2.84E-08 |
| TIM3-0026 | 6.23E-09 | 1.52E-09 | n.f. |
| TIM3-0028 | 1.73E-07 | 2.77E-09 | n.f. |
| TIM3-0030 | 3.11E-09 | 1.28E-09 | n.f. |
| TIM3-0033 | n.f. | 3.05E-09 | n.f. |
| TIM3-0038 | 7.56E-09 | 5.69E-10 | n.f. |
| Reference antibody Biolegend F38-2E2 | 1.36E-08 | 7.50E-09 | 1.68E-07 |
| Reference antibody USB 11E365 | 1.34E-08 | 7.73E-09 | 1.41E-07 |

**Determination of the affinity to Tim3 via SPR (Chimeric TIM3-0016 variant (0018) and humanized versions)**

Protein A was immobilized by amine coupling to the surface of a (Biacore) CM5 sensor chip. The samples were then captured and hu Tim3-ECD was bound to them. The sensor chip surface was regenerated after each analysis cycle. The equilibrium constant and kinetic rate constants were finally gained by fitting the data to a 1:1 langmuir interaction model.

About 2000 response units (RU) of 20 μg/ml Protein A were coupled onto the spots 1, 2, 4 and 5 of all flow cells of a CM5 sensor chip in a Biacore B4000 instrument using an amine coupling kit supplied by GE Healthcare.

The sample and running buffer was HBS-EP+ (0.01 M HEPES, 0.15 M NaCl, 3 mM EDTA, 0.05 % v/v Surfactant P20, pH 7.4). Flow cell temperature was set to 25 °C and sample compartment temperature to 12 °C. The system was primed with running buffer.

Different samples were injected for 30 seconds with a concentration of 10 nM and bound consecutively to the spots 1 and 5 in all flow cells. Then a complete set of monomeric human Tim3-ECD dilutions (600 nM, 200 nM, 66.7 nM, 2 × 22.2 nM, 7.4 nM, 2.5 nM and 2 × 0 nM) was consecutively injected over each sample for 300s. Each antigen injection was followed by a dissociation time of 12s/1000s and two 30s regeneration steps with a Glycine-HCl pH 1.5 solution, of which the last one contained a stabilization period after injection of 20 seconds.

Finally the double referenced data was fitted to a 1:1 langmuir interaction model using the Biacore B4000 Evaluation Software. Resulting $K_D$ values are shown in Table 3b.

**Determination of the affinity to Tim3 via SPR ((Chimeric TIM3-0028 and humanized versions))**

Anti-human Fc IgG was immobilized by amine coupling to the surface of a (Biacore) CM5 sensor chip. The samples were then captured and hu Tim3-ECD was bound to them. The sensor chip surface was regenerated after each analysis cycle. The equilibrium constant and kinetic rate constants were finally gained by fitting the data to a 1:1 langmuir interaction model.

About 2500 response units (RU) of 10 µg/ml anti-human Fc IgG (GE Healthcare #BR-1008-39) were coupled onto the spots 1, 2, 4 and 5 of all flow cells of a CM5 sensor chip in a Biacore B4000 instrument using an amine coupling kit supplied by GE Healthcare.

The sample and running buffer was HBS-EP+ (0.01 M HEPES, 0.15 M NaCl, 3 mM EDTA, 0.05 % v/v Surfactant P20, pH 7.4). Flow cell temperature was set to 25 °C and sample compartment temperature to 12 °C. The system was primed with running buffer.

Different samples were injected for 30 seconds with a concentration of 10 nM and bound consecutively to the spots 1 and 5 in all flow cells. Then a complete set of monomeric human Tim3-ECD dilutions (600 nM, 200 nM, 66.7 nM, 2 × 22.2 nM, 7.4 nM, 2.5 nM and 2 × 0 nM) was consecutively injected over each sample for 300s. Each antigen injection was followed by a dissociation time of 12s/700s and two 30s regeneration steps with a 3 M MgCl$_2$ solution, of which the last one contained an "extra wash after injection" with running buffer.

Finally the double referenced data was fitted to a 1:1 langmuir interaction model using the Biacore B4000 Evaluation Software. Resulting K$_D$ values are shown in Table 3b.

Table 3b: Binding affinities determined by BIAcore-KD values gained by a kinetic SPR measurement

| Sample | huTim3 K$_D$ (25 °C) [M] |
|---|---|
| Chimeric TIM3-0016 variant (0018) | 2.78E-09 |
| TIM3-0433 | 5.74E-09 |
| TIM3-0434 | 5.76E-09 |
| Chimeric TIM3-0028 | 2.35E-07 |
| TIM3-0438 | 3.05E-07 |
| TIM3-0443 | 2.87E-07 |

**Example 2: Generation of anti-Tim3 antibodies derivatives**

**Chimeric antibody derivatives**

Chimeric Tim3 antibodies were generated by amplifying the variable heavy and light chain regions of the anti-TIM3 mouse antibodies Tim3-0016, Tim3-0016 variant (0018), Tim3-0021, Tim3-0022, Tim3-0026, Tim3-0028, Tim3-0030, and Tim3-0033, Tim3-0038 from via PCR and cloning them into heavy chain expression vectors as fusion proteins with human IgG1 backbones / human CH1-Hinge-CH2-CH3 with LALA and PG mutations (Leucine 234 to Alanine, Leucine 235 to Alanine, Proline 329 to Glycine) abrogating effector functions and light chain expression vectors as fusion proteins to human C-kappa. LC and HC Plasmids were then cotransfected into HEK293 and purified after 7 days from supernatants by standard methods for antibody purification.

**Removal of glycosylation site NYT: Modifying 1 HVR-L1 position in Tim3-0016, Tim3_0016 variant (named 0018 or Tim3_0018) by substitution of N by Q or S**

Mutations within the variable light chain region of Tim3_0016 and Tim3_0016 variant (0018) were generated by in vitro mutagenesis using Agilent "Quick Change Lightning Site-directed Mutagenesis Kit" according manufacturer's instructions. By this method the asparagine (N) of the glycosylation site motif NYT in the light chain HVR-L1 (SEQ ID NO: 4) was replaced by glutamine (Q) (resulting in SEQ ID NO: 11 = Tim3_0016_HVR-L1 variant 1_NQ) or, alternatively, the asparagine (N) was replaced by serine (S) (resulting in SEQ ID NO: 12 = Tim3_0016_HVR-L1 variant 2_ NS). In both, the glycosylation site motif NYT was successfully modified. LC and HC Plasmids coding for the variants were then cotransfected into HEK293 and purified after 7 days from supernatants by standard methods for antibody purification.

The generated mutants were tested by ELISA on human Tim3, ELISA on cynomolgus Tim3 and cellular ELISA on adherent CHO-K1 cells expressing full-length human Tim3.

**TABLE 4:**

| Antibodies and mutant antibodies tested | Biochem Human | | Biochem Cyno | | Cellular binding CHO-TIM3 | |
|---|---|---|---|---|---|---|
| | EC50 [ng/ml] values in relation to the samples max value | Inflexion point [ng/ml] | EC50 [ng/ml] values in relation to the samples max value | Inflexion point [ng/ml] | EC50 [ng/ml] values in relation to the samples max value | Inflexion point [ng/ml] |
| Anti Tim3_F38-2E2 | 73.2 | 88.3 | 423.0 | 209871.3 | 150.2 | 224.3 |
| Tim3_0018 (TIM3-0016 variant) | 15.1 | 15.3 | 14.6 | 14.6 | 26.4 | 29.4 |
| Tim3_0018MutNQ | 12.0 | 10.8 | 13.2 | 10.8 | 13.4 | 12.8 |
| Tim3_0018MutNS | 10.3 | 6.5 | 11.9 | 6.5 | 11.2 | 11.1 |
| Tim3_0016MutNQ | 7.6 | 5.7 | 8.3 | 5.7 | 6.3 | 5.4 |
| Tim3_0016MutNS | 8.5 | 5.5 | 9.7 | 5.5 | 9.1 | 8.5 |

All mutants generated were found to show even more functional binding to human TIM3 (human), cyno TIM3 (cyno) or human TIMR on CHO cells than the parental antibodies Tim3_0016 or the Tim3_0016 antibody variant Tim3_0018 respectively.

**Humanized antibody derivatives**

Humanization of the VH and VL domains of murine anti-Tim3-0016 variant (0018) and anti-Tim3_0028

Based upon the amino acid sequence of the VH and VL domains of a) anti-Tim3 antibody Tim3_0016 variant (0018) (with the amino acid sequences of the 6 HVRs wherein in the light chain the HVR-L1 variant 2_NS (removal of glycosylation site by N to S mutation) was used (SEQ ID NOs: 1, 2,3,12 ,5 and 6) humanized anti-Tim3 antibody variants Tim3-0433 and Tim3-0434 were generated and based upon the amino acid sequence of the VH and VL domains of b) anti-Tim3 antibody Tim3_0028 (with the amino acid sequences of the 6 HVRs (SEQ ID NOs: 37, 38,39,40 ,41 and 42) humanized anti-Tim3 antibody variants Tim3-0438 and Tim3-0443 were generated.

The humanized amino acid sequences for heavy and light chain variable regions of were backtranslated in to DNA and the resulting cNDA were synthesized (GenArt) and then cloned into heavy chain expression vectors as fusion proteins with human IgG1 backbones /human CH1-Hinge-CH2-CH3 with LALA and PG mutations (Leucine 234 to Alanine, Leucine 235 to Alanine, Proline 329 to Glycine) abrogating effector functions or into light chain expression vectors as fusion proteins to human C-kappa. LC and HC Plasmids were then cotransfected into HEK293 and purified after 7 days from supernatants by standard methods for antibody purification. The resulting humanized Tim3-antibodies are named as follows:

**Table: VH and VL sequences of humanized antibodies**

| Humanized antibodies of Tim3_0016 variant (0018) | VH/SEQ ID NO: | VL/SEQ ID NO: |
|---|---|---|
| Tim3-0433 | SEQ ID NO: 79 | SEQ ID NO: 80 |
| Tim3-0434 | SEQ ID NO: 81 | SEQ ID NO: 82 |
| **Humanized antibodies of Tim3_0028** | **VH/SEQ ID NO:** | **VL/SEQ ID NO:** |
| Tim3-0438 | SEQ ID NO: 83 | SEQ ID NO: 84 |
| Tim3-0443 | SEQ ID NO: 85 | SEQ ID NO: 86 |

**Table: HVR sequences of humanized antibodies**

| Humanized antibodies of Tim3_0016 variant (0018) | HVR-H1, HVR-H2, and HVR-H3 /SEQ ID NOs: | HVR-L1, HVR-L2, and HVR-L3 t/SEQ ID NOs: |
|---|---|---|
| Tim3-0433 | SEQ ID NOs: 1 , 2 and 3 | SEQ ID NOs: 12 , 5 and 6 |
| Tim3-0434 | SEQ ID NOs: 1 , 2 and 3 | SEQ ID NOs: 12 , 5 and 6 |
| Humanized antibodies of Tim3_0028 | HVR-H1, HVR-H2, and HVR-H3 /SEQ ID NOs: | HVR-L1, HVR-L2, and HVR-L3 /SEQ ID NOs: |
| Tim3-0438 | SEQ ID NOs: 37 , 38 and 39 | SEQ ID NOs: 40 , 41 and 42 |
| Tim3-0443 | SEQ ID NOs: 37 , 38 and 39 | SEQ ID NOs: 40 , 41 and 42 |

**Example 3: Fluorescent Labeling of Purified Monoclonal Antibody**

The fluorescent labeling of the hybridoma derived monoclonal antibody was carried out by using Alexa Fluor 488 Monoclonal Antibody Labeling Kit (manufactured by Invitrogen) according to the manufacturer's instructions. After the labeling, each antibody was confirmed to be positively labeled with Alexa Fluor 488 (hereinafter referred to as "Alexa-488") by FACSCalibur (manufactured by BD Biosciences) analysis for TIM-3 expressing RPMI-8226 and Pfeiffer cells.

**Example 4: Classification of Binding Epitope Groups using FACS based Competition Assay**

The relation of epitopes between generated anti-TIM3 antibodies and six anti-TIM3 reference antibodies was analyzed by a FACS based binding competition assay. The TIM3 reference antibodies were the following: antibodies 4177 and 8213 as described in US2012/189617, antibodies 1.7E10 and 27.12E12 as described in WO2013/06490; antibody 344823 (Clone 344823, manufactured by R&D Systems) and antibody F38-2E2 (Clone F38-2E2, manufactured by BioLegend and R&D Systems). In brief, the test antibody was allowed to interact and bind with the TIM-3 expressing RPMI-8226 cells (ATCC ® CCL-155™) and then it was evaluated by flow cytometry method whether another anti-TIM-3 antibody could also bind to TIM-3 expressing cells.

In short human TIM3 expressing RPMI-8226 cells were incubated with BD human Fc Block for 10 min at RT and stained in two different experimental setups to exclude the impact of the difference in the affinity of the tested antibodies on the binding:

1) with disclosed purified anti-TIM3 (10μg/ml in BD staining buffer for 0.5h at 4°C), which were conjugated with Alexa*488 according to the manufacturer's instructions (Molecular Probes A-20181) with an average of 2.7 fluorophores per antibody. Than a) unlabeled (1-4) reference recombinant anti-TIM3 antibodies or Isotype control were added (10μg/ml) for 0.5h at 4oC in BD SB and after washing with BD SB stained with PE-labeled anti-huFcy Abs (JIR, 109-116-098, 1:200, 0.5h at 4oC in BD SB) or b) PE labeled (5-6) available reference anti-TIM3 antibodies or appropriate Isotype controls were added (10μg/ml) for 0.5h at 4oC in BD SB. After washing and centrifugation MFI signals of stained RPMI-8226 cells were analyzed by BD Biosciences FACSCanto flow cytometer.

### Table 5: Summary of epitope characterization- competition of Tim3-antibodies for binding to Tim3

| | Max % Inhibition of Binding | | | | | |
|---|---|---|---|---|---|---|
| | Epitope group 1 | | | Epitope group 3 | | |
| | 1a | | 1b | 3a | 3b | |
| | Tim3_0016 | Tim3_0018 | Tim3_0026 | Tim3_0022 | Tim3_0028 | Tim3_0038 |
| clone 4177 | 1 | -9 | 29 | 79 | -3 | 0 |
| clone 8213 | -2 | 9 | 9 | 9 | 38 | 29 |
| clone 1-7E10 | -5 | 15 | 24 | 0 | 20 | 7 |
| clone 27-12E12 | -1 | 4 | 22 | 40 | 82 | 94 |
| clone 344823 | 0 | 0 | 3 | 102 | 107 | 99 |
| clone F38-2E2 | -7 | 6 | 2 | 77 | 75 | 94 |

Results from the FACS based epitope groups mapping show that Tim3_0016 and Tim3_0016 variant Tim3_0018 show no binding competition with any tested anti-TIM-3 reference antibodies and it was suggested that these Abs recognized the new epitope different from the epitopes to which all previous described TIM3 reference antibodies recognized whereas Tim3_0022, Tim3_0026, Tim3_0028 and Tim3_0038 compete to different extend for binding to surface expressed TIM3 on JRPMI-8226 cells with various competitors.

**Example 5: Effect of human anti-TIM-3 Antibodies on Cytokine Production in a Mixed Lymphocyte Reaction (MLR)**

A mixed lymphocyte reaction was used to demonstrate the effect of blocking the TIM-3 pathway to lymphocyte effector cells. T cells in the assay were tested for activation and IFN-gamma secretion in the presence or absence of an anti-TIM-3 mAbs.

Human Lymphocytes were isolated from peripheral blood of healthy donor by density gradient centrifugation using Leukosep (Greiner Bio One, 227 288). Briefly, heparinized blood were diluted with the three fold volume of PBS and 25 ml aliquots of the diluted blood were layered in 50 ml Leukosep tubes. After centrifugation at 800 x g for 15 min at room temperature (w/o break) the lymphocyte containing fractions were harvested, washed in PBS and used directly in functional assay or resuspended in freezing medium (10% DMSO, 90 %FCS) at 1.0E+07 cells/ml and stored in liquid nitrogen. 1:1 target/responder cell ratio was used in MLR assay (i.e. each MLR culture contained - 2.0E+05 PBMCs from each donor in a total volume of 200 $\mu$l. Anti-TIM3 monoclonal antibodies Tim3_0016, Tim3_0016 variant (Tim3_0018), Tim3_0021, Tim3_0022, Tim3_0026, Tim3_0028, Tim3_0030, Tim3_0033, Tim3_0038 and F38-2E2 (BioLegend), were added to each culture at different antibody concentrations. Either no antibody or an isotype control antibody was used as a negative control and rec hu IL-2 (20 EU/ml) was used as positive control. The cells were cultured for 6 days at 37°C. After day 6 100 $\mu$l of medium was taken from each culture for cytokine measurement. The levels of IFN-gamma were measured using OptEIA ELISA kit (BD Biosciences).

The results are shown in Table 6 (IFN-g secretion/release). The anti-TIM-3 monoclonal antibodies promoted T cell activation and IFN-gamma secretion in concentration dependent manner. The anti-TIM3 antibodies Tim3_0021, Tim3_0022, Tim3_0028, and Tim3_0038 reduce release of the inflammatory cytokine IFN-gamma) more than the F38-2E2 antibody. Tim3_0016, Tim3_0016 variant (Tim3_0018), Tim3_0033 and Tim3_0038 showed a similar release when compared the F38-2E2 antibody. In contrast, cultures containing the isotype control antibody did not show an increase in IFN-gamma secretion.

## Table 6a: Percentage of anti-Tim3 antibody induced IFNgamma release in comparison to rec hu IL-2 (20 EU/ml) ( = 100%) as positive control and no antibody as negative control

| Com-pound concen-tration | MLR +IL-2 20U/ml | Iso-type IgG2a | F38-2E2 | Tim3 0016 | Tim3 0018 | Tim3 0021 | Tim3 0022 | Tim3 0026 | Tim3 0028 | Tim3 0030 | Tim3 0033 | Tim3 0038 | Iso-type hIgG1 |
|---|---|---|---|---|---|---|---|---|---|---|---|---|---|
| 40 μg/ml | | 2 | 36 | 33 | 36 | 112 | 58 | 25 | 40 | 14 | 35 | 51 | 0 |
| 10 μg/ml | 100 | 0 | 26 | 22 | 30 | 108 | 38 | 16 | 38 | 4 | 30 | 38 | 5 |
| 1 μg/ml | | 0 | 7 | 7 | 12 | 101 | 18 | 18 | 12 | 3 | 0 | 1 | 0 |

In further experiments the EC50 values of the following chimeric and humanized antibodies (generated as described above) in combination with 0,1 μg/ml anti-PD1 mAb were measured: chimeric chi_Tim3_018 antibody and its humanized versions Tim3-433 and Tim3-434, chimeric chi_Tim3_028 antibody and its humanized versions Tim3-438 and Tim3-443 were measured with different lymphocyte donor mixtures (D2 and D3, or D1 and D5, respectively) Results are shown in Table 6b

### Table 6b: EC50 of anti-Tim3 antibody induced (IFN-g secretion/release)

| Antibody | EC50 [nM] with donors D2+D3 | EC50 [nM] with donors D1+D5 |
|---|---|---|
| chi_Tim3_018 | 3.1 | 4.2 |
| Tim3-433 | 3.0 | 2.4 |
| Tim3-434 | 1.7 | 2.6 |
| chi_Tim3_028 | 2.9 | 6.4 |
| Tim3-438 | 1.9 | 2.7 |
| Tim3-443 | 3.0 | 4.7 |

**Example 6: Internalization of anti-TIM-3 Antibodies into TIM-3 expressing cells**

TIM-3-specific antibodies described herein can be internalized into TIM-3-expressing cells, including TIM-3 expressing lymphoma, multiple myeloma and AML cells. For example, the disclosed TIM-3 specific antibodies and fragments thereof are shown to be internalized into rec TIM3 CHO cells stabile expressing human TIM-3 as evaluated by cell based ELISA, flow cytometry (FACS) and confocal microscopy.

E.g. stable Tim3-transfected CHO-K1 cells (clone 8) ($4x10^4$ cells/well/100μl) were seeded into 98 well-MTP using fresh culture medium. After overnight cell attachment, cell culture medium was removed and the test antibodies were added to the cells (10μg/ml in cell culture medium) and incubated for 0.5 hour at 4°C. As reference, a commercial mouse-anti-human antibody (TIM3 MAB 11E365 (US Biological, T5469-92P) was used. After washing (2x with cell culture medium) and centrifugation cells were incubated for 3 hours at a) 4°C or b) 37°C in 200 μl cell culture medium. Internalization typically occurs at 37°C, but not at 4oC, which provides another control for the reaction. Than cells were fixated with 100 μl/well 0.05 % glutharaldehyde (Sigma Cat.No: G5882) in 1xPBS for 10 min at room temperature (RT). This was followed by three washing steps with 200 μl PBS-T and secondary antibody sheep-anti-mouse-POD (Horseradish POD linked F(ab')$_2$ Fragment; GE NA9310)) were added for 1 hour at RT. After the final washing steps (3xPBS-T), TMB substrate was added (Roche order no. 11835033001) for 15 min and color development was stopped using 1N HCl. Final ODs were determined by measurement at 450/620 nm in an ELISA reader. This cellular ELISA procedure was used for medium throughput evaluation of the internalizing capacity of the testing antibodies which were purified from hybridoma supernatants.

The percentage of internalization was calculated as follow:

$$\text{Internalization } [\%] = (1 - \text{OD}_{\text{sample\_37°C}} / \text{OD}_{\text{sample\_4oC}}) * 100$$

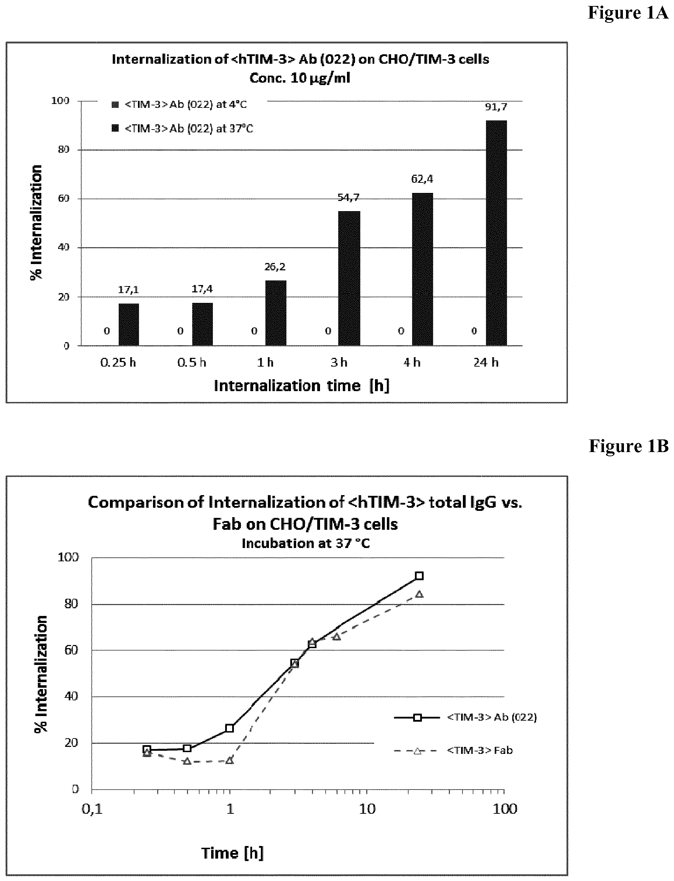

The results are shown in FIG. 1A and B for (Internalization). Almost all tested anti-TIM-3 monoclonal antibodies were similar well internalized into stable Tim3-transfected CHO-K1 cells after 3h incubation at 37°C (not all data shown).
The determination of EC50 internalizing values (time dependency) as well as comparison of the kinetics of the internalization depending on mono- vs. bivalency was estimated by FACS for selected candidates.
In short human TIM3 stable expressing CHO-K1 cells were seeded ($4 \times 10^5$ cells/well/50μl) into 98 well-v bottom MTP using fresh culture medium and incubated with Redimune® NF Liquid for 10 min at RT to block unspecific binding. Than 50 μl/well of selected purified anti-TIM3 (10μg/ml in cell culture medium) were added and incubated for 1h at 4oC. After washing (with cell culture medium) and centrifugation cells were incubated for 0.25, 0.5, 1, 2, 3, 4, 6 and 24 hours at a) 4°C or b) 37°C in 200 μl cell culture medium. Than cells were washed with PBS/1%BSA and secondary antibody Alexa Fluor 488 Goat-anti-mouseIgG, F(ab)2 were added for 1 hour at 4°C. After washing and centrifugation 125 μl of CellFix (BD Bioscience, 1:1000) were added and MFI signals of stained cells were analyzed by BD Biosciences FACSCanto flow cytometer.
The percentage of internalization was calculated as follow:

$$\text{Internalization } [\%] = (1 - \text{MFI}_{\text{sample\_37°C}} / \text{MFI}_{\text{sample\_4oC}}) * 100$$

**Example for the evaluation of time dependent internalization of anti-TIM3 antibodies Tim3_0016, Tim3_0016 variant (Tim3_0018), Tim3_0021, Tim3_0028, Tim3_0030, Tim3_0033, Tim3_0038 on RPMI-8226 cells (ATCC ® CCL-155™):**

The presently disclosed anti-TIM3 antibodies are internalized rapidly into TIM3 expressing RPMI-8226 cells (ATCC ® CCL-155™) at a high level. The experiments were conducted as described above with TIM3 expressing RPMI-8226 cells (ATCC ® CCL-155™) instead of rec CHOK1 cells expressing huTIM-3. Results are shown in the Table 7. As TIM3 reference antibodies were the following antibodies were used: antibody 8213 as described in US2012/189617 , antibody 27.12E12 as described in WO2013/06490. Tim3_0016, Tim3_0016 variant (Tim3_0018), Tim3_0038 were used as human IgG1 chimeric versions.

**Table 7: Percentage internalization at the indicated time point (0 min set as 0 percent)**

| Antibody | Percentage internalization of anti-TIM3 antibodies | | | | |
|---|---|---|---|---|---|
| | 30 Min | 60 Min | 120 Min | 240 Min | 26h |
| 8213 | 22 | 22 | 43 | 52 | 72 |
| 27.12E12 | 19 | 22 | 25 | 46 | 59 |
| Tim3_0016 | 33 | 52 | 55 | 66 | 87 |
| Tim3_0018 | 39 | 41 | 80 | 70 | 88 |
| Tim3 -0021 | 70 | 75 | 74 | 78 | 77 |
| Tim3 -0028 | 50 | 59 | 67 | 68 | 83 |
| Tim3 -0033 | 75 | 81 | 82 | 82 | 80 |
| Tim3_0038 | 22 | 20 | 45 | 46 | 63 |

From the results antibodies of the invention are rapidly internalized at high percentage compared to reference antibodies on RPMI-8226 cells (ATCC ® CCL-155™)

**Example 7: Binding of anti-TIM-3 Antibodies to isolated human Monocytes expressing TIM-3**

CD14+ Monocytes were isolated from anticoagulated peripheral blood of healphy donors by density gradient centrifugation using Ficoll-Paque (GE Healthcare) (see General Protocols in the User Manuals or visit www.miltenyibiotec.com/protocols) and subsequent positiv selection via CD14 MicroBeads. First the CD14+ cells are magnetically labeled with CD14 MicroBeads. Then the cell suspension is loaded onto a MACS® Column which is placed in the magnetic field of a MACS Separator. The magnetically labeled CD14+ cells are retained in the column. The unlabeled cells run through, this cell fraction is depleted of CD14+ cells. After removal of the column from the magnetic field, the magnetically retained CD14+ cells can be eluted as the positively selected cell fraction. After centrifugation at 200 x g for 10 min at room temperature the monocytes were harvested and and used directly in binding assay or resuspended in freezing medium (10% DMSO, 90 %FCS) at 1.0E+07 cells/ml and stored in liquid nitrogen.

As shown in the literature Monocytes express constitutively TIM3 on their surface. $1 \times 10^5$ CD14+ isolated human monocytes (50µl/well) were put into 98 well-v bottom MTP in fresh culture medium and incubated with Redimune® NF Liquid for 15 min at RT to block unspecific binding. Than 50 µl/well of disclosed anti-TIM3 mAbs or reference anti-TIM-3 mAbs 344823 (R&D) and F38-2E2 (BioLegend) (10µg/ml in cell culture medium) were added and incubated for 1h at 4oC. Than cells were washed with PBS/1%BSA and secondary antibody PE-labeled Goat-anti-mouse F(ab')2 were (Jackson Lab 115-006-072) added for 1 hour at 4oC. After washing and centrifugation MFI signals of stained cells were analyzed by BD Biosciences FACSCanto flow cytometer.

The specific binding was calculated as follow:

$$\text{Specific Binding [MFI]} = \text{Geom. Mean MFI}_{\text{sample}} - \text{Geom. Mean MFI}_{\text{isotype control}}$$

The results are shown in Table 8: (Binding to human Monocytes). TIM3 clones Tim3_0016, Tim3_0018, Tim3_0020, Tim3_0028 and Tim3_0038 bind to human monocytes of different donors even better than the reference anti- TIM-3 Abs.

## Table 8: Binding to human Monocytes

| | donor1 (CD14+) | donor2 (CD14+) | donor3 (CD14+) |
|---|---|---|---|
| Tim3 0016 | 2122 | 1634 | 1690 |
| Tim3 0018 | 2326 | 1818 | 1943 |
| Tim3 0020 | 1917 | 1377 | 1462 |
| Tim3 0021 | 1134 | 951 | 1197 |
| Tim3 0022 | 1468 | 1111 | 1235 |
| Tim3 0026 | 1665 | 1016 | 900 |
| Tim3 0030 | 1411 | 419 | 466 |
| Tim3 0038 | 1637 | 1368 | 1401 |
| Tim3 0028 | 1351 | 950 | 1607 |
| Tim3 0033 | 480 | 328 | 595 |
| M-IgG2b | 0 | 13 | 0 |
| M-IgG1 | 144 | 55 | 213 |
| <TIM-3>PE Mab, M-IgG1 (Clone F38-2E2; Biolegend) | 516 | 493 | 460 |
| <TIM-3> PE Mab , Rat IgG2A (Clone 344823, R&D) | 1010 | 917 | 814 |
| Rat-IgG2A-PE | 71 | 68 | 70 |

**Example 8: Binding of anti-TIM-3 Antibodies to isolated cyno Monocytes expressing TIM-3**

CD14+ Monocytes were isolated from cynomolgus monkey anticoagulated peripheral blood (Covance) by density gradient centrifugation using Ficoll-Paque (GE Healthcare) (see General Protocols in the User Manuals or visit www.miltenyibiotec.com/protocols) and subsequent positiv selection via NHP CD14 MicroBeads. First the CD14+ cells are magnetically labeled with CD14 MicroBeads. Then the cell suspension is loaded onto a MACS® Column which is placed in the magnetic field of a MACS Separator. The magnetically labeled CD14+ cells are retained in the column. The unlabeled cells run through, this cell fraction is depleted of CD14+ cells. After removal of the column from the magnetic field, the magnetically retained CD14+ cells can be eluted as the positively selected cell fraction. After centrifugation at 200 x g for 10 min at room temperature the monocytes were harvested and and used directly in binding assay or resuspended in freezing medium (10% DMSO, 90 %FCS) at 1.0E+07 cells/ml and stored in liquid nitrogen.

As shown in the literature Monocytes express constitutively TIM3 on their surface. 1x105 CD14+ isolated cyno monocytes (50μl/well) were put into 98 well-v bottom MTP in fresh culture medium and incubated with Redimune® NF Liquid for 15 min at RT to block unspecific binding. Than 50 μl/well of Alexa488 labeled anti-TIM3 (10μg/ml in cell culture medium) were added and incubated for 1h at 4oC. After washing and centrifugation MFI signals of stained cells were analyzed by BD Biosciences FACSCanto flow cytometer.

The specific binding was calculated as follow:

$$\text{Specific Binding [MFI]} = \text{Geom. Mean MFI}_{\text{sample}} - \text{Geom. Mean MFI}_{\text{isotype control}}$$

The results are shown in Table 9 (Binding to Cyno Monocytes). TIM3 clones Tim3_0016, Tim3_0018, Tim3_0026, Tim3_0028 and , Tim3_0030 bind to cyno monocytes of different cyno donors.

## Table 9: Binding to Cyno Monocytes

|  | cyno1 (16719M) CD14+ | cyno2 (17435M) CD14+ | cyno3 (30085F) CD14+ |
|---|---|---|---|
| AF +PI | 75 | 83 | 84 |
| HumTIM-3 Alexa488 R&D (34482) | 158 | 121 | 143 |
| Rat-IgG2A-Alexa488 | 84 | 86 | 91 |
| hum TIM-3 A488 F38-2E2 (NOVUS Biol) | 135 | 136 | 124 |
| M-IgG1-Alexa 488 | 72 | 82 | 83 |
| Tim3 _0016- A488 | 157 | 177 | 187 |
| Tim3 _0016 variant 0018- A488 | 301 | 480 | 417 |
| Tim3 0022- A488 | 115 | 134 | 138 |
| Tim3 0026- A488 | 137 | 184 | 197 |
| Tim3 0028- A488 | 3936 | 2996 | 4090 |
| Tim3 0038- A488 | 97 | 107 | 120 |
| Tim3_0020- A488 | 274 | 378 | 354 |
| Tim3 0021 A488 | 348 | 473 | 399 |
| Tim3 0030 A488 | 119 | 163 | 144 |
| Tim3 0033 A488 | 71 | 81 | 83 |
| TIM-3 (4177) A488 | 78 | 83 | 85 |
| TIM-3 (8213) A488 | 75 | 83 | 87 |

**Example 9: Binding of anti-TIM-3 Antibodies to NHL and MM cell lines expressing TIM-3**

The binding capacity of disclosed anti-TIM3 antibodies and two anti-TIM3 reference antibodies clones (1) 4177 and (2) 8213 (Kyowa) was analyzed by a FACS. In short human TIM3 expressing B cell lymphoma cells (exemplified as Pfeiffer cells) and multiple myeloma cells (exemplified as RPMI-8226 cells) were incubated with BD human Fc Block for 10 min at RT to block unspecific binding. Than 2x105 cells (50μl/well) were put into 98 well-v bottom MTP and 50 μl/well of Alexa488 labeled anti-TIM3 (10μg/ml in BD Staining buffer) were added and incubated for 1h at 4oC. After washing and centrifugation MFI signals of stained cells were analyzed by BD Biosciences FACSCanto flow cytometer.
The specific binding was calculated as follow:

$$\text{Specific Binding [MFI]} = \text{Geom. Mean MFI}_{\text{sample}} - \text{Geom. Mean MFI}_{\text{isotype control}}$$

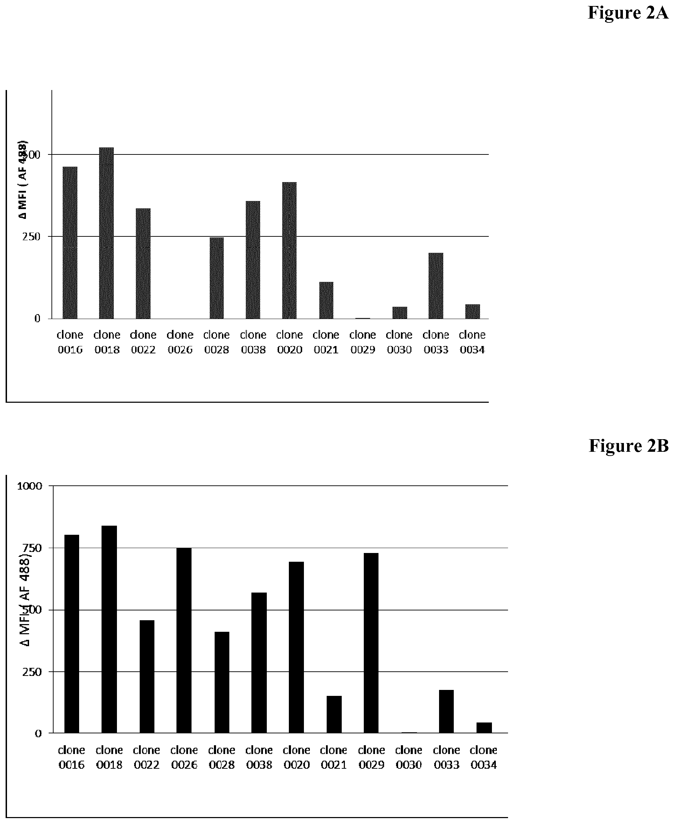

The results are shown in FIG. 2A and 2B (Binding to RPMI-8226 and Pfeiffer cells).

**Example 10: Cytotoxic activity of anti-TIM-3 Antibodies on TIM-3 expressing NHL and MM cells**

TIM3-specifc antibodies conjugated with pseudomonas exotoxin (PE 24) effectively kill TIM3-expressing cells.
The cytotoxic activity of disclosed anti-TIM3 antibodies and one commercial available anti-TIM3 reference antibody clone 11E365 (available from US Biological) was analyzed with Promega CellTiter-Glo Luminescent Cell Viability Assay. In short to $5x10^3$ (50μl/well in 98 well MTP, in triplicate) recombinant CHO K1 stabile expressing human TIM-3 or $2x10^4$ cells (50μl/well in 98 well MTP, in triplicate) human TIM3 expressing B cell lymphoma cells (exemplified as Pfeiffer cells) or multiple myeloma cells (exemplified as RPMI-8226 cells) were added 25μl/well 1:5 serial dilution of disclosed anti-TIM-3 antibodies with the highest concentration of 10μg/ml or appropriate media to untreated cells or Isotype control to untargeted treated cells. Treatment ranges from 10μg/ml to 1ng/ml in triplicate. All antibodies were used as full length mouse Fcγ versions . For conjugation of the conjugation of the Pseudomonas exotoxin 10μg/ml of mouse Fcγ fragment specific Fabs conjugated with PE 24 were added and incubated for 3 days at 37°C. Cycloheximide as a known protein synthesis inhibitor in eukaryotes was used as positive control. Viability of treated cells were measured with Promega CellTiter-Glo Luminescent Cell Viability Assay.
The cytotoxic activity was calculated as follow:

$$\text{Rel. Inhibition [\%]} = (1-(\text{E}_{\text{sample}} - \text{E}_{\text{negative control}})/(\text{E}_{\text{positive control}} - \text{E}_{\text{negative control}}))*100$$

The results are shown in Tab. 10 (Cytotoxic activity of anti-TIM3 mAbs on TIM-3 expressing recombinant, NHL and MM cell lines in sandwich format).

**Table 10:**

| Antibodies and references (all anti TIM3 antibodies conjugated to a deimmunized Pseudomonas exotoxin A) | IC50 [nM] | | |
|---|---|---|---|
| | recTIM-3 CHO cells | Pfeiffer cells | RPMI-8226 |
| Tim3_0016 | 0,04 | 0,09 | 0,55 |
| Tim3_0016 variant 1 (Tim3_0018) | 0,05 | 0,10 | 0,66 |
| Tim3_0020 | 0,07 | 0,11 | >64 |
| Tim3_0021 | 0,04 | 0,10 | 5,9 |
| Tim3_0022 | 0,02 | 0,07 | 0,36 |
| Tim3_0023 | 0,03 | 0,08 | >64 |
| Tim3_0026 | 0,03 | 0,08 | >64 |
| Tim3_0030 | 0,03 | 0,10 | >64 |
| Tim3_0033 | 0,11 | 0,20 | 0,79 |
| Tim3_0038 | 0,01 | < 0.002 | 0,16 |

(continued)

| Antibodies and references (all anti TIM3 antibodies conjugated to a deimmunized Pseudomonas exotoxin A) | IC50 [nM] | | |
|---|---|---|---|
| | recTIM-3 CHO cells | Pfeiffer cells | RPMI-8226 |
| US Biol. Clone 11E365 | 0,7 | 1,2 | 1,1 |
| Cells w/o Ab | - | - | - |
| Cells + <mFc> Fab PE | - | - | - |
| IgG2A + <mFc> Fab PE | - | - | - |
| Cycloheximide | 135 | 181 | 245 |

All tested TIM3 clones are highly potent (IC50 range 0,01-0,2 nM) on recombinant CHO K1 stabile expressing human TIM-3 and Pfeiffer cells expressing high and moderate levels of TIM-3 and even more potent in their cytotoxic activity than the strong internalizing reference anti-TIM-3 Ab clone 11E365, US Biological. TIM3 clones 0016, 0018, 0021, 0022, 0033 and 0038 are also potent on RPMI-8226 cells expressing 5 fold lower TIM-3 level compare to recombinant CHO TIM-3 cells.

**Example 11: Comparison of the cytotoxic activity of disclosed anti-TIM3 antibodies vs. two anti-TIM3 reference antibodies 1.7.E10 and 27-12E12 (as described in WO2013/06490).**

The cytotoxic activity of disclosed anti-TIM3 antibodies and two anti-TIM3 reference antibodies the TIM3 reference antibodies 1.7E10 and 27.12E12 as described in WO2013/06490 was analyzed with Promega CellTiter-Glo Luminescent Cell Viability Assay as described above. All antibodies were used as full length human IgG1 format including the human Fcgamma part. In this experiment conjugation of the Pseudomonas exotoxin was achieved via human Fc$\gamma$ fragment specific Fabs conjugated with PE 24 (10$\mu$g/ml) which were added and incubated for 5 days at 37°C.

The results are shown in Tab. 11. - Comparison of cytotoxic activity of anti-TIM3 mAbs on TIM-3 expressing NHL and MM cell lines

**Table 11: Comparison of cytotoxic activity of anti-TIM3 mAbs on TIM-3 expressing NHL and MM cell lines**

| Antibodies and references (all anti TIM3 antibodies conjugated to a deimmunized Pseudomonas exotoxin A) | Pfeiffer cells | | RPMI-8226 cells | |
|---|---|---|---|---|
| | Max. killing | Rel. IC50 [nM] | Max. killing | Rel. IC50 [nM] |
| Cycloheximide | 100 [%] | 271 | 100 [%] | 111 |
| 1.7E10 | 60.3 [%] | 0,68 | 65.7 [%] | 2,544 |
| 27-12E12 | 75.7 [%] | 0,02 | 86.6 [%] | 0,111 |
| Tim3_0016 | 84.9 [%] | 0,05 | 86.6 [%] | 0,063 |
| Tim3_0016 variant (Tim3_0018) | 82.9 [%] | 0,06 | 88.1 [%] | 0,081 |
| Tim3_0026 | 78.3 [%] | <0.02 | 83.1 [%] | 0,067 |
| Tim3_0038 | 82.6 [%] | <0.02 | 83.8 [%] | 0,047 |
| Isotype Control hIgG1 | 3.2 [%] | N.A | 0.4 [%] | N.A |

All disclosed TIM3 clones are highly active (IC50 range 0,02-0,08 nM) on Pfeiffer and RPMI-8226 cells expressing TIM-3 and even more potent in their cytotoxic activity than the strong internalizing reference anti-TIM-3 Ab clone 27-12E12. All antibodies were compared as Pseudomonas exotoxin (PE24) conjugates using the same Pseudomonas exotoxin under the same conditions

**Example 12: Cytotoxic activity of Fab-PE24 constructs of disclosed anti-TIM3 antibodies on MM, NHL and AML cell lines (expressing TIM3, but not PSMA).**

The cytotoxic activity was analyzed with Promega CellTiter-Glo Luminescent Cell Viability Assay as described above. 1:5 serial dilutions of Fab-fragments of disclosed anti-TIM3 antibodies directly conjugated to PE24 with the highest concentration of 50μg/ml or appropriate media to untreated cells or non-binding anti-PSMA Fab-PE24 control to untargeted treated cells were incubated with 7,5 x10$^3$ Pfeiffer cells or 2 x10$^3$ RPMI-8226 cells (50μl/well in 98 well MTP) for 4 days at 37°C. Treatment ranges from 50μg/ml to 8ng/ml in triplicate. Cycloheximide was used as positive control.

The results are shown in Tab. 12. (Cytotoxic activity of Fab-PE24 constructs of disclosed anti-TIM3 antibodies on MM, NHL and AML cell lines).

**Table 12: Cytotoxic activity of Fab-PE24 constructs of disclosed anti-TIM3 antibodies on diferent MM, NHL and AML cell lines (RPMI-8226, Karpas-299, CMK, TF-1, MOLM-13)**

| | RPMI-8226 | | Karpas-299 | | CMK | | TF-1 | | MOLM-13 | |
|---|---|---|---|---|---|---|---|---|---|---|
| Antibodies and references (all anti TIM3 antibodies conjugated to a deimmunized Pseudomonas exotoxin A) | Max. killing | IC50 [nM] | Max. killing | IC50 [nM] | Max. killing | IC50 [nM] | Max. killing | IC50 [nM] | Max. killing | IC50 [nM] |
| Cycloheximide | 100 [%] | 281 | 100 [%] | 113 | 100 [%] | 149,0 | 100 [%] | 207 | 100 [%] | 156 |
| Anti_PSMA | 10.5 [%] | N.A. | 40.1 [%] | N.A. | 8.98 [%] | N.A. | 5.27 [%] | N.A. | 18.9 [%] | N.A. |
| Tim3_0022 | 99.1 [%] | 1,9 | 98.8 [%] | 10 | 67.1 [%] | 255 | 58.6 [%] | 299 | 58.5 [%] | 579 |
| Tim3_0016 | 99.3 [%] | 1,1 | 99.2 [%] | 4 | 64.8 [%] | 225 | 54.2 [%] | 534 | 62.7 [%] | 459 |

All tested Fab-PE24 constructs of disclosed anti-TIM3 antibodies are highly potent (IC50 range 1-10 nM) on MM (RPMI-8226) and NHL (Karpas-299) cells expressing moderate level of TIM-3 and demonstrate significant cytotoxic activity on AML cell lines (CMK, TF-1, MOLM-13) expressing very low levels of TIM-3.

**Example 13: Cytotoxic activity of Immuno conjuagets (Pseudomonas Exotoxin A conjugates (Fab-PE24 constructs) of disclosed anti-TIM3 on primary leukemic stem/progenitor AML cells from relapsed/refractory patients**

CD34+ cells from peripheral blood of relapsed/refractory patients were obtained from AllCells, LLC, Alameda, CA.

**Table 13: Clinical characteristics of AML patients**

| Specimens | Donor | Clinical Diagonis | Gender | Age: y | FAB subtype | WBC [x10*9/L] | CD 34+ cells [%] | PLT [x10*9/L ] | Cytogen. abnormal. |
|---|---|---|---|---|---|---|---|---|---|
| AML1 | EBO PB0136 | AML Relaps./ Refract. | F | 64 | M2 | 4 | 50 | | N.A. |
| AML2 | EBO PB0142 | AML Relaps./ Refract. | F | 35 | N.A. | 65 | 22 | | Normal |
| AML3 | EBO PB0135 | AML Relaps./ Refract. | M | 72 | M0 | 4 | 15 | | N.A. |
| AML4a | EBO PB0193 | AML Relaps./ Refract. | M | 76 | N.A. | 21 | 52 | | der(7)t(7;? 13) |

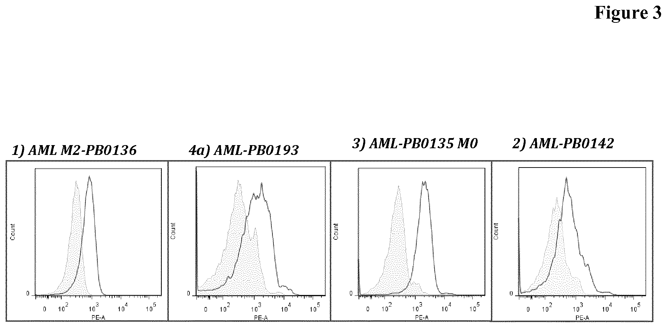

After confirmation of purity and viability of all samples (purity range 84-94% and viability range 95-99% ) the expression level of TIM-3 was evaluated by FACS as described in Example 7 using anti-TIM-3 mAbs 344823 (R&D). (see Figure 3)

All tested (4/4) primary leukemic stem/progenitor (CD34+) AML samples from relapsed/refractory patients demonstrate homogeneous expression of TIM-3 at different levels.

For the evaluation of cytotoxic activity of Fab-PE24 constructs of disclosed anti-TIM3 clones 0016 and 0022 on primary CD34+ AML cells 1 x10$^4$ cells (50$\mu$l/well in 98 well MTP, in triplicate) were incubated with 1:5 serial dilutions of Fab-fragments with the highest concentration of 50$\mu$g/ml or appropriate media to untreated cells or non-binding anti-PSMA Fab-PE24 control to untargeted treated cells for 3 days at 37°C. Cycloheximide was used as positive control. Cytotoxic activity was analysed with Promega CellTiter-Glo Luminescent Cell Viability Assay as described above in Example 12.

The results are shown in Tab.14. (Cytotoxic activity of Fab-PE24 constructs of disclosed anti-TIM3 antibodies on primary CD34+ AML cells).

**Table 14: Cytotoxic activity of Fab-PE24 constructs of disclosed anti-TIM3 antibodies on primary CD34+ AML cells**

| Antibodies and references (all anti TIM3 antibodies conjugated to a deimunized Pseudomonas exotoxin A) | D1; AML CD34+ PB0136 cells | | D2; AML CD34+ PB0142 cells | | D3; AML CD34+ PB0135 cells | | D4; AML CD34+ PB0193 cells | |
|---|---|---|---|---|---|---|---|---|
| | **Max. killing** | **IC50 [nM]** | **Max. killing** | **IC50 [nM]** | **Max. killing** | **IC50 [nM]** | **Max. killing** | **IC50 [nM]** |
| Cycloheximide | 100 [%] | 212 | 100 [%] | 262 | 100 [%] | 121 | 100 [%] | 208 |
| anti-PSMA | 2 [%] | N.A. | 8 [%] | N.A. | 18 [%] | N.A. | 12 [%] | N.A. |
| TIM-3 0022-cFP | **38 [%]** | >691 | **75 [%]** | **107** | **31 [%]** | >691 | **57 [%]** | **375** |
| TIM-3 0016-cFP | **48 [%]** | >691 | **79 [%]** | **30** | **44 [%]** | >691 | **69 [%]** | **116** |

Fab-PE24 constructs of anti-TIM3 antibodies Tim3_0016 and Tim3_0022 are highly potent on (2/4) primary AML samples (PB0142 and PB0135) (IC50 range 30-116 nM) and demonstrate significant cytotoxic activity on all (4/4) primary leukemic stem/progenitor (CD34+) AML cells expressing different levels of TIM-3.

**Example 14: Comparison of potency of Fab-PE24 constructs of selected anti-TIM3 antibodies on NHL and MM cell lines**

The evaluation of cytotoxic activity of sortase coupled Fab-PE24 constructs of selected disclosed anti-TIM3 antibodies was analysed with Promega CellTiter-Glo Luminescent Cell Viability Assay as described above in Example 12.

The results are shown in Tab. 15. (Cytotoxic activity of Fab-PE24 constructs of selected anti-TIM3 antibodies on NHL and MM cells).

**Table 15: Cytotoxic activity of Fab-PE24 constructs of selected anti-TIM3 antibodies on NHL and MM cells**

| Antibodies and references (all anti TIM3 antibodies conjugated to a deimunized Pseudomonas exotoxin A) | Pfeiffer cells | | RPMI-8226 cells | |
|---|---|---|---|---|
| | **Max. killing** | **IC50 [nM]** | **Max. killing** | **IC50 [nM]** |
| Cycloheximide | 100 [%] | 271,1 | 100 [%] | 153 |
| anti-PSMA | 25.2 [%] | N.A. | 21.5 [%] | N.A. |
| TIM-3 0022 | 99.9 [%] | 1,58 | 99.6 [%] | 2,14 |
| TIM-3 0016 | 99.6 [%] | 0,77 | 99.2 [%] | 0,61 |
| TIM-3 0021 | 98.4 [%] | 2,15 | 99.1 [%] | 3,61 |
| TIM-3 0033 | 99.8 [%] | 5,30 | 99.7 [%] | 5,73 |

(continued)

|  | Pfeiffer cells | | RPMI-8226 cells | |
| Antibodies and references (all anti TIM3 antibodies conjugated to a deimunized Pseudomonas exotoxin A) | Max. killing | IC50 [nM] | Max. killing | IC50 [nM] |
|---|---|---|---|---|
| TIM-3 0038 | 99.6 [%] | 0,47 | 98.3 [%] | 0,32 |

High cytotoxic potency was demonstrated with Fab-PE24 constructs of all selected disclosed anti-TIM3 antibodies (IC50 range 0,3-5 nM) on NHL (Pfeiffer) and MM (RPMI-8226) cells expressing moderate level of TIM-3.

The highest cytotoxic activity was observed with Fab-PE24 constructs of disclosed anti-TIM3 antibodies Tim3_0016 and Tim3_0038.

**Example 15: Comparison of cytotoxic activity of Fab-PE24 construct vs. total-IgG-Amatoxin conjugate of the same clone of disclosed anti-TIM-3 antibody on Pfeiffer cells**

The evaluation of cytotoxic activity of conjugated Fab-PE24 construct of disclosed anti-TIM3 clone 0016 vs. total IgG of the same clone conjugated with Amatoxin (according to th procedures described in WO2012/041504 (conjugated via the 6'C-atom of amatoxin amino acid 4, particularly via an oxygen atom bound to the 6'C-atom of amatoxin amino acid, and wherein the TIM3 antibody is connected by a linker via a urea moiety) was analysed with Promega CellTiter-Glo Luminescent Cell Viability Assay as described above in Example 12.

The results are shown in Tab. 16. (Cytotoxic activity of Fab-PE24 construct vs. total IgG- Amatoxin conjugate of anti-TIM3 clone 0016 on NHL cells).

**Table 16: Cytotoxic activity of Fab-PE24 construct vs. total IgG- Amatoxin conjugate of anti-TIM3 clone 0016 on NHL cells**

| Pfeiffer cells | Max. killing | IC50 [nM] |
|---|---|---|
| Cycloheximide | 100 [%] | 163 |
| Isotype hIgG1 Amatoxin | 28 [%] | N.A. |
| TIM-3 0016-Amatoxin | 93.3 [%] | 0,81 |
| TIM-3 0016-PE24 | 99.8 [%] | 0,25 |

Cytotoxic activity of Amanitin-conugated anti-TIM-3 clone 0016 (IC50 0,8 nM) is comparable with cytotoxic activity of Fab-PE24 construct of the same clone (IC50 0,3 nM) on NHL (Pfeiffer) cells expressing moderate level of TIM-3.

**Example 15: Direct omparison of binding of TIM3 antibodies to different peripheral blood mononuclear cells (PBMC)**

**Binding assay**

Freshly isolated PBMCs or 3 days polyclonally activated (plate bound anti-CD3 and soluble anti-CD28 antibodies, 1 ug/ml each, both from BD Pharmingen) CD4 T cells were stained with Alexa 647-directly conjugated anti-TIM-antibodies Tim3-0018, Tim3-0028 or chimerized or humanized versions thereof for 1 hour at 4 C degrees. The cells were then washed to eliminate unbound antibody and stained for surface markers for 30 minutes at 4 C degrees to discriminate monocytes (CD14[+] (BD Pharmingen)), NK cells (CD16[+] (eBioscience), CD56[+] (BioLegend) and CD3[-]) and T cells (CD3[+] (eBioscience)) before being fixed with BD Cell Fix. The cells were aquired at LSRFortessa, BD Biosciences.

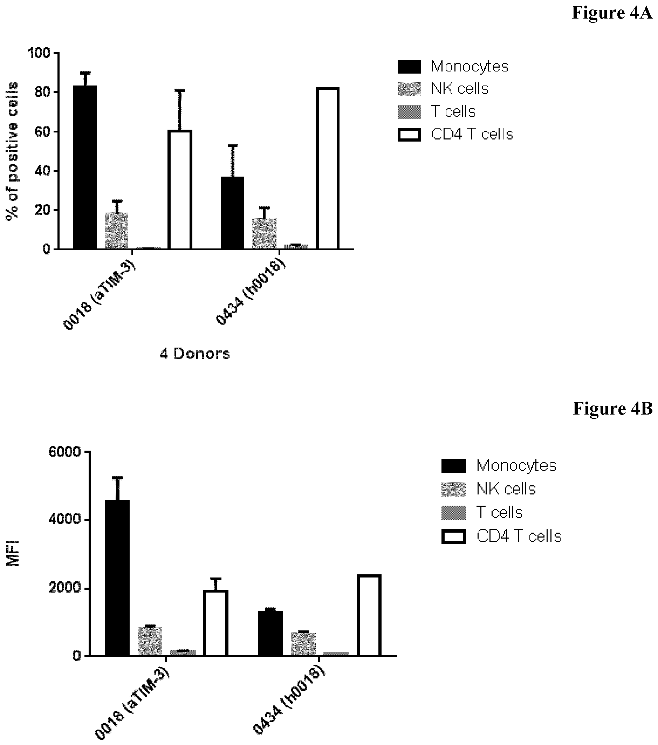

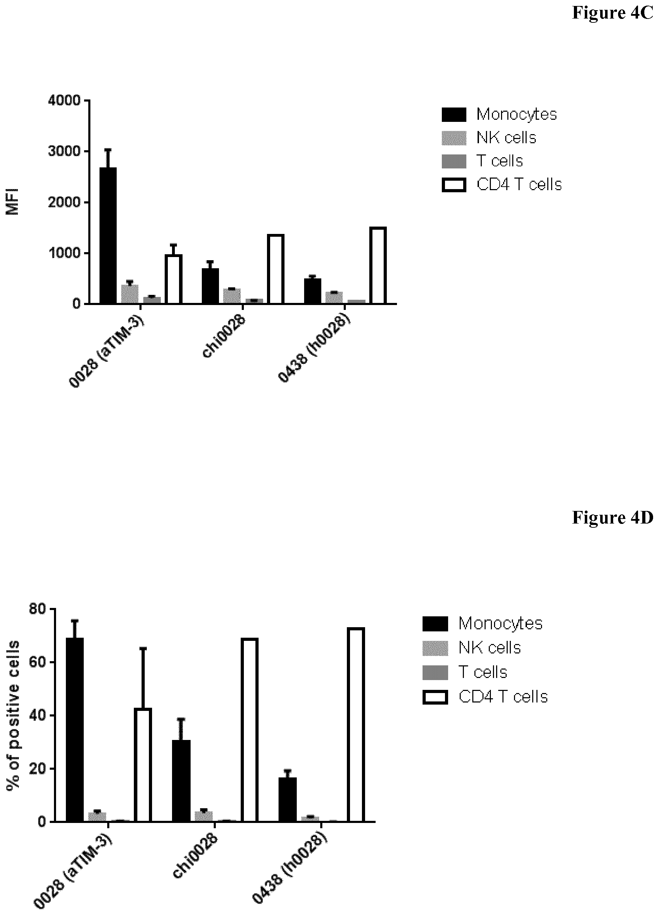

Results are shown in Figures 4A to 4D (in the Figures the following designations were used: for Tim3-0018: 0018 (aTIM-3), for humanized Tim3-0018 version Tim3-0434: 0434(h0018), for Tim3-0028: 0028 (aTIM-3), for chimeric Tim3-0028: chi0028, for humanized Tim3-0028 version Tim3-0438: 0438(h0028)). The data show that the humanized antibodies have improved binding and binding specificity to CD4 Tcells when compared to the parental antibodies.

## Claims

1. An isolated antibody that binds to TIM3, wherein the antibody comprises

   i) a VH sequence of SEQ ID NO:83 and a VL sequence of SEQ ID NO:84, or
   ii) a VH sequence of SEQ ID NO:85 and a VL sequence of SEQ ID NO:86.

2. The antibody according to claim 1, wherein the antibody comprises a VH sequence of SEQ ID NO:83 and a VL sequence of SEQ ID NO:84.

3. The antibody according to claim 1, wherein the antibody comprises a VH sequence of SEQ ID NO:85 and a VL sequence of SEQ ID NO:86.

4. The antibody according to any one of the preceding claims, which is a full length IgG1 antibody.

5. The antibody of according to any one of the preceding claims, which is a full length IgG1 antibody with mutations L234A, L235A and P329G (numbering according to the EU index of Kabat).

6. Isolated nucleic acid encoding the antibody according to any one of the preceding claims.

7. A host cell comprising the nucleic acid of embodiment 6.

8. A method of producing an antibody comprising culturing the host cell of embodiment 7 so that the antibody is produced.

9. The method of claim 8, further comprising recovering the antibody from the host cell.

10. An immunoconjugate comprising the antibody according any one of claims 1 to 5 and a cytotoxic agent.

11. A pharmaceutical formulation comprising the antibody according any one of claims 1 to 5 or the immunoconjugate of claim 10 and a pharmaceutically acceptable carrier.

12. The antibody according any one of claims 1 to 5 or the immunoconjugate of claim 10 for use as a medicament.

13. The antibody according any one of claims 1 to 5 or the immunoconjugate of claim 10 for use in treating cancer.


**Patentansprüche**

1. Isolierter Antikörper, der an TIM3 bindet, wobei der Antikörper Folgendes umfasst

   i) eine VH-Sequenz von SEQ ID NO:83 und eine VL-Sequenz von SEQ ID NO:84, oder
   ii) eine VH-Sequenz von SEQ ID NO:85 und eine VL-Sequenz von SEQ ID NO:86.

2. Antikörper nach Anspruch 1, wobei der Antikörper Folgendes umfasst eine VH-Sequenz von SEQ ID NO:83 und eine VL-Sequenz von SEQ ID NO:84.

3. Antikörper nach Anspruch 1, wobei der Antikörper Folgendes umfasst eine VH-Sequenz von SEQ ID NO:85 und eine VL-Sequenz von SEQ ID NO:86.

4. Antikörper nach einem der vorhergehenden Ansprüche, der ein IgG1-Antikörper voller Länge ist.

5. Antikörper nach einem der vorhergehenden Ansprüche, der ein IgG1-Antikörper voller Länge mit Mutationen L234A, L235A und P329G (Nummerierung nach dem EU-Index von Kabat) ist.

6. Isolierte Nukleinsäure, die den Antikörper nach einem der vorhergehenden Ansprüche codiert.

7. Wirtszelle, umfassend die Nukleinsäure von Ausführungsform 6.

8. Verfahren zum Produzieren eines Antikörpers, umfassend das Kultivieren der Wirtszelle von Ausführungsform 7, sodass der Antikörper produziert wird.

9. Verfahren nach Anspruch 8, ferner umfassend das Gewinnen des Antikörpers aus der Wirtszelle.

10. Immunkonjugat, umfassend den Antikörper nach einem der Ansprüche 1 bis 5 und ein zytotoxisches Mittel.

11. Pharmazeutische Formulierung, umfassend den Antikörper nach einem der Ansprüche 1 bis 5 oder das Immunkonjugat nach Anspruch 10 und einen pharmazeutisch unbedenklichen Träger.

**12.** Antikörper nach einem der Ansprüche 1 bis 5 oder das Immunkonjugat nach Anspruch 10 zur Verwendung als ein Medikament.

**13.** Antikörper nach einem der Ansprüche 1 bis 5 oder das Immunkonjugat nach Anspruch 10 zur Verwendung beim Behandeln von Krebs.

**Revendications**

**1.** Anticorps isolé qui se lie à la TIM3, dans lequel l'anticorps comprend

i) une séquence VH de SEQ ID NO : 83 et une séquence VL de SEQ ID NO : 84, ou
ii) une séquence VH de SEQ ID NO : 85 et une séquence VL de SEQ ID NO : 86.

**2.** Anticorps selon la revendication 1, dans lequel l'anticorps comprend une séquence VH de SEQ ID NO : 83 et une séquence VL de SEQ ID NO : 84.

**3.** Anticorps selon la revendication 1, dans lequel l'anticorps comprend une séquence VH de SEQ ID NO : 85 et une séquence VL de SEQ ID NO : 86.

**4.** Anticorps selon l'une quelconque des revendications précédentes, qui est un anticorps IgG1 pleine longueur.

**5.** Anticorps selon l'une quelconque des revendications précédentes, qui est un anticorps IgG1 pleine longueur avec les mutations L234A, L235A et P329G (numérotation selon l'indice EU de Kabat).

**6.** Acide nucléique isolé codant pour l'anticorps selon l'une quelconque des revendications précédentes.

**7.** Cellule hôte comprenant l'acide nucléique selon la revendication 6.

**8.** Procédé de production d'un anticorps comprenant la culture de la cellule hôte selon la revendication 7 de sorte à produire l'anticorps.

**9.** Procédé selon la revendication 8, comprenant en outre la récupération de l'anticorps de la cellule hôte.

**10.** Immunoconjugué comprenant l'anticorps selon l'une quelconque des revendications 1 à 5 et un agent cytotoxique.

**11.** Formulation pharmaceutique comprenant l'anticorps selon l'une quelconque des revendications 1 à 5 ou l'immunoconjugué selon la revendication 10 et un véhicule pharmaceutiquement acceptable.

**12.** Anticorps selon l'une quelconque des revendications 1 à 5 ou immunoconjugué selon la revendication 10 pour une utilisation comme médicament.

**13.** Anticorps selon l'une quelconque des revendications 1 à 5, ou immunoconjugué selon la revendication 10 pour une utilisation dans le traitement d'un cancer.

Figure 1A

Figure 1B

Figure 2A

Figure 2B

**Figure 3**

1) AML M2-PB0136          4a) AML-PB0193          3) AML-PB0135 M0          2) AML-PB0142

**Figure 4A**

**Figure 4B**

**Figure 4C**

**Figure 4D**

**REFERENCES CITED IN THE DESCRIPTION**

**Patent documents cited in the description**

- WO 9627603 A
- WO 2003063792 A
- WO 2009091547 A
- WO 201306490 A
- US 2012189617 A
- WO 2005052006 A
- WO 2007016150 A
- WO 2007014743 A
- WO 2007031741 A
- WO 200932954 A
- WO 201132022 A
- WO 2012154530 A
- WO 2012170617 A
- WO 2010115630 A
- WO 2010115629 A
- WO 2012119787 A
- WO 2012041504 A
- WO 2014135282 A
- WO 9316185 A
- US 5571894 A
- US 5587458 A
- US 5869046 A
- EP 0404097 A
- WO 199301161 A
- US 6248516 B1
- US 4816567 A
- US 5821337 A
- US 7527791 B
- US 6982321 B
- US 7087409 B
- US 6075181 A
- US 6150584 A
- US 5770429 A
- US 7041870 B
- US 20070061900 A
- US 7189826 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- WO 9308829 A
- US 5731168 A
- WO 2009089004 A
- US 4676980 A
- US 20060025576 A
- US 20080069820 A
- WO 2009080251 A
- WO 2009080252 A
- WO 2009080253 A
- WO 2009080254 A
- WO 2010112193 A
- WO 2010115589 A
- WO 2010136172 A
- WO 2010145792 A
- WO 2010145793 A
- WO 2011117330 A
- WO 2012025525 A
- WO 2012025530 A
- WO 2013026835 A
- WO 2013026831 A
- WO 2013164325 A
- WO 2013174873 A
- US 6737056 B
- US 7332581 B
- WO 2004056312 A
- US 20050014934 A
- US 7371826 B
- US 5648260 A
- US 5624821 A
- WO 9429351 A
- US 7521541 B
- US 5648237 A
- US 5789199 A
- US 5840523 A
- US 5959177 A
- US 6040498 A
- US 6420548 B
- US 7125978 B
- US 6417429 B
- US 5208020 A
- US 5416064 A
- EP 0425235 B1
- US 5635483 A
- US 5780588 A
- US 7498298 B
- US 5712374 A
- US 5714586 A
- US 5739116 A
- US 5767285 A
- US 5770701 A
- US 5770710 A
- US 5773001 A
- US 5877296 A

- US 6630579 B
- WO 9411026 A
- US 4737456 A
- US 20050260186 A
- US 20060104968 A
- US 6267958 B
- US 6171586 B
- WO 2006044908 A
- EP 14192175
- EP 15188056

**Non-patent literature cited in the description**

- **KOGUCHI K et al.** *J Exp Med.,* 2006, vol. 203, 1413-1418
- **MAJETI R et al.** *PNAS,* 2009, vol. 106, 3396-3401
- **LIU W et al.** *PNAS,* 2012, vol. 109, 11782-11787
- **MAZOR R et al.** *PNAS,* 2014, vol. 111, 8571-8576
- **ALEWINE C et al.** *Mol Cancer Ther.,* 2014, 2653-61
- **KABAT, E.A. et al.** Sequences of Proteins of Immunological Interest. Public Health Service, National Institutes of Health, 1991
- **KABAT, E.A. et al.** Sequences of Proteins of Immunological Interest. 1991, vol. 1-3
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **MACCALLUM et al.** *J. Mol. Biol.,* 1996, vol. 262, 732-745
- **FLATMAN, S. et al.** *J. Chromatogr. B,* 2007, vol. 848, 79-87
- **KINDT, T.J. et al.** Kuby Immunology. W.H. Freeman and Co, 2007, 91
- **PORTOLANO, S. et al.** *J. Immunol.,* 1993, vol. 150, 880-887
- **CLACKSON, T. et al.** *Nature,* 1991, vol. 352, 624-628
- **CHEN, Y. et al.** *J. Mol. Biol.,* 1999, vol. 293, 865-881
- **HUDSON, P.J. et al.** *Nat. Med.,* 2003, vol. 9, 129-134
- **PLUECKTHUN, A.** The Pharmacology of Monoclonal Antibodies. Springer-Verlag, 1994, vol. 113, 269-315
- **HOLLIGER, P. et al.** *Proc. Natl. Acad. Sci. USA,* 1993, vol. 90, 6444-6448
- **HUDSON, P.J. et al.** *Nat. Med.,* vol. 9 (20039), 129-134
- **MORRISON, S.L. et al.** *Proc. Natl. Acad. Sci. USA,* 1984, vol. 81, 6851-6855
- **ALMAGRO, J.C. ; FRANSSON, J.** *Front. Biosci.,* 2008, vol. 13, 1619-1633
- **RIECHMANN, I. et al.** *Nature,* 1988, vol. 332, 323-329
- **QUEEN, C. et al.** *Proc. Natl. Acad. Sci. USA,* 1989, vol. 86, 10029-10033
- **KASHMIRI, S.V. et al.** *Methods,* 2005, vol. 36, 25-34
- **PADLAN, E.A.** *Mol. Immunol.,* 1991, vol. 28, 489-498
- **DALL'ACQUA, W.F. et al.** *Methods,* 2005, vol. 36, 43-60
- **OSBOURN, J. et al.** *Methods,* 2005, vol. 36, 61-68
- **KLIMKA, A. et al.** *Br. J. Cancer,* 2000, vol. 83, 252-260
- **SIMS, M.J. et al.** *J. Immunol.,* 1993, vol. 151, 2296-2308
- **CARTER, P. et al.** *Proc. Natl. Acad. Sci. USA,* 1992, vol. 89, 4285-4289
- **PRESTA, L.G. et al.** *J. Immunol.,* 1993, vol. 151, 2623-2632
- **BACA, M. et al.** *J. Biol. Chem.,* 1997, vol. 272, 10678-10684
- **ROSOK, M.J. et al.** *J. Biol. Chem.,* vol. 271 (19969), 22611-22618
- **VAN DIJK, M.A. ; VAN DE WINKEL, J.G.** *Curr. Opin. Pharmacol.,* 2001, vol. 5, 368-374
- **LONBERG, N.** *Curr. Opin. Immunol.,* 2008, vol. 20, 450-459
- **LONBERG, N.** *Nat. Biotech.,* 2005, vol. 23, 1117-1125
- **KOZBOR, D.** *J. Immunol.,* 1984, vol. 133, 3001-3005
- **BRODEUR, B.R. et al.** Monoclonal Antibody Production Techniques and Applications. Marcel Dekker, Inc, 1987, 51-63
- **BOERNER, P. et al.** *J. Immunol.,* 1991, vol. 147, 86-95
- **LI, J. et al.** *Proc. Natl. Acad. Sci. USA,* 2006, vol. 103, 3557-3562
- **NI, J.** *Xiandai Mianyixue,* 2006, vol. 26, 265-268
- **VOLLMERS, H.P. ; BRANDLEIN, S.** *Histology and Histopathology,* 2005, vol. 20, 927-937
- **VOLLMERS, H.P. ; BRANDLEIN, S.** *Methods and Findings in Experimental and Clinical Pharmacology,* 2005, vol. 27, 185-191
- **HOOGENBOOM, H.R. et al.** *Methods in Molecular Biology,* 2001, vol. 178, 1-37
- **MCCAFFERTY, J. et al.** *Nature,* 1990, vol. 348, 552-554
- **MARKS, J.D. et al.** *J. Mol. Biol.,* 1992, vol. 222, 581-597

- **MARKS, J.D. ; BRADBURY, A.** *Methods in Molecular Biology,* 2003, vol. 248, 161-175
- **SIDHU, S.S. et al.** *J. Mol. Biol.,* 2004, vol. 338, 299-310
- **LEE, C.V. et al.** *J. Mol. Biol.,* 2004, vol. 340, 1073-1093
- **FELLOUSE, F.A.** *Proc. Natl. Acad. Sci. USA,* 2004, vol. 101, 12467-12472
- **LEE, C.V. et al.** *J. Immunol. Methods,* 2004, vol. 284, 119-132
- **WINTER, G. et al.** *Ann. Rev. Immunol.,* 1994, vol. 12, 433-455
- **GRIFFITHS, A.D. et al.** *EMBO J.,* 1993, vol. 12, 725-734
- **HOOGENBOOM, H.R. ; WINTER, G.** *J. Mol. Biol.,* 1992, vol. 227, 381-388
- **MILSTEIN, C. ; CUELLO, A.C.** *Nature,* 1983, vol. 305, 537-540
- **TRAUNECKER, A. et al.** *EMBO J.,* 1991, vol. 10, 3655-3659
- **BRENNAN, M. et al.** *Science,* 1985, vol. 229, 81-83
- **KOSTELNY, S.A. et al.** *J. Immunol.,* 1992, vol. 148, 1547-1553
- **GRUBER, M et al.** *J. Immunol.,* 1994, vol. 152, 5368-5374
- **TUTT, A. et al.** *J. Immunol.,* 1991, vol. 147, 60-69
- **CHOWDHURY, P.S.** *Methods Mol. Biol.,* 2008, vol. 207, 179-196
- **HOOGENBOOM, H.R. et al.** *Methods in Molecular Biology,* 2002, vol. 178, 1-37
- **CUNNINGHAM, B.C. ; WELLS, J.A.** *Science,* 1989, vol. 244, 1081-1085
- **SHIELDS, R.L. et al.** *J. Biol. Chem.,* 2001, vol. 276, 6591-6604
- **GUYER, R.L. et al.** *J. Immunol.,* 1976, vol. 117, 587-593
- **KIM, J.K. et al.** *J. Immunol.,* 1994, vol. 24, 2429-2434
- **DUNCAN, A.R. ; WINTER, G.** *Nature,* 1988, vol. 322, 738-740
- **KAM, N.W. et al.** *Proc. Natl. Acad. Sci. USA,* 2005, vol. 102, 11600-11605
- **CHARLTON, K.A.** Methods in Molecular Biology. Humana Press, 2003, vol. 248, 245-254
- **GERNGROSS, T.U.** *Nat. Biotech.,* 2004, vol. 22, 1409-1414
- **LI, H. et al.** *Nat. Biotech.,* 2006, vol. 24, 210-215
- **GRAHAM, F.L. et al.** *J. Gen Virol.,* 1977, vol. 36, 59-74
- **MATHER, J.P.** *Biol. Reprod.,* 1980, vol. 23, 243-252
- **MATHER, J.P. et al.** *Annals N.Y. Acad. Sci.,* 1982, vol. 383, 44-68
- **URLAUB, G. et al.** *Proc. Natl. Acad. Sci. USA,* 1980, vol. 77, 4216-4220
- **YAZAKI, P. ; WU, A.M.** Methods in Molecular Biology. Humana Press, 2004, vol. 248, 255-268
- Epitope Mapping Protocols. Methods in Molecular Biology. Humana Press, 1996, vol. 66
- **HARLOW, E. ; LANE, D.** Antibodies: A Laboratory Manual. Cold Spring Harbor Laboratory, 1988
- **HINMAN, L.M. et al.** *Cancer Res.,* 1993, vol. 53, 3336-3342
- **LODE, H.N. et al.** *Cancer Res.,* 1998, vol. 58, 2925-2928
- **KRATZ, F. et al.** *Curr. Med. Chem.,* 2006, vol. 13, 477-523
- **JEFFREY, S.C. et al.** *Bioorg. Med. Chem. Lett.,* 2006, vol. 16, 358-362
- **TORGOV, M.Y. et al.** *Bioconjug. Chem.,* 2005, vol. 16, 717-721
- **NAGY, A. et al.** *Proc. Natl. Acad. Sci. USA,* 2000, vol. 97, 829-834
- **DUBOWCHIK, G.M. et al.** *Bioorg. & Med. Chem. Letters,* 2002, vol. 12, 1529-1532
- **KING, H.D. et al.** *J. Med. Chem.,* vol. 45 (20029), 4336-4343
- **VITETTA, E.S. et al.** *Science,* 1987, vol. 238, 1098-1104
- **CHARI, R.V. et al.** *Cancer Res.,* 1992, vol. 52, 127-131
- Remington's Pharmaceutical Sciences. 1980